# EUROPEAN PATENT SPECIFICATION

(11) **EP 3 999 156 B1**
(45) Date of publication and mention of the grant of the patent: **16.10.2024**
(21) Application number: 20840905.2
(22) Date of filing: 20.07.2020
(51) Int. Cl.: A61M 16/06

(54) **PATIENT INTERFACE**
PATIENTENSCHNITTSTELLE
INTERFACE PATIENT

(30) Priority: 18.07.2019 US 201962875678 P
(43) Date of publication of application: 25.05.2022
(60) Divisional application: 24198956.5
(73) Proprietor: Fisher & Paykel Healthcare Limited, East Tamaki Auckland 2013 (NZ)
(72) Inventor: ZOELLNER, Sascha Kristopher, Auckland, 2013 (NZ); DUTHIE, Neil Gray, Auckland, 2013 (NZ); NIHOTTE, Joseph Jules, Auckland, 2013 (NZ); PERERA, Ashani Melisha, Auckland, 2013 (NZ); NELSON, Grant Leigh, Auckland, 2013 (NZ); O'NEILL, Brendan, Auckland, 2013 (NZ); GORDON, James Alexander, Auckland, 2013 (NZ); GRAHAM, Ryan Anthony, Auckland, 2013 (NZ)
(74) Representative: Boult Wade Tennant LLP
(86) International application number: PCT/NZ2020/050072
(87) International publication number: WO 2021/010845

(56) References cited:
- WO-A1-2018/065926
- WO-A1-2019/180668
- WO-A1-2020/110010
- FR-A1- 2 397 199
- US-A1- 2007 006 879
- US-A1- 2007 006 879
- US-A1- 2010 122 705
- US-A1- 2014 026 889
- US-A1- 2014 026 889
- US-A1- 2016 022 944

## Description

### FIELD OF THE INVENTION

Present invention relates to a patient interface for delivering respiratory gas to a patient. In particular, the present invention relates to a non-invasive patient interface.

### BACKGROUND

One current treatment for obstructive respiration diseases, such as chronic obstructive pulmonary disease (COPD - which includes emphysema, refractory asthma and Chronic bronchitis), is non-invasive ventilation (NIV). This treatment applies a positive airway pressure to the lungs throughout the inhalation and exhalation cycle so as to splint the airways open. This improves the flow of respiratory gas into and out of the lungs.

However, one side effect of the positive pressure applied in current NIV treatments is that it can make patients uncomfortable and, therefore, less willing to undergo the treatment. A follow-on effect of the positive pressure is that it requires the patient interface to be secured firmly to the patient to avoid leakages and, thereby ensure that the pressure is maintained in the patient interface and the respiratory system. Such firm application of the interface can cause pressure sores, particularly for patients that are semi-conscious or unconscious and, therefore, are unable to provide feedback on any soreness caused by the pressure of the patient interface on their skin.

The NIV treatment gives rise to two challenges, namely compliance (the extent to which patients are willing to submit to the treatment) and pressure sores. In addition to these challenges, a further challenge for patients with obstructive respiration diseases is flushing carbon dioxide out of anatomical dead space. Specifically, the end of the exhalation cycle is characterised by a reduction in pressure of the exhaled respiratory gas. This means that the carbon dioxide-loaded respiratory gas remains in the throat, nose and mouth of the patient and is pulled back into the lungs at the commencement of the inhalation cycle. Replacing the carbon dioxide-loaded respiratory gas in these regions with respiratory gas that includes oxygen in suitable concentrations for treating obstructive respiration diseases therefore assists patients.

It is desirable to provide a patient interface that improves patient comfort and that reduces pressure sores.

It is also desirable to provide a patient interface that assists with flushing anatomical dead space.

US 2007/0006879 describes a multi-chamber mask that includes a shell and a partition. The shell is adapted to cover portions of a user's face including the mouth and at least portions of the nose including the nostrils. The shell is adapted to substantially prevent gas from escaping between the shell and the portions of the user's face that are contacted by the shell. The partition is coupled to the shell and cooperates with the shell to define a first chamber and a second chamber. The first chamber is adapted to be positioned proximate the user's nose to direct inflow of gas from a gas supply source to the user's nasal passages. The second chamber is adapted to be positioned proximate the user's mouth and the partition is adapted to restrict flow of gas between the first and the second chamber.

US 2014/0026889 described in one embodiment, a ventilation system for providing gas under a first pressure to the nose of a user and second pressure to the mouth of a user is disclosed. The described system can include a flow generator that can be connected to a gas supply tube. The gas supply tube can be in fluid communication with a ventilation interface. At least a portion of the gas supply tube can have a divider within the channel of the tube forming a nasal passageway and an oral passageway. The ventilation interface can include a nasal breathing chamber in fluid communication with the nasal passageway and an oral breathing chamber in fluid communication with the oral passageway.

### SUMMARY OF THE INVENTION

The invention is defined by claim 1 and its appended claims.

According to a first aspect, there is provided a non-invasive patient interface having a seal member which is shaped to encompass a mouth and nares of a patient, the interface defining:
(a) a primary flow path to communicate respiratory gas from a gas source to each of the mouth and the nares separately, and
(b) a flushing flow path to communicate respiratory gas from the primary flow path and/or from the gas source to the nares.

The primary flow path may include a primary flow cavity having one or more primary flow inlets for respiratory gas and having one or more primary flow outlets to deliver the respiratory gas to each of the mouth and the nares.

The primary flow path may include a primary flow cavity having one or more primary flow inlets for respiratory gas and having one or more primary flow outlets to deliver the respiratory gas to each of the mouth and the nares separately.

The flushing flow path may include a flushing flow cavity having one or more flushing flow inlets for respiratory gas and one or more flushing flow outlets to deliver the respiratory gas to the nares of a patient.

The interface may include a mask housing and wherein at least one of the one or more primary flow inlets is in the housing.

The primary flow cavity may be formed by the seal member and the housing.

At least one of the one or more primary flow inlets may be formed in the mask housing. The patient interface may include one or more of the flushing flow inlets in the seal member.

The interface may include one or more of the flushing flow inlets in the mask housing.

The flushing flow cavity may be a bifurcated cavity having one flushing fluid inlet, two passages downstream of a bifurcation and respective flushing flow outlets at a downstream end of each of the passages.

The flushing flow cavity may be integrally formed with the seal member.

The flushing flow outlets may be flush with primary flow outlets to the nares.

The flushing flow outlets may be recessed within the primary flow cavity from the one or more primary flow outlets to the nares.

The flushing flow cavity may be shaped to direct respiratory gas into the nares.

The flushing flow cavity may be shaped to accelerate respiratory gases into the nares.

The flushing flow cavity may be defined by a cavity wall extending between the one or more flushing flow inlets and the one or more flushing flow outlets.

The flushing flow cavity may be defined by the cavity wall and the outer wall of the seal member.

The flushing flow cavity may be configured to accelerate the respiratory gas. The acceleration of the respiratory gas is an increase in the velocity of the respiratory gas. References, throughout this specification and the claims, to acceleration of the respiratory gas are taken to have the same meaning, unless indicated otherwise.

The seal member may have a first primary flow outlet defined by a first portion of the mask seal which forms a seal or substantially forms a seal surrounding the mouth of the patient and a second primary flow outlet defined by a second portion of the mask seal which forms a seal or substantially forms a seal around the nares of the patient.

The seal member may be a resilient material and may be connected to the mask housing to form a unitary structure.

The seal member may be a resilient material and may be mechanically locked to the mask housing to form a unitary structure.

The seal member may be over moulded onto the mask housing to mechanically lock with the mask housing.

The interface may include a mask frame that has one or more respiratory gas flow channels that enable respiratory gas flow from a gas source to the one or more primary flow inlets and to the one or more flushing flow inlets.

The mask frame may have a single respiratory gas flow channel.

The mask frame may have separate flow channels for delivering respiratory gas to the one or more primary flow inlets and to the one or more flushing flow inlets, and wherein the flow channel or channels for delivering respiratory gas to the one or more flushing flow inlets is configured to accelerate the respiratory gas.

The mask frame may be removably connectable to the mask housing.

The mask frame may be removably connectable by co-operable snap-fit formations on the mask housing and on the mask frame.

The mask frame may be permanently connectable to the housing by co-operable formations on the mask housing and the mask frame.

The mask frame and the housing may be integrally formed.

The interface includes vent holes to allow gas, including exhaled gas, to escape the primary flow cavity.

The vent holes may be in the mask housing.

The seal member has one or more primary flow outlets that direct respiratory gas into the nares and one or more flushing flow outlets that direct respiratory gas into the nares and wherein the primary flow outlets and the flushing flow outlets may be arranged to collectively form a nasal aperture.

The cavity wall may extend along an outer wall of the seal member so that the flushing flow cavity is defined by the cavity wall and the outer wall.

The flushing flow cavity may be a single passage from the one or more flushing flow inlets to the one or more flushing flow outlets.

The cavity wall of the flushing flow cavity may separate the one or more flushing flow outlets from the one or more primary flow outlets which direct respiratory gas to the nares.

The one or more flushing flow outlets may have a predetermined shape.

The cavity wall may include a tether that links to a rim of the nasal aperture and that resists deformation of the one or more flushing flow outlets from the predetermined shape.

The interface includes two flushing flow outlets which may be separated by the tether so that the flushing flow outlets are substantially aligned with the nares.

The tether may be substantially aligned with the septum of a patient when in use.

The predetermined shape may be an hourglass or lemniscate or hippopede shape.

The predetermined shape may be figure-eight shaped.

The tether and an outlet end of the cavity wall may be flush with the primary flow outlets which direct respiratory gas into the nares.

The tether may be recessed into the primary flow cavity to avoid contact with the patient when the interface is fitted to the patient.

The cavity wall may be recessed into the primary flow cavity to avoid contact with the patient when the interface is fitted to the patient.

The cavity wall may be recessed from the nasal aperture to avoid contact with the patient when the interface is fitted to the patient.

The flushing flow cavity may have higher resistance to gas flow than compared to the resistance to gas flow of the primary flow cavity and wherein the shape of the flushing flow cavity is selected to accelerate the gas flow sufficiently to cause flushing of anatomical dead space.

The mask frame may have a primary flow channel and a flushing flow channel that communicate respectively with the one or more primary flow inlets and the one or more flushing flow inlets.

The flushing flow channel may have internal dimensions that permit a lower volumetric flow rate of respiratory gas than the volumetric flow rate of respiratory gas permitted by the primary flow channel at the same temperature and pressure.

The interface may include a flow splitter that has a single inlet which is connectable to a single gas source and has two outlets that are connectable respectively to the primary flow channel and to the flushing flow channel of the mask frame.

The one or more primary flow inlets and the one or more flushing flow inlets may be in the mask housing.

The one or more primary flow inlets may be in the mask housing and the one or more flushing flow inlets may be in the seal member.

The flushing flow path may include a flushing flow cavity which has at least one outlet that is separate from the seal member such that the seal member and the outlet can move independently of each other.

The interface may include one primary flow inlet and one flushing flow inlet and wherein the interface further includes a mask frame that has a first respiratory gas flow channel that enables respiratory gas to flow from a gas source to the primary flow inlet and a second respiratory gas flow channel that enables respiratory gas to flow from the gas source to the flushing flow inlet.

The flushing flow cavity may be formed as a conduit which is separate of the seal member and wherein the conduit has a distal end connected to the second respiratory gas flow channel and a proximal end that terminates at or adjacent to the nasal aperture.

The proximal end of the conduit terminates at a location that may be recessed from the nasal aperture.

The conduit may be surrounded by the primary flow cavity.

The conduit may be formed of resilient material.

The conduit may be linked to the seal member such that the flushing flow outlet tracks the nasal aperture during seal deformation.

The conduit may be linked to the seal member by one or more web members.

The location and shape of the web members may be selected to substantially retain the flexibility of the seal member without the web members.

The one or more web members may be linked to the seal member at locations that cause the one or more web members to impart a force on the conduit when the seal member is deformed by the patient's nose during fitting or adjustment.

The one or more web members may link the proximal end of the conduit to the seal member at a location adjacent to or at a rim of the nasal aperture.

The one or more web members may extend from a position intermediate the proximal and distal ends of the conduit to link with the seal member at a location remote from the nasal aperture.

At least one web member may be a partition wall in a plane of symmetry of the seal member.

One or more web members may have a constant cross-section throughout their length.

One or more web members may have a cross-section that tapers.

The cross-section may taper outwardly toward ends of the web member from a point intermediate the ends.

The seal member may include flexible regions which adapt to the shape of a patient's face and relatively inflexible structural regions that support the flexible regions and wherein the one or more web members are linked to the structural regions.

The structural regions may coincide with a perimeter of the mask frame
The structural regions may comprise a portion of the seal member that attaches to the mask housing via over-moulding.

The seal member may include flexible regions which adapt to the shape of a patient's face and relatively inflexible structural regions that support the flexible regions and wherein the one or more web members are linked to the flexible regions.

The conduit and the second respiratory gas flow channel may have co-operable formations that enable the conduit to be fitted to the second respiratory gas flow channel such that the flushing flow outlet is aligned to direct respiratory gas into nares of a patient when the interface is fitted to a patient.

The co-operable formations may comprise a flange about an outlet of the second respiratory gas flow channel and a flange-receiving groove in an inner wall of the conduit.

The groove may be shaped to limit the extent to which the conduit can be fitted onto the second respiratory gas flow channel, thereby ensuring proper alignment of the conduit to direct respiratory gas into nares of a patient when the interface is fitted to a patient.

The shape of the groove may complement the shape of the flange.

The co-operable formations may comprise a flange portion extending at least partly about an outlet of the secondary respiratory gas channel, a recess which is adjacent and distal to the flange and a flange-receiving groove in an inner wall of the conduit which defines an inwardly directed lip, wherein the lip latches into the recess when the flange portion is seated in the groove, thereby ensuring proper alignment of the conduit to direct respiratory gas into nares of a patient when the interface is fitted to a patient.

The inner wall of the conduit may be flush with the inner wall of the secondary respiratory gas channel at the point where the conduit connects to the secondary respiratory gas channel.

The conduit may have one or more preferential deformation zones remote from the flushing flow outlet to enable the conduit to track movement of the nasal aperture whilst substantially maintaining the shape of the flushing flow outlet.

The one or more deformation zones may have reduced wall thickness compared to the wall thickness of adjacent areas.

The one or more deformation zones may comprise bands.

The bands may have a curved profile or a square profile.

The deformation zones may be formed in an outer wall of the conduit to maintain a low resistance flow path in the flushing flow cavity.

According to a second aspect, there is provided a non-invasive patient interface having a seal-forming seal member which is shaped to encompass a mouth and nares of a patient, the interface defining:
(a) a primary flow cavity to communicate respiratory gas from a gas source to each of the mouth and the nares separately,
(b) a flushing flow cavity to communicate respiratory gas from the gas source to the nares; and
wherein the primary flow cavity and the flushing flow cavity are separated by a cavity wall that forms a partition between inlets to the primary flow cavity and to the flushing flow cavity.

The primary flow cavity may have one or more primary flow inlets for respiratory gas and may have one or more primary flow outlets to deliver the respiratory gas to each of the mouth and the nares.

The primary flow cavity may have one or more primary flow inlets for respiratory gas and may have one or more primary flow outlets to deliver the respiratory gas to each of the mouth and the nares separately.

The flushing flow cavity may have one or more flushing flow inlets for respiratory gas and may have one or more flushing flow outlets to deliver the respiratory gas to the nares of a patient.

The interface may include a housing and wherein at least one of the one or more primary flow inlets is in the housing.

The primary flow cavity may be formed by the seal member and the housing.

The mask housing includes an opening that is partitioned by the cavity wall to define the primary flow inlet and the flushing flow inlet.

The flushing flow cavity may be integrally formed with the seal member.

The flushing flow cavity may be shaped to direct respiratory gas into the nares.

The flushing flow cavity may have a shape that accelerates the respiratory gas as it flows from the flushing flow inlet to the one or more flushing flow outlets.

The seal member may have a first primary flow outlet defined by a first portion of the mask seal which forms a seal or substantially forms a seal surrounding the mouth of the patient and a second primary flow outlet defined by a second portion of the mask seal which forms a seal or substantially forms a seal with nares of the patient.

The flushing flow outlets may be flush with a primary flow outlet to the nares.

The seal member may be a resilient material and may be connected to the mask housing to form a unitary structure.

The seal member may be a resilient material and is mechanically locked to the mask housing to form a unitary structure.

The seal member may be over-moulded onto the mask housing to mechanically lock with the mask housing.

The interface may include a mask frame that has one or more respiratory gas flow channels that enable respiratory gas flow from a gas source to the one or more primary flow inlets and to the one or more flushing flow inlets.

The mask frame may have a single respiratory gas flow channel that communicates respiratory gas to the opening that defines the primary flow inlet and the flushing flow inlet.

The mask frame may have separate flow channels for delivering respiratory gas to the one or more primary flow inlets and to the one or more flushing flow inlets, and wherein the flow channel or channels for delivering respiratory gas to the one or more flushing flow inlets is configured to accelerate the respiratory gas.

The mask frame may be removably connectable to the mask housing.

The mask frame may be removably connectable by co-operable snap-fit formations on the mask housing and on the mask frame.

The mask frame may be removably connectable by co-operable formations on the mask housing and on the mask frame.

The mask frame and the housing may be integrally formed.

The interface may include vent holes to allow gas, including exhale gas, to escape the primary flow cavity.

The vent holes may be in the mask housing.

The interface may include an exhaust cavity that enables exhaled respiratory gas to be vented externally of the interface.

The exhaust cavity may be defined in part by an exhaust cavity wall which separates the exhaust cavity from the flushing flow cavity

The exhaust cavity may be defined by the exhaust cavity wall and the outer wall of the seal member.

The exhaust cavity may have at least one exhaust gas inlet to receive exhaled gas from the nares and has an exhaust outlet in the seal member that vents the exhaled gas externally of the interface.

The at least one exhaust gas inlet may be adjacent to the flushing flow outlet.

The at least one exhaust gas inlet may be in the exhaust cavity wall

The at least one exhaust gas inlet may be in the outer wall adjacent the one or more flushing flow outlets so that the nares overlap the at least one exhaust gas inlet and the one or more flushing flow outlets when the interface is fitted to a patient.

According to a third aspect, there is provided, a non-invasive patient interface having a seal-forming seal member which is shaped to encompass a mouth and nares of a patient, the interface defining:
(a) a primary flow path to communicate respiratory gas from a gas source to each of the mouth and the nares separately, and
(b) a flushing flow path to communicate respiratory gas from the primary flow path and/or from the gas source to the nares; and
wherein the primary flow path includes a primary flow cavity having a primary flow inlet and the flushing flow path includes a flushing flow cavity having a flushing flow inlet and wherein the primary flow inlet and the flushing flow inlet are formed by the seal member.

The primary flow cavity may have one or more primary flow outlets to deliver the respiratory gas to each of the mouth and the nares.

The primary flow cavity may have one or more primary flow outlets to deliver the respiratory gas to each of the mouth and the nares separately.

The flushing flow cavity may have one or more flushing flow outlets to deliver the respiratory gas to the nares of a patient.

The interface may include a housing fixed to the seal member such that the primary flow cavity is formed by the seal member and the housing.

The flushing flow cavity may be integrally formed with the seal member.

The one or more flushing flow outlets may be flush with a primary flow outlet to the nares.

The flushing flow cavity may be shaped to direct respiratory gas into the nares.

The flushing flow cavity may be defined by a cavity wall extending between the one or more flushing flow inlets and the one or more flushing flow outlets.

The cavity wall may extend along an outer wall of the seal member so that the flushing flow cavity is defined by the cavity wall and the outer wall.

The flushing flow cavity may be configured to accelerate the respiratory gas.

The seal member may have a first primary flow outlet defined by a first portion of the mask seal which forms a seal or substantially forms a seal surrounding the mouth of the patient and a second primary flow outlet defined by a second portion of the mask seal which forms a seal or substantially forms a seal with nostrils of the patient.

The seal member may be a resilient material and may be connected to the mask housing to form a unitary structure.

The seal member may be a resilient material and is mechanically locked to the mask housing to form a unitary structure.

The seal member may be over moulded onto the mask housing to mechanically lock with the mask housing.

The interface may include a mask frame that has a respiratory gas flow channel that enables respiratory gas to flow from a gas source to the primary flow inlet and to the flushing flow inlet.

The mask frame may be removably connectable to the mask housing.

The mask frame may be removably connectable by co-operable snap-fit formations on the mask housing and on the mask frame.

The mask frame may be removably connectable by co-operable formations on the mask housing and on the mask frame.

The mask frame and the housing may be integrally formed.

The interface may include vent holes to allow gas, including exhale gas, to escape the primary flow cavity.

The vent holes may be in the mask housing.

The shapes of the primary flow cavity and the flushing flow cavity may be selected to provide a resistance to gas flow which enables the delivery of respiratory gas through the primary flow cavity to provide pressure support therapy and which delivers respiratory gas flow through the flushing flow cavity to provide flushing of anatomical dead space.

The seal member may include a nasal aperture which is a combination of the flushing flow outlet and the primary flow outlet that delivers respiratory gas to the nares, each of these two outlets contributes a cross-sectional area to an overall cross-sectional area of the nasal aperture and wherein a ratio of the cross-sectional area of the flushing flow outlet to the cross-sectional area of the primary flow outlet that delivers respiratory gas to the nares is selected to provide a desired resistance to flow of respiratory gas through the primary flow cavity and through the flushing flow cavity.

The cavity wall may be connected to the seal member so that the ratio remains substantially the same when the interface is fitted to a patient.

The cavity wall may be connected to a rim of the nasal aperture such that the cavity wall partitions the nasal aperture.

The flushing flow cavity may have higher resistance to gas flow compared to the resistance to gas flow of the primary flow cavity and wherein the shape of the flushing flow cavity is selected to accelerate the gas flow sufficiently to cause flushing of anatomical dead space.

The mask housing may have a generally U-shaped form.

The primary flow inlet and the flushing flow inlet may have a combined cross-sectional area that enables mould tool cores, used to form the flushing flow cavity and the primary flow cavity when moulding the seal member to the mask housing, to be removed through the primary flow inlet and the flushing flow inlet.

According to a fourth aspect, there is provided a non-invasive patient interface having a seal-forming seal member which is shaped to encompass a mouth and nares of a patient, the interface defining:
(a) a primary flow cavity having one or more primary flow inlets for respiratory gas and having one or more primary flow outlets to deliver the respiratory gas to each of the mouth and the nares separately; and
(b) a flushing flow cavity having one or more flushing flow inlets for respiratory gas and one or more flushing flow outlets to deliver the respiratory gas to the nares of a patient; and
wherein the primary flow cavity and the flushing flow cavity are separated by a cavity wall and the primary flow outlet to the nares and the flushing flow outlet to the nares are adjacent each other and the cavity wall is located to enable respiratory gas from the flushing flow cavity to enter the nares and to enable exhaled gas to exit the nares into the primary flow cavity.

The cavity wall includes one or more preferential deformation regions which accommodate deformation of the cavity wall without occluding the flushing flow cavity.

In a fifth aspect, there is provided a non-invasive patient interface that forms a seal or substantially forms a seal about the mouth and nares of a patient, the interface defining:
(a) a primary flow cavity having one or more primary flow inlets for respiratory gas and having one or more primary flow outlets to deliver the respiratory gas to each of the mouth and the nares separately; and
(b) a flushing flow cavity having one or more flushing flow inlets for respiratory gas and one or more flushing flow outlets to deliver the respiratory gas to the nares of a patient; and
wherein the primary flow cavity and the flushing flow cavity are separated by a cavity wall and cavity wall includes one or more preferential deformation regions which accommodate deformation of the cavity wall without occluding the flushing flow cavity.

The interface may include a seal member and a mask housing and wherein the interface includes one primary flow inlet and one flushing flow inlet and both are located in the seal member.

The flushing flow cavity may be integrally formed with the seal member.

The flushing flow outlets may be flush with a primary flow outlet to the nares.

The flushing flow cavity may be shaped to direct respiratory gas into the nares.

The flushing flow cavity may be defined by a cavity wall extending between the one or more flushing flow inlets and the one or more flushing flow outlets.

The cavity wall may extend along the inside of an outer wall of the seal member so that the flushing flow cavity is defined by the cavity wall and the outer wall.

The flushing flow cavity may be configured to accelerate the respiratory gas.

The seal member may have a first primary flow outlet defined by a first portion of the mask seal which forms a seal or substantially forms a seal surrounding the mouth of the patient and a second primary flow outlet defined by a second portion of the mask seal which forms a seal or substantially forms a seal with nostrils of the patient.

The seal member may be a resilient material and may be connected to the mask housing to form a unitary structure.

The seal member may be a resilient material and is mechanically locked to the mask housing to form a unitary structure.

The seal member may be over-moulded onto the mask housing to mechanically lock with the mask housing.

The interface may include a mask frame that has first and second respiratory gas flow channels that enable respiratory gas flow from a gas source to the flushing flow inlet and to the primary flow inlet respectively.

The first respiratory gas flow channel may have a first inlet and the second respiratory gas flow channel has a second inlet and wherein the cross-sectional areas of the first and second inlets are selected to provide a desired resistance to flow through the first and second respiratory gas flow channels.

The cross-sectional area of the first inlet may be greater than the cross-sectional area of the second inlet such that the first respiratory gas flow channel has a lower resistance to flow than the resistance to flow of the second respiratory gas flow channel.

The interface may include one or more pressure ports for monitoring pressure within the patient interface.

The mask frame may be removably connectable to the mask housing.

The mask frame and the housing may be integrally formed.

The mask frame may be removably connectable by co-operable snap-fit formations on the mask housing and on the mask frame.

The mask frame may be removably connectable by co-operable formations on the mask housing and on the mask frame.

The interface may include vent holes to allow gas, including exhaled gas, to escape the primary flow cavity.

The vent holes may be in the mask housing.

The mask seal may have one or more primary flow outlets that direct respiratory gas into the nares and one or more flushing flow outlets that direct respiratory gas into the nares and wherein the primary flow outlets and the flushing flow outlets are arranged side-by-side to form a nasal aperture.

The flushing flow cavity may be a single passage from the one or more flushing flow inlets to the one or more flushing flow outlets.

The cavity wall of the flushing flow cavity may separate the one or more flushing flow outlets from the one or more primary flow outlets which direct respiratory gas to the nares.

The one or more flushing flow outlets may have a predetermined shape.

The cavity wall may include a tether that links to a rim of the nasal aperture and that retains the one or more flushing flow outlets in the predetermined shape.

The interfaces may include two flushing flow outlets which are separated by the tether so that the flushing flow outlets are aligned with the nares.

The tether may be configured to be aligned with a patient's septum when the mask is in use.

The seal member may be formed so that the cavity wall does not come into contact with the patient.

The cavity wall may be recessed from the nasal aperture.

The cavity wall may be linked to a rim of the nasal aperture or is linked to the seal member adjacent to the rim by a tether which is recessed from the nasal aperture to avoid contact with the patient.

The deformation region may decouple one portion of the cavity wall from another portion of the cavity wall such that a force applied to one portion is not transferred to the other portion.

The deformation region may decouple one portion of the cavity wall from another portion of the cavity wall such that the two portions can move relative to each other.

The deformation region may be shaped to roll over one portion of the cavity wall when the two portions of the cavity wall translate with respect to one another.

The deformation region may have a wall thickness that is less than the wall thickness of the two portions such that the deformation region is more flexible than each of the two portions.

The cavity wall may have upper and lower cavity wall portions which are linked by the deformation region such that the upper cavity wall portion translates with respect to the lower cavity wall portion.

The two portions of the cavity wall may be each shaped to resist deformation due to forces on the seal by engagement with the user's face.

The upper and lower cavity wall portions may be inclined relative to each other and are connected by the deformation region which enables relative shear movement between the portions.

The upper cavity wall portion may have an inclined valley shape with sides that curve upwardly and the lower cavity wall portion may be a curved wall with sides that curve outwardly in a rearward direction and the deformation region links the upper cavity wall portion and the lower cavity wall portion.

The deformation region may have a curved profile. Optionally, the deformation region may be a U-shaped wall.

The lower cavity wall portion may terminate in a rim that is recessed from the nasal aperture such that the tether and the deformation region co-operate to retain the rim at a location recessed from the nasal aperture when a deformation force is applied to the seal member.

The shapes of the upper cavity wall portion and the lower cavity wall portion may be selected to avoid occlusion of the primary flow cavity and the flushing flow cavity when they are deformed.

The tether may be recessed from the nasal aperture so that the tether does not contact the patient when the interface is fitted to the patient.

The cavity wall may be connected to a rim of the nasal aperture so that the nasal aperture remains in the same relative position to the rim of the cavity wall.

The cavity wall may be connected to a rim of the nasal aperture to define cross-sectional areas of the flushing flow outlet and the primary flow outlet to the nares and to resist changing the cross-sectional areas of the flushing flow outlet and the primary flow outlet when the seal member is deformed.

The seal member may include deformation resistant regions that translate deformation forces into the deformation regions such that deformation of the cavity wall is substantially confined to the one or more preferential deformation regions.

The seal member may include a bead surrounding the rim of the nasal aperture wherein the bead has a wall thickness that is greater than the wall thickness of the surrounding seal member such that the bead is less flexible than the surrounding seal member and, therefore, resists occlusion of the nasal aperture.

The tether may be connected to the bead so that deformation forces are transmitted through the bead, to the tether, to one or both of the two cavity wall portions and then to the one or more preferential deformation regions.

The tether may contribute to resisting deformation of the nasal aperture.

According to a sixth aspect, there is provided a non-invasive patient interface having a seal-forming seal member which is shaped to encompass a mouth and nares of a patient, the interface defining:
(a) a primary flow cavity to communicate respiratory gas to each of the mouth and the nares separately, and
(b) an exhaust flow cavity to communicate exhaled gas from the mouth and nares and to communicate excess respiratory gas from the primary flow cavity or both to externally of the interface; and
wherein the seal member includes a cavity wall that separates the primary flow cavity from the exhaust flow cavity and includes a nasal aperture that enables respiratory gas to flow into and from the nares and wherein the cavity wall and the nasal aperture are arranged to enable respiratory gas from the primary flow cavity to flow to the nares via the nasal aperture and enable exhaled respiratory gas from the nares, the primary flow cavity, or from both to flow to the exhaust cavity via the nasal aperture.

The primary flow cavity may have one or more primary flow inlets for respiratory gas and have one or more first primary flow outlets to deliver the respiratory gas to the mouth and one or more second primary flow outlets to deliver the respiratory gas to the nares.

The seal member may include the nasal aperture and an oral aperture that enables respiratory gas to flow into and from the mouth via the primary flow cavity.

The interface may include an exhaust vent to communicate exhaled gas and excess respiratory gas from the exhaust flow cavity to a location external of the interface.

An exhaust vent is used to exhaust respiratory gas from the cushion module. An exhaust vent may comprise a single aperture or a group of apertures. The terms "vents", "vent holes", "bias vents", "bias vent apertures", "vent apertures" and "exhaust vent" are used throughout the specification to describe an exhaust vent.

The patient interface may include a mask housing and wherein the interface includes one primary flow inlet and one exhaust flow outlet and both are located in the seal member.

The exhaust flow cavity may be integrally formed with the seal member.

The exhaust flow inlets may be flush with the primary flow outlet to the nares.

The seal member may be a resilient material and may be connected to the mask housing to form a unitary structure.

The seal member may be a resilient material and may be mechanically locked to the mask housing to form a unitary structure.

The seal member may be over-moulded onto the mask housing to mechanically lock with the mask housing.

The interface may include a mask frame that includes the exhaust vent.

The interface may include one or more pressure ports for monitoring pressure within the interface.

The mask frame may be removably connectable to the mask housing.

The mask frame may be removably connectable by co-operable snap-fit formations on the mask housing and on the mask frame.

The mask frame may be removably connectable by co-operable formations on the mask housing and on the mask frame.

The seal member may have one or more second primary flow outlets that direct respiratory gas into the nares and one or more exhaust flow inlets that receive gas flow from the nares and from the primary flow cavity and wherein the second primary flow outlets and the exhaust flow inlets are adjacent to form the nasal aperture.

The seal member may be formed so that the cavity wall does not come into contact with the patient.

The cavity wall may be recessed from the nasal aperture.

The cavity wall may be linked to a rim of the nasal aperture or is linked to the seal member adjacent to the rim by a tether which is recessed from the nasal aperture to avoid contact with the patient.

The cavity wall may be recessed from the rim of the nasal aperture by an extent that permits gas from the primary flow cavity to flow into the exhaust flow cavity.

The cavity wall may be recessed from the rim of the nasal aperture by an extent that, in use of the patient interface, permits gas from the primary flow cavity to flow into the exhaust flow cavity.

The nasal aperture may comprise a volume between the rim of the nasal aperture and an end of the cavity wall recessed from the rim.

The cavity wall may include one or more preferential deformation regions which accommodate deformation of the exhaust flow cavity without occluding the exhaust flow cavity.

The deformation region may decouple one portion of the cavity wall from another portion of the cavity wall such that a force applied to one portion is not transferred to the other portion.

The deformation region may decouple one portion of the cavity wall from another portion of the cavity wall such that the two portions can move relative to each other.

The two portions of the cavity wall may be shaped to resist deformation.

The seal member may include deformation resistant regions that translate deformation forces into the deformation regions such that deformation of the seal member is substantially confined to the deformation regions.

The seal member may include a bead surrounding the rim of the nasal aperture wherein the bead has a wall thickness that is greater than the wall thickness of the surrounding seal member such that the bead is less flexible than the surrounding seal member and, therefore, resists occlusion of the nasal aperture.

The tether may be connected to the bead so that deformation forces are transmitted through the bead, to the tether, to one or both of the two cavity wall portions and then to the one or more preferential deformation regions.

The tether contributes to resisting deformation of the nasal aperture.

The mask frame may be co-operable with the seal member to separate the primary flow path from the exhaust flow path.

The mask frame may include a dividing wall which is co-operable with the cavity wall to separate the primary flow path from the exhaust flow path.

The mask frame may include a gas inlet that is opposite or substantially opposite the one or more first primary flow outlets such that the primary flow path is generally linear.

The mask frame may include a gas inlet arranged relative to the first primary flow outlets such that the respiratory gas undergoes small (0 to 5°) changes in direction between the gas inlet and the first primary flow outlets.

The mask frame may include a gas inlet arranged relative to the first primary flow outlet such that the primary flow path has a low resistance to gas flow. The first primary flow outlet may be opposite the gas inlet to define a primary flow path that is substantially straight.

The cavity wall may contact the mask housing.

The mask housing may include the exhaust vent and the cavity wall may be associated with the housing so that the exhaust cavity communicates with the exhaust vent.

The cavity wall may contact the mask housing so that the exhaust cavity communicates with the exhaust vent.

The exhaust vent may comprise a series of apertures in the cushion module arranged in a grouping.

The series of apertures may be in the mask housing.

The cavity wall may contact the mask housing to form a seal separating the exhaust cavity from the primary flow cavity.

The cavity wall may connect to the mask housing by over-moulding the cavity wall with the mask housing.

The mask housing may include a series of openings through which the cavity wall is able to be over-moulded to connect the cavity wall to the mask housing.

The series of openings may be disposed between a primary flow inlet and the exhaust vent in the mask housing.

The series of openings may form a U-shaped curve.

The series of openings may form a curved shape.

The seal member may be adapted to maintain a spaced relationship between the cavity wall and the nasal aperture when a deformation force is applied to the seal member.

The spaced relationship may be retained by linking a face-contacting portion of the seal member with the cavity wall.

The seal member may include a linking member extending from the face-contacting wall portion of the seal member to the cavity wall so that at least some of a deformation force applied to the face-contacting wall portion is directed to the cavity wall.

The face-contacting wall portion of the seal member may be a first wall portion between the nasal aperture and the oral aperture so that forces applied to the first wall portion are transferred to the cavity wall.

The face-contacting wall portion of the seal member may be a second wall portion of the seal member located between the nasal aperture and the exhaust vent so that forces applied to the second wall portion are transferred to the cavity wall.

The linking member may be connected to the first wall portion along a line of connection.

The line of connection may comprise at least 10% of the first wall portion distance measured between the nasal aperture and the oral aperture on the exterior of the seal member.

The line of connection may comprise at least 20% of the first wall portion distance measured between the nasal aperture and the oral aperture on the exterior of the seal member.

The linking member may be connected to the second wall portion along a line of connection.

The linking member may be connected to the cavity wall along a line of connection.

The one or more lines of connection may terminate at a location or at respective locations spaced from the rim of the nasal aperture.

The location or the respective locations may be spaced from the bead.

The linking member may be recessed from the nasal aperture.

The linking member may be recessed from the nasal aperture so that it does not contact the bead of the nasal aperture.

The deformation region may be interposed between first and second resilient regions.

The deformation region may comprise first and second resilient regions and a deformation panel disposed between the regions.

The deformation panel may have a thickness that is less than the thickness of the first and second resilient regions.

The deformation panel may comprise a first wall projecting from the first resilient region and a second wall connecting the first wall with the second resilient region.

The first and second resilient regions of the cavity wall may have a thickness that is at least three times the thickness of the first wall.

The second wall may have a curved profile from the first wall to the second resilient region to induce a rolling movement in the second wall to accommodate deformation in the deformation region.

The second wall may increase in thickness from the first wall to the second resilient region to cause initial folding of the first wall during deformation and subsequent rolling in the second wall starting from an intersection between the second wall and the first wall.

The intersection between the second wall and the first wall is configured to cause deformation to occur along the intersection.

The linking member may be connected with the second resilient region to direct deformation forces into the deformation region.

A notional line extending from an intersection line between the first resilient region and the first wall may converge at a pivot point with another notional line extending along the intersection line between the first wall and the second wall.

The first wall may project from the first resilient region to the intersection with the second wall by a distance in the range of 1 to 10 mm. The distance may in the range of 2 to 5 mm. The distance may be 3 mm.

The second wall may project from the second deformation resistant region by a distance in the range of 2 to 15 mm. The distance may be in the range of 2 to 10 mm.

The first resilient region may comprise a first thickened region of the cavity wall.

The first thickened region may comprise a rim adjoining the deformation region and a terminal panel extending from the rim and connected to the mask housing.

The terminal panel may have a thickness that is at least double the thickness of the first wall. The terminal panel may have a thickness that is in the range of 0.5 to 3 mm.

The second resilient region may comprise a load-spreading member which joins to a side of the deformation region opposite to the side that the first resilient region joins.

The load-spreading member may comprise a second thickened region of the cavity wall.

The load-spreading member may be formed as a rib along one side of the deformation region and may be connected to the linking member such that forces imparted on the face-contacting wall portion of the seal member are directed into the deformation region.

The second resilient region may extend laterally across the cavity wall at least the same distance as the width of the nasal aperture. Alternatively, the second resilient region may extend across the entire width of the cavity wall.

The second resilient region may taper to the same thickness of the surrounding cavity wall.

The second resilient region may taper at lateral ends to the same thickness of the surrounding cavity wall.

The cavity wall may be configured to channel respiratory gas from the primary flow cavity into the nasal aperture.

The cavity wall may include a deflector panel which is recessed from the rim of the nasal aperture and which forms a channel for the flow of respiratory gas from the primary flow cavity to the nasal aperture.

The linking member may connect the face-contacting wall portion with the deflector panel to direct forces into the deformation region.

The deflector panel may abut the second resilient region such that forces imparted on the linking member are transferred to the second deformation resistant region.

The deflector panel may connect with an inner wall of the seal member remote from the rim of the nasal aperture.

The deflector panel may connect with an inner wall of the seal member remote from the bead surrounding the rim of the nasal aperture.

The primary flow inlet of the mask housing may have one or more key formations.

The patient interface may include a socket insert with a formation that is complimentary to the one or more key formations of the mask housing to restrict rotation movement of the socket insert relative to the mask housing.

The socket insert may include a connecting portion that is configured to connect the socket insert with an inlet on the housing and with an outlet portion of a conduit-connecting elbow.

The mask housing may include headgear connectors.

Alternatively, the patient interface may include a frame that includes headgear connectors.

The frame may further include a formation that is configured to interact with the formation of the socket insert to rotationally lock the frame relative to the housing when the frame and socket insert are assembled with the housing.

The socket insert may include opposing formations between which the frame and the housing may be retained together.

The frame and the housing may be retained together in compression between the formations of the socket insert when assembled.

The frame may include an aperture which is configured to align with the exhaust vent of the mask housing to permit venting of respiratory gas through the frame to ambient environment.

The frame may be fastened to the mask housing. The frame may be fastened to the housing by gluing or by welding.

The cavity wall may be formed according to the twelfth aspect.

The primary flow cavity and the exhaust flow cavity may be within a cushion module formed by the seal member and a housing.

The cavity wall may be positioned relative to the nasal aperture and an oral aperture of the primary flow cavity to enable respiratory gas to flow from the primary flow cavity to the nares via the nasal aperture.

The cavity wall may be positioned relative to the nasal aperture and the oral aperture to enable exhaled respiratory gas from the mouth and nares to flow into the exhaust flow cavity.

The cavity wall may be at a recessed position relative to a rim of the nasal aperture.

The cavity wall may be positioned relative to the nasal aperture such that the primary flow and exhaust flow cavities are in communication with the nasal aperture.

An outlet of the primary flow cavity and an inlet of the exhaust flow cavity may be in communication with the nasal aperture.

The cavity wall may be configured so that the linking member directs forces imparted on the face-contacting wall portion into the deformation region of the cavity wall.

The exhaust vent may be formed as a series of apertures in the mask housing which are arranged in two or more separate groupings.

In one embodiment, the mask housing includes a cluster or grouping on each lateral side of the primary flow inlet and includes a series of openings extending about each grouping.

The series of openings may be arranged to form a V-shape or U-shape about each grouping.

The series of openings may form a W-shape.

According to a seventh aspect, there is provided a mask frame that is co-operable with a cushion module to form a pressurisable patient interface, the cushion module having a primary flow cavity for delivering respiratory gas to the mouth and nares of a patient and an exhaust flow cavity for transmitting exhaled respiratory gas from the patient interface and wherein the mask frame includes a respiratory gas inlet that is substantially aligned with a primary flow outlet of the cushion module which delivers respiratory gas to a mouth of the patient.

The mask frame may include a dividing wall that is co-operable with the cushion module to define separate primary flow and exhaust flow paths through an assembled patient interface.

The mask frame further may include vent holes to communicate exhaled gas from the exhaust cavity to the exterior of the mask frame.

In an eighth aspect, there is provided a mask frame for a patient interface, wherein the mask frame includes first and second respiratory gas flow channels that enable respiratory gas to flow from a gas source to a cushion module of a patient interface and includes a flow transfer valve that enables:
(a) respiratory gas to flow from the first respiratory gas flow channel to the second respiratory gas flow channel; or
(b) respiratory gas to flow from the second respiratory gas flow channel to the first respiratory gas flow channel; or
(c) respiratory gas to flow from the first respiratory gas flow channel to the second respiratory gas flow channel and from the second respiratory gas flow channel to the first respiratory gas flow channel.

The flow transfer valve may operate when the gas pressure in the first respiratory gas flow channel or the second respiratory gas flow channel exceeds a threshold gas pressure.

The threshold gas pressure may be the gas pressure in the first respiratory gas flow channel or the second respiratory gas flow channel when there is a full or partial obstruction of the first respiratory gas flow channel, the second respiratory gas flow channel, the flushing flow cavity or the nasal aperture.

The flow transfer valve may include an opening which is sealed by a resilient cover and the elasticity of the resilient cover is selected such that the resilient cover is deformable by gas pressure exceeding the threshold gas pressure to allow respiratory gas to pass through the opening when the gas pressure exceeds the threshold gas pressure.

The resilient cover may be a poppet valve.

The flow transfer valve may be incorporated into the mask frames that include at least first and second respiratory gas flow channels.

In an alternative form, the flow transfer valve may be incorporated into the dividing wall of a housing in a patient interface.

In a ninth aspect there is provided a patient-interface housing that comprises a transverse member with lateral sections which are spaced apart and which define a spacing that opens outwardly on at least one side.

The housing, according to this aspect, may be U-shaped, inverted U-shaped, V-shaped or H-shaped.

The housing may have a perimeter formation that permits fixing or connection of a resilient seal-forming seal member to form a cushion module that incorporates the housing.

The perimeter formation may comprise a series of holes which are dimensioned to permit fixing or connection of the resilient seal-forming seal member by over-moulding.

In a tenth aspect, there is provided a patient interface that includes a cushion module which comprises a housing according to the aspect disclosed above and a resilient seal-forming seal member that contacts the patient's face.

The resilient seal-forming seal member may be formed in accordance with any aspect disclosed above.

An eleventh aspect provides a cushion module for a patient interface, the cushion module comprising a first cavity, a second cavity, a nasal aperture and an oral aperture and wherein:
a. the first and second cavities are separated by a cavity wall that enables respiratory gas to flow within the cushion module between the first and second cavities when in use;
b. the first cavity is configured to communicate respiratory gas to both the mouth and the nares of a patient via the oral aperture and the nasal aperture respectively;
c. the cushion module comprises an exhaust vent to communicate respiratory gas from within the cushion module to externally of the cushion module; and
d. the second cavity is in communication with the exhaust vent.

The first cavity may be a primary flow cavity and the second cavity may be an exhaust cavity.

The cushion module may be configured to accelerate respiratory gas through the first cavity into the nares.

The first cavity may be configured to accelerate respiratory gas and to direct the accelerated respiratory gas toward the nasal aperture.

The first cavity may be configured with a taper that narrows toward the nasal aperture.

The taper may be formed between the cavity wall and a face-contacting wall portion that may be disposed between the oral aperture and the nasal aperture.

The cavity wall comprises a deformation region which preferentially deforms with respect to the remainder of the cavity wall when a deformation force is applied to the cavity wall.

The deformation region comprises a deformation panel that may be less resilient than the remainder of the deformation region such that the deformation panel preferentially deforms when a deformation force is applied to the seal member.

The deformation region further may comprise first and second resilient regions between which the deformation panel is disposed and wherein the resilient regions may direct a deformation force into the deformation panel to cause preferential deformation of the deformation panel.

The deformation of the deformation region may involve a reduction in the spacing between the first and second resilient regions and an associated deformation of the deformation panel to accommodate the reduction in spacing.

The deformation panel may include first and second walls that are adapted to deform in a predetermined sequence.

The predetermined sequence may include the deformation panel rolling over itself.

The predetermined sequence may include the second wall rolling over the first wall.

The first wall may project from the first resilient region in a first direction, the second wall projects from the second resilient region in a second direction different from the first direction and the first wall meets the second wall, and wherein the predetermined sequence may include the first wall being folded against the first resilient region.

The second wall may be configured to induce rolling of the second wall over the first wall.

The second wall may have a curved profile from the first wall to the second resilient region to induce a rolling movement in the second wall.

The second wall may increase in thickness from the first wall to the second resilient region to cause initial folding of the first wall during deformation and may cause subsequent rolling in the second wall starting from an intersection between the second wall and the first wall.

The deformation panel may have a wall thickness that is selected to induce deformation of the deformation panel in preference to the first and second resilient regions.

The first and second resilient regions may have a wall thickness that is at least three times the wall thickness of the deformation panel.

The cushion module may comprise a housing and a flexible seal member connected with a perimeter of the housing and wherein the seal member includes the cavity wall.

The cavity wall may connect with the housing interiorly of the connection between the perimeter of the seal member and the perimeter of the housing.

The housing may include the exhaust vent.

The cavity wall connection with the housing extends at least partially around the exhaust vent.

The exhaust vent may be bound by the cavity wall connection with the housing and a connection between the perimeter of the seal and the perimeter of the housing.

The seal member may be adapted to maintain a spaced relationship between the cavity wall and the nasal aperture when a deformation force is applied to the seal member.

The spaced relationship may comprise the cavity wall being recessed from a rim the nasal aperture.

The spaced relationship may further comprise the cavity wall being located such that first and second cavities open to the nasal aperture.

The seal member may be configured to direct at least some of a deformation force into the deformation region.

The cavity wall may be linked to the face-contacting portion of the seal member to maintain the spaced relationship between the cavity wall and the nasal aperture when a deformation force is applied to the seal member.

The seal member may include a linking member which connects the deformation region to a face-contacting portion of the seal member such that at least some of a deformation force applied to the face-contacting portion is directed to the deformation region.

The cavity wall may comprise a deflector panel adjacent to the nasal aperture, a main panel associate with the housing and the deformation region between the deflector panel and the main panel.

The deflector panel may be recessed from the rim of the nasal aperture and the linking member connects the face-contacting wall portion with the deflector panel to direct a deformation force into the deformation region.

An outlet from the first cavity to the nares and an inlet to the second cavity may form the nasal aperture.

The first cavity may be a lower cavity and the second cavity may be a upper cavity disposed above the first cavity.

The nasal aperture may be in communication with an outlet of the first cavity outlet and an inlet of the second cavity inlet.

In a twelfth aspect, there is provided a cavity wall for separating first and second cavities in a cushion module of a patient interface, the cavity wall having a deformation region that is adapted to preferentially deform under a deformation force applied to the cushion module.

The deformation region may comprise a deformation panel that deforms under a deformation force.

The deformation region may further comprise first and second resilient regions between which the deformation panel is disposed and wherein the resilient regions direct a deformation force into the deformation panel to cause preferential deformation of the deformation panel.

The deformation of the deformation region may involve a reduction in the spacing between the first and second resilient regions and an associated deformation of the deformation panel to accommodate the reduction in spacing.

The deformation panel may include first and second walls that are adapted to deform in a predetermined sequence.

The first wall may project from the first resilient region in a first direction, the second wall projects from the second resilient region in a second direction different from the first direction and the first and second walls have a connecting portion between them, and wherein the predetermined sequence may include the first wall being folded against the first resilient region.

The predetermined sequence may include the second wall buckling to accommodate the reduction in spacing between the first and second resilient regions.

The second wall may be configured to induce the buckling when the distance between the second resilient region and the connection portion is less than length of the second wall.

The second wall may have a curved profile from the connecting portion to the second resilient region to induce buckling of the second wall.

The second wall may increase in thickness from the connecting portion to the second resilient region to cause initial folding of the first wall during deformation and subsequent buckling in the second wall.

The deformation panel may have a wall thickness that is selected to induce deformation of the deformation panel in preference to the first and second resilient regions.

The first and second resilient regions may have a wall thickness that is at least three times the wall thickness of the deformation panel.

A thirteenth aspect provides a non-invasive patient interface that is configured to deliver pressurized respiratory gas to the mouth and nares of a patient, the patient interface having a cushion module that has first and second cavities with respective nasal and oral apertures which are configured to communicate respiratory gas with the mouth and nares respectively of a patient and wherein:
(a) the first and second cavities are separated by a cavity wall that enables respiratory gas to flow within the cushion module between the first and second cavities when in use; and
(b) the cavity wall is formed according to the twelfth aspect and is configured to direct external deformation forces on a face-contacting portion of the cushion module into the deformation region so that the cavity wall preferentially deforms in the deformation region.

The first cavity may be adapted to receive respiratory gas from a source and the second cavity may be adapted to vent respiratory gas from within the cushion module.

The cavity wall may be positioned relative to the nasal aperture and the oral aperture to enable respiratory gas to flow from the first cavity to the nares through the nasal aperture.

The cavity wall may be positioned relative to the nasal aperture and the oral aperture to enable exhaled respiratory gas from the mouth and nares to flow into the second cavity.

The cavity wall may be at a recessed position relative to a rim of the nasal aperture.

The cavity wall may be positioned relative to the nasal aperture such that the first and second cavities are in communication with the nasal aperture.

An outlet of the first cavity and an inlet of the second cavity may be in communication with the nasal aperture.

The cushion module may include a linking member extending from the face-contacting wall portion to the cavity wall so that at least some of the deformation force applied to the face-contacting wall portion is directed into the cavity wall.

The cavity wall may be configured so that the linking member directs forces imparted on the face-contacting wall portion into the deformation region of the cavity wall.

The linking member may be connected to the cavity wall along a first line of connection.

The linking member may be recessed from the nasal aperture

The face-contacting wall portion of the seal member may be a first wall portion between the nasal aperture and the oral aperture so that at least some of the forces applied to the first wall portion are directed into the cavity wall.

The linking member may be connected to the first wall portion along a second line of connection.

The second line of connection comprises at least 10% of the first wall portion distance measured between the nasal aperture and the oral aperture on the exterior of the seal member.

The second line of connection comprises at least 20% of the first wall portion distance measured between the nasal aperture and the oral aperture on the exterior of the seal member.

The face-contacting wall portion of the seal member may be a second wall portion of the seal member located between the nasal aperture and the exhaust vent so that forces applied to the second wall portion are transferred to the cavity wall.

The linking member is connected to the second wall portion along a third line of connection.

The second and/or third lines of connection may terminate at a location or at respective locations spaced from the rim of the nasal aperture.

The location or the respective locations may be spaced from a bead surrounding a rim of the nasal aperture.

The first cavity may be a lower cavity which is configured to communicate respiratory gas to both the mouth and the nares.

The cushion module may further comprise an exhaust vent to communicate respiratory gas from within the cushion module to externally of the cushion module

The second cavity may be an upper cavity disposed above the first cavity and may be in communication with the exhaust vent.

The cushion module may comprise a housing and a seal member and the cavity wall connects with the housing.

The housing may include the exhaust vent.

The cavity wall connection with the housing may at least partially surrounds the exhaust vent.

The exhaust vent may be bound by the cavity wall connection with the housing and a connection between the perimeter of the seal and the perimeter of the housing.

The cavity wall may further comprise a main panel which connects the housing to the first resilient region.

The cavity wall may further comprise a deflector panel which is recessed from the rim of the nasal aperture and which forms a channel for the flow of respiratory gas from the first cavity to the nasal aperture.

The deflector panel may abut the second resilient region such that deformation forces are directed to the second resilient region.

The deformation region may be arranged to structurally decouple the deflector panel from the main panel.

The invention provides a non-invasive patient interface that is configured to deliver pressurized respiratory gas to the mouth and nares of a patient, the patient interface comprising a cushion module which comprises:
(a) a seal for sealing around the mouth and nares of the patient;
(b) a housing connected to the seal;
(c) an interior volume defined by the seal and the housing; and
(d) the seal comprises a cavity wall located within the interior volume so as to define first and second cavities within the interior volume of the cushion module.

The seal comprises a preferential deformation region that comprises a deformation panel.

The deformation region is part of the cavity wall and the deformation panel futher includes first and second resilient regions between which the deformation panel is disposed.

Deformation of the deformation region may involve a reduction in the spacing between the first and second resilient regions and an associated deformation of the deformation panel to accommodate the reduction in spacing.

The deformation panel may include first and second walls and a connecting portion between the first and second walls.

The first wall may project from the first resilient region in a first direction, the second wall projects from the second resilient region in a second direction different from the first direction.

The second direction may be inclined downwardly from a plane intersecting the second resilient region and the connecting portion.

The connecting portion may have a bend profile which, at rest, aligns with the first direction of the first wall and aligns with an end of the second wall remote from the second resilient region.

The second wall may have a curved profile from the connecting portion to the second resilient region.

The second wall may increases in thickness from the connecting portion to the second resilient region.

The deformation panel may have a wall thickness that is less than the wall thickness of the first and second resilient regions.

The first and second resilient regions may have a wall thickness that is at least three times the wall thickness of the deformation panel.

The cavity wall may further comprise a main panel which connects the housing to the first resilient region.

The cavity wall may further comprise a deflector panel which is recessed from a rim of the nasal aperture and which forms a channel configured to direct respiratory gas from the first cavity to the nasal aperture.

The deflector panel may abut the second resilient region.

The deformation region may be arranged to structurally decouple the deflector panel from the main panel.

The cavity wall may be positioned relative to the nasal aperture and the oral aperture to enable respiratory gas to flow from the first cavity to the nares through the nasal aperture.

The cavity wall may be positioned relative to the nasal aperture and the oral aperture to enable exhaled respiratory gas from the mouth and nares to flow into the second cavity.

The nasal aperture may be defined at the seal by a rim and the cavity wall is recessed within the cushion module relative to the rim.

The cavity wall may be positioned relative to the nasal aperture to enable the first and second cavities to communicate respiratory gas with the nasal aperture.

An outlet of the first cavity and an inlet of the second cavity may be in communication with the nasal aperture.

The cushion module may further comprise (a) a face-contacting wall portion of the seal and (b) a linking member extending from the face-contacting wall portion to the cavity wall.

The linking member may brace the cavity wall in position relative to the nasal aperture and the face-contacting wall portion.

The linking member may be connected to the cavity wall along a first line of connection.

The linking member may be recessed from the nasal aperture

The face-contacting wall portion may be a first wall portion between the nasal aperture and the oral aperture.

The linking member may be connected to the first wall portion along a second line of connection.

The second line of connection may comprise at least 10% of the first wall portion distance measured between the nasal aperture and the oral aperture on the exterior of the seal member.

The second line of connection may comprise at least 20% of the first wall portion distance measured between the nasal aperture and the oral aperture on the exterior of the seal member.

The face-contacting wall portion of the seal member may be a second wall portion of the seal member located on an opposite side of the nasal aperture to the face contacting wall portion.

The linking member may be connected to the second wall portion along a third line of connection.

The second and/or third lines of connection may terminate at a location or at respective locations spaced from the rim of the nasal aperture.

The location or the respective locations may be spaced from a bead surrounding a rim of the nasal aperture.

The first cavity may be a lower cavity which is configured to communicate respiratory gas to both the mouth and the nares.

The cushion module may further comprise an exhaust vent to communicate respiratory gas from within the cushion module to externally of the cushion module.

The second cavity is an upper cavity disposed above the first cavity and may be in communication with the exhaust vent.

The housing may include the exhaust vent and the cavity wall connection with the housing at least partially surrounds the exhaust vent.

The exhaust vent may be bound by the cavity wall connection with the housing and a connection between the perimeter of the seal and the perimeter of the housing.

The exhaust vent may comprise one or more groups of apertures.

The first cavity may be configured to accelerate respiratory gas and to direct the accelerated respiratory gas toward the nasal aperture.

The first cavity may be configured with a taper that narrows toward the nasal aperture.

The patient interface according to any aspect may be adapted to connect to a flow generator to deliver respiratory gas from a flow generator to a cushion module and to transfer respiratory gas from the cushion module to the flow generator.

The patient interface may include an inlet path that is configured to deliver respiratory gas to the inlet of the cushion module and an exhaust path that is configured to receive respiratory gas from cushion module and wherein the inlet path and the exhaust path are co-axial.

The patient interface may include a co-axial conduit having an inner conduit and an outer conduit that surrounds the inner conduit, and the inner and outer conduits define flow paths for respiratory gas.

The inner conduit may define the inlet path and the outer conduit may define the exhaust path.

The patient interface may further include a mask frame which is adapted to extend the inlet path to the primary flow inlet of the cushion module and which is adapted to extend the exhaust path from the exhaust vent to the outer conduit.

The mask frame may have an inner duct which links the inner conduit to the primary flow inlet of the cushion module to deliver respiratory gas to the primary flow cavity and may have an outer duct surrounding the inner duct and which links the exhaust vent to the outer conduit.

The outer duct may include a connecting formation that is configured to seal with the cushion module in an area that overlaps with the exhaust vent.

The connecting formation may be a flange.

The cushion module includes an over-mould where the seal member is over-moulded to the mask housing and wherein the flange is configured to seal against the over-mould.

The patient interface may include a splitter which is configured to link the inner conduit to an inspiratory flow conduit of a flow generator and which is configured to link the outer conduit to an expiratory flow conduit of a flow generator.

The splitter may include one or more interface connections which are configured to interact with an expiratory flow conduit.

The patient interface may further include a bias flow vent in the exhaust path configured to vent respiratory gas to an ambient atmosphere.

The bias flow vent may be adjustable to vary the flow of respiratory gas to the ambient atmosphere.

The bias flow vent may be configured to vent 5 to 15 L/m.

The bias flow vent may be included in the splitter.

The bias flow vent may be configured to inhibit connection to a conduit. For instance, the bias flow vent may include one or more formations that provide a visual indication that a respiratory conduit should not be connected with the bias flow vent. Alternatively the one or more formations may inhibit a sealed connection with a respiratory conduit to prevent occlusion of the bias flow vent.

Alternatively, the bias flow vent may have a non-standard size or shape to indicate that a respiratory conduit should not be connected with the bias flow vent.

The bias flow vent may be configured to connect with a filter.

The patient interface according to any aspect may include an inlet channel that is configured to deliver respiratory gas to the primary flow cavity of the cushion module and an outlet channel that is configured to receive respiratory gas from cushion module and wherein the inlet channel and the outlet channel are configured at least partly as separate channels in a single conduit.

The outlet channel may be receive respiratory gas from the exhaust cavity of the cushion module.

The separate channels may be separated by a common dividing wall.

The separate channels may diverge at a distance remote from a cushion-module end of the frame to become separate conduits.

The conduit associated with the inlet channel may be connectable with the inspiratory flow conduit of a flow generator and the conduit associated with the outlet channel may be connectable with the expiratory flow conduit of a flow generator.

The patient interface may further include a bias flow vent in the exhaust path configured to vent respiratory gas to an ambient atmosphere.

The separate channels may form a combined opening at the cushion-module end of the frame with the dividing wall dividing the opening.

The dividing wall may be configured to interact with a cavity wall of the cushion module to form a seal that separates the inlet a channel from the outlet channel.

The dividing wall may form a transom that extends across the combined opening of the single conduit and both the dividing wall and the cavity wall may interact with the transom to form a seal that separates the inlet path from the exhaust path.

The patient interface may have a fitting member extending at least partly about the common opening and which is configured to couple the single conduit to the cushion module.

The fitting member may be configured for releasable coupling of the single conduit to the cushion module.

The patient interface may include headgear connectors.

The fitting member may partly comprise a sleeve surrounding an opening in the frame and which sleeve is configured to be coupled to the cushion module. The fitting member may further comprise an end of the single conduit that is adapted to couple with the sleeve.

The fitting member may include friction fit formations or interference fit formations or both for coupling with the single conduit with the sleeve or the sleeve with the cushion module.

The patient interface according to any aspect may include a first conduit that is configured to deliver respiratory gas to the primary flow cavity of the cushion module and a second conduit that is configured to receive respiratory gas from the cushion module and wherein the first and second conduits are spaced apart.

The first and second conduits may open into the cushion module at locations either side of the cavity wall.

The first conduit may be configured to open into the primary flow cavity and the second conduit may be configured to open into the exhaust cavity.

The patient interface may include a mask frame with respective openings through which pass the first and second conduits.

One or both of the first and second conduits may be connected to the cushion module to permit limited rotational movement of the one or both conduits relative to the cushion module.

The one or both conduits may be connected by a ball and socket joint.

The one or both conduits may comprise an elbow which includes a portion shaped as a spherical segment and may further comprise a socket which is adapted to couple with the cushion module and which is adapted to receive the spherical segment of the elbow to provide the limited rotational movement.

The first and second conduits may be coupled to the cushion module in a fixed orientation.

The first and second conduits may be coupled to a mask frame in a fixed orientation and the mask frame may provide respective respiratory gas flow paths between the cushion module and the first and the second conduits.

The patient interface may further include a bias flow vent in the second conduit configured to vent respiratory gas to an ambient atmosphere.

According to a fifteenth aspect, there is provided a method of delivering respiratory gas to a patient, the method comprising:
(a) delivering respiratory gas at an elevated pressure to a first cavity in a cushion module of a patient interface to supply pressurised respiratory gas to the mouth and nares of the patient from the first cavity; and
(b) accelerating the respiratory gas flow through a portion of the first cavity to deliver an accelerated respiratory gas flow to the nares of the patient.

The method may further comprise exhausting respiratory gas from a second cavity in the cushion module, the second cavity being in fluid communication with the first cavity.

Although various features are disclosed above in relation to one or more aspect, it will be appreciated that one or more features of one aspect may be combined with other aspects to arrive at additional embodiments. It follows that disclosure of features in the preceding statements should not be interpreted as meaning that the features are limited in application to the aspects in respect of which they are disclosed. For example, the deformation region may be incorporated into the patient interface of any aspect described above. As another example, the flow transfer valve may be incorporated into the patient interface of any aspect described above. As a further example, the patient-interface housing disclosed above may be incorporated into the patient interface of any one of the previous aspects. As a further example, the seal member disclosed above may be incorporated into the patient interface of any one of the previous aspects.

Ordinal references (e.g. first, second, third etc.) to aspects disclosed above serve to differentiate aspects from one another only. The ordinal references are not to be interpreted as the order of importance of the aspects.

### BRIEF DESCRIPTION OF THE DRAWINGS

The aspects of the patient interface disclosed above are described in detail below by reference to embodiments, which serve as examples only, and with reference to the accompanying drawings, in which:
Figure 1 is a front view of a sub-nasal patient interface having a seal, a housing and a mask frame and shows the ordinary location and orientation of the patient interface on a patient interface during use.
Figure 2 is an oblique front view of an embodiment of a patient interface according the first aspect disclosed above and including a mask frame.
Figure 3 is the same view of the patient interface shown in Figure 2 without the mask frame.
Figure 4 is an oblique view from above of the rear of the patient interface in Figure 1.
Figure 5 is a cross-sectional view of the patient interface in Figure 1 along the line A-A.
Figure 6 is an oblique view from above of the rear of an embodiment of a patient interface according to another embodiment of the first aspect described above.
Figure 7 is a view from above the seal of the patient interface in Figure 6 and showing a combined respiratory gas outlet to the nares formed by a flushing flow outlet and a primary flow outlet to the nares.
Figure 8 is side view of a cross-section of the patient interface in Figure 6 along the line A-A and with arrows indicating the primary and flushing flow paths.
Figure 9 is an oblique view from the rear of the cross-section shown in Figure 8 without the arrows.
Figure 10 is an oblique view from above of the rear of an embodiment of a patient interface without the mask frame according to the second aspect described above.
Figure 11 is an oblique cross-sectional view of the patient interface in Figure 10.
Figure 12 is a side view of the cross-section shown in Figure 11 with arrows indicating the flushing, primary and exhaust flow paths.
Figure 13 is an oblique view of the front of the patient interface shown in Figure 10.
Figure 14 is an oblique side view of an embodiment of a patient interface according to the third aspect disclosed above and without a mask frame.
Figure 15 is an oblique view from above of the rear of patient interface in Figure 14.
Figure 16 is an oblique view from slightly below the front of the patient interface in Figure 14.
Figure 17 is an oblique side view of the patient interface shown in Figure 14 with a mask frame.
Figure 18 is a cross-sectional view of the patient interface in Figure 17 along the line A-A with arrows indicating the flushing and primary flow paths.
Figures 19A and 19B are a front plan view and a rear oblique view of a housing according to an embodiment of the ninth aspect described above and shown in the mask in Figures 14 to 18.
Figures 20A is a cross-sectional view of a patient interface according to an embodiment of the first aspect disclosed above and Figure 20B is an enlarged view of a portion of the cross-section in Figure 20A and which shows the structure of the seal around the flushing flow outlet and the primary flow path outlet to the nares.
Figure 21 is a side cross-section view of the patient interface in Figure 20A with arrows indicating the flushing and primary flow paths.
Figures 22A to 22D show different seal structures around the flushing flow outlet and the primary flow path outlet to the nares.
Figure 23 is a side view of the patient interface in Figure 20A.
Figures 24A to 24D are cross-section views along the line A-A in Figure 23.
Figure 25 is a front view of another embodiment of a patient interface according to the first aspect disclosed above.
Figure 26 is a cross-sectional view along the line A-A in Figure 25.
Figure 27 is a cross-sectional view along the line A-A in Figure 25 with arrows indicating the flushing and primary flow paths.
Figure 28A is an oblique view, from an inlet end, of the conduit of the patient interface in Figures 26 and 27 and Figure 28B is a cross-sectional view of the conduit in Figure 28A.
Figures 29A and 29B are oblique views and cross-sectional views respectively of an alternative flushing flow channel.
Figures 29C and 29D are oblique view and cross-sectional views respectively of a further alternative flushing flow channel.
Figure 30 is an oblique side view of an embodiment of a patient interface according to the fourth and fifth aspects disclosed above, without a mask frame and with arrows indicating the flushing and primary flow inlets in the seal.
Figure 31 is an oblique view from above of the rear of the patient interface in Figure 30 with arrows indicating the flushing and primary flow outlets.
Figure 32 is a cross-section of the patient interface in Figure 30 along the line A-A.
Figure 33 is the same cross-section as in Figure 32, but is an oblique view from slightly below the patient interface.
Figure 34 is an oblique view from below of the cross-section in Figure 32 without the arrows.
Figure 35 is a top view of a cross-section along the line B-B in Figure 30.
Figures 35B and 35C are oblique rear views from above of a cross-section along the lines B-B and C-C respectively in Figure 30.
Figure 36 is an oblique view of the patient interface in Figure 30 with an embodiment of a mask frame according to the eighth aspect disclosed above.
Figure 37 is a cross-section view of the patient interface in Figure 36 along the line A-A with arrows indicating the flushing and primary flow paths through the patient interface.
Figure 38 is an underneath view of the mask frame in Figures 36 and 37.
Figure 39 is a cross sectional perspective view of the mask frame in Figure 38.
Figure 40 is an oblique view of the mask frame in Figure 39 without a pressure relief valve.
Figures 41A and 41B are cross-sectional views of the mask frame in Figure 40 with a pressure relief valve closed and opened, respectively.
Figures 42A and 42B are schematic cross-sectional views of the patient interface in Figure 37 when fitted to a patient to show a flow path of respiratory gas in the inhalation and exhalation phases of the breathing cycle when inhaling and exhaling through the nares.
Figure 43 is an oblique side view of an embodiment of a patient interface according to the sixth and seventh aspects disclosed above.
Figure 44 is a cross-section of the patient interface in Figure 43 along the line A-A.
Figure 45 is a schematic cross-sectional view of the patient interface in Figure 43 when fitted to a patient with arrows showing the flow of respiratory gas and showing flushing of anatomical dead space during exhalation through the nose when the mouth is open.
Figure 46 is a schematic cross-sectional view of the patient interface in Figure 43 when fitted to a patient with arrows showing the flow of respiratory gas and showing flushing of anatomical dead space during exhalation through the nose when the mouth is closed (although the drawing shows the mouth as being open, the drawing is to be interpreted as the mouth being closed).
Figure 47 is a schematic cross-sectional view of the patient interface in Figure 43 when fitted to a patient with arrows showing the flow of respiratory gas and showing flushing of anatomical dead space during exhalation through the mouth.
Figure 48 is an oblique front view of the mask frame on the patient interface in Figure 43.
Figure 49 is an oblique rear view of the mask frame on the patient interface in Figure 43.
Figure 50 is an oblique front view of an embodiment of a patient interface according to the sixth aspect disclosed above.
Figure 51 is a front view of the patient interface shown in Figure 50.
Figure 52 is a rear view of the patient interface shown in Figure 50.
Figure 53 is an exploded oblique front view of the patient interface shown in Figure 50.
Figure 54 is a cross-section the patient interface shown in Figure 51 along the line A-A.
Figure 55 is a magnified front oblique view of a cross-section along the line B-B of the seal only from the patient interface shown in Figure 51.
Figure 56 is a magnified top oblique view of a cross-section along the line C-C of the patient interface shown in Figure 51.
Figure 57 is a rear oblique view of the seal cross-section of the patient interface shown in Figure 55.
Figure 58 is a magnified view a seal and housing cross section along the line A-A of the patient interface shown in Figure 51.
Figure 59A, B and C are sequential cross-sections view of the patient interface shown in Figure 51 along the line A-A showing how a deformation zone deforms.
Figure 60 is an oblique top view of a cross-section of the seal in Figure 61 along the line D-D.
Figure 61 is a front view of a cushion module from the patient interface in Figures 50 to 53.
Figure 62 is a front oblique view of the cushion module in Figures 61.
Figure 63 is a front view of a housing which forms part of the cushion module shown in Figures 61 and 62.
Figure 64 is a rear view of the housing shown in Figure 63.
Figure 65 is a cross-sectional view of the housing in Figure 63 along the line E-E.
Figure 66 is a front view of a frame which forms part of the patient interface shown in Figures 50 to 53.
Figure 67 is a rear view of the frame shown in Figure 66.
Figure 68 is a cross-sectional view of the frame in Figure 66 along the line F-F.
Figure 69 is a rear oblique view of a socket insert which forms part of the patient interface shown in Figures 50 to 53.
Figure 70 is a front oblique view of a socket insert which forms part of the patient interface shown in Figures 50 to 53.
Figure 71 is another embodiment of a cushion module for a patient interface according to the sixth aspect described above.
Figure 72 is an oblique top view of a cross-section of the seal only in Figure 71 along the line H-H.
Figure 73 is a front view of a housing which forms part of the cushion module shown in Figure 71.
Figure 74 is a rear view of the housing shown in Figure 71.
Figure 75 is a front view of a frame that co-operates with the cushion module and the housing shown in Figures 71 to 74.
Figure 76 is a front oblique view of the cushion module shown in Figures 61 and 62 with a co-axial dual limb assembly.
Figure 77 is a rear view of a co-axial frame which forms part of the co-axial assembly shown in Figure 76.
Figure 78 is a cross-section of the co-axial assembly shown in Figure 76 along the line J-J.
Figure 79 is an oblique view of an upper part of the co-axial assembly shown in cross-section Figure 78 when coupled by the socket insert shown Figures 69 and 70 to the cushion module shown in Figures 61 and 62.
Figure 80 is an oblique view of a lower part of the co-axial assembly shown in cross-in Figure 78.
Figure 81 is an oblique view of the co-axial conduit connector which forms part of the co-axial assembly shown in Figure 76.
Figure 82 is a front oblique view of the cushion module shown in Figures 30 to 34 with an alternative dual limb assembly.
Figure 83 is a rear view of the dual limb assembly shown in Figure 82.
Figure 84 is an oblique view of the dual limb assembly and the cushion module shown in Figure 82 when coupled together in cross-section along the line K-K in Figure 82.
Figure 85 is a cross-section along the line K-K in Figure 82 of the dual limb assembly and the cushion module shown in Figure 82 when coupled together.
Figure 86 is an oblique front view of a patient interface comprising an alternative dual limb assembly coupled to the cushion module shown Figures 61 and 62.
Figure 87 is a cross-section of the patient interface in Figure 86 along the line L-L.

### DETAILED DESCRIPTION

Preferred embodiments of the present invention will now be described in the following text which includes reference numerals that correspond to features illustrated in the accompanying figures. Where possible, the same reference numeral has been used to identify the same or substantially similar features in the different embodiments. To maintain the clarity of the figures, however, all reference numerals are not included in each figure.

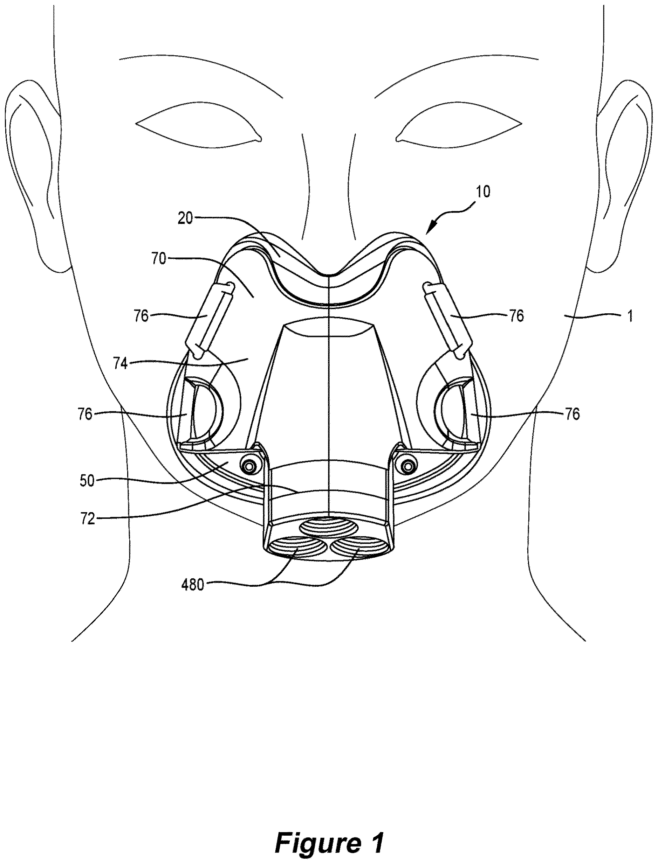

The aspects of the patient interface disclosed above will be described in detail below by reference to embodiments of a patient interface in the general form shown in Figure 1. The embodiments described below are variations on that general form. However, it will be appreciated that the scope of the aspects should not be limited by reference to that general form or to the specific embodiments described below and, instead, the aspects should be interpreted as relating to other forms of patient interface that also deliver pressurised respiratory gas to the patient, including patient interfaces that extend across the bridge of the nose, full-face masks and full-head helmets.

Some cross-sectional views of patient interfaces include arrows to indicate the flow of respiratory gas through the patient interface. The arrows should not be interpreted as vectors, meaning that the size of the arrows should not be interpreted as an indication of the volumetric flow rate, velocity or pressure of the respiratory gas at the location of the arrow. The arrows are a schematic indication of the flow direction of the respiratory gas at the location of the arrow.

The term "respiratory gas" as used throughout this specification is taken to mean a gas used in human respiration. The term "inhaled respiratory gas" as used throughout this specification is taken to mean respiratory gas that is inhaled during the inhalation phase of the breathing cycle. The term includes within its scope ambient air or air that is conditioned for treating a patient, such as having elevated humidity or oxygen levels or both compared to ambient air. The term "exhaled respiratory gas" as used throughout this specification is taken to mean respiratory gas that is exhaled from the lungs and airways of a patient. It, therefore, includes respiratory gas from the lungs and which gas occupies anatomical dead space at the end of the exhalation phase of the breathing cycle.

Having regard to Figure 1, the general form comprises a patient interface 10 in the form of a sub-nasal mask 10 that includes a cushion module 20, comprising a resilient seal member 12 and a housing 50 and the mask 10 further includes a mask frame 70 that connects to the cushion module 20. The cushion module 20 comprises a nasal portion and an oral portion. A conduit from a gas sources, such as a humidifier or ventilator, connects the mask frame 70 to deliver respiratory gas to the mask 10.

The mask frame 70 includes the central body portion 72 that includes one or more conduits for conveying respiratory gas from a gas source to the cushion module 20 and therefore to the patient. The mask frame 70 includes side wings 74 extending from the central body portion 72. Each side-wing 74 includes a pair of connectors in the form of bars 76 that are arrange to co-operate with headgear (such as resilient straps) for pulling the mask 10 into contact with the patient's face to form a substantially air-tight seal when respiratory gas at elevated gas pressure is delivered to the patient via the mask 10.

Having regard to the comments above regarding variations on the general form shown in Figure 1, one such variation of the general form, and which is applicable to the aspects and embodiments described below, is where the housing and the mask frame are formed integrally. In other words, the interface may include a unitary structure that performs the same function of the housing and mask frame as described in the following aspects and embodiments. It follows that, while the following aspects and embodiments describe the housing and the mask frame as being separate components of the patient interface, the description should be read as including the option of an integrally formed component that functions in the same way as the housing and mask frame.

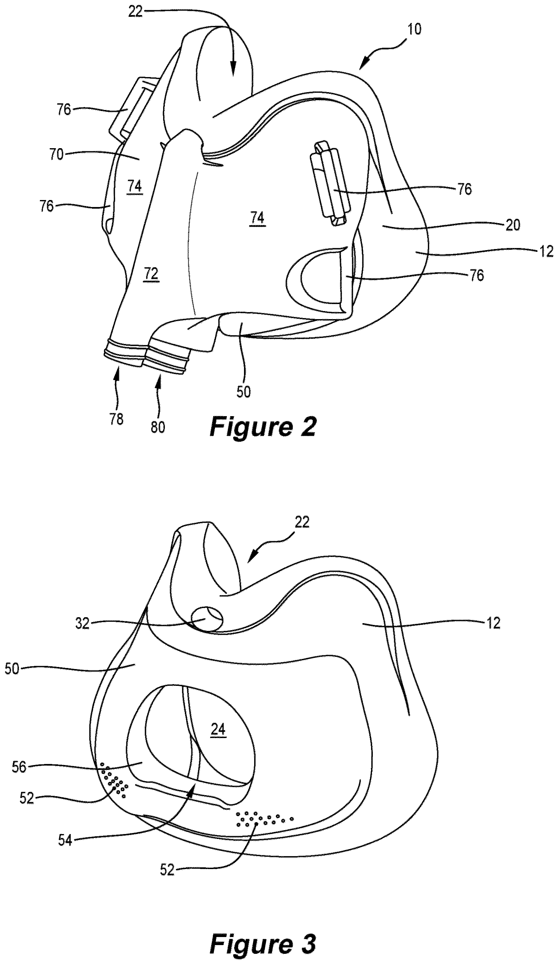

An embodiment of a mask 10 according to the first aspect is shown in Figures 2 to 5.

In this embodiment, the mask frame 70 includes a flushing flow channel 84 having a flushing flow path inlet 78 and a flushing flow channel outlet 86. The flushing flow channel 84 is tapered from the inlet 78 to the outlet 86 so that a supply of respiratory gas at a constant pressure is accelerated through the flushing flow channel 84 to provide a higher flow velocity at the outlet 86 than at the inlet 78.

The mask frame 70 also includes a primary flow channel 82 having a primary flow path inlet 80 and a primary flow channel outlet 88. The outlet end portion of the primary flow channel 82 is formed by a sleeve 90 which includes circumferentially located groove formations 92 on an exterior wall of the sleeve 90 for co-operating with a further sleeve 56 which defines the opening 54 in the housing 50. The sleeve 56 includes snap-fit formations on a radially inner wall which are co-operable with the snap-fit formation 92 of the sleeve 90 to form a snap fit connection between the two of them. The co-operable snap-fit formations 92 and 58 may provide a permanent connection between the mask frame 70 and the housing 50. Alternatively, the co-operable snap fit formations 92 and 58 may provide a releasable connection between the mask frame 70 in the housing 50, thereby enabling disassembly for replacement of parts of the mask 10 and for cleaning.

The housing 50 is formed of substantially rigid plastics material to provide a chassis for carrying or adding structural support to the cushion module 20. The opening 54 has a size and shape for receiving the sleeve 90 of the mask frame 70 and, in the embodiment shown in the figures, the sleeve 56 and the sleeve 90 have a corresponding non-circular profile that requires correct alignment of the mask frame 70 with the housing 50 before they can be fitted together. In this way, the mask frame 70 is correctly aligned with the housing 50 such that the flushing flow channel outlet 86 is received within the flushing flow cavity inlet 32 to enable communication of respiratory gas from the flushing flow channel 84 to a flushing flow cavity 28 provided in the seal member.

In an alternative embodiment, the sleeve 56 is configured to fit within the sleeve 90 of the mask frame 70. The sleeve 56 and the sleeve 90 also have a corresponding non-circular profile in this embodiment to ensure correct alignment of the mask frame 70 with the housing 50 before they can be fitted together.

The housing further includes two groupings of vent apertures 52 located below and slightly to the side of the opening 54. The vent apertures 52 enable exhaled respiratory gas to be vented externally of the mask 10. The housing in a further aspect includes a single set of vent apertures 52.

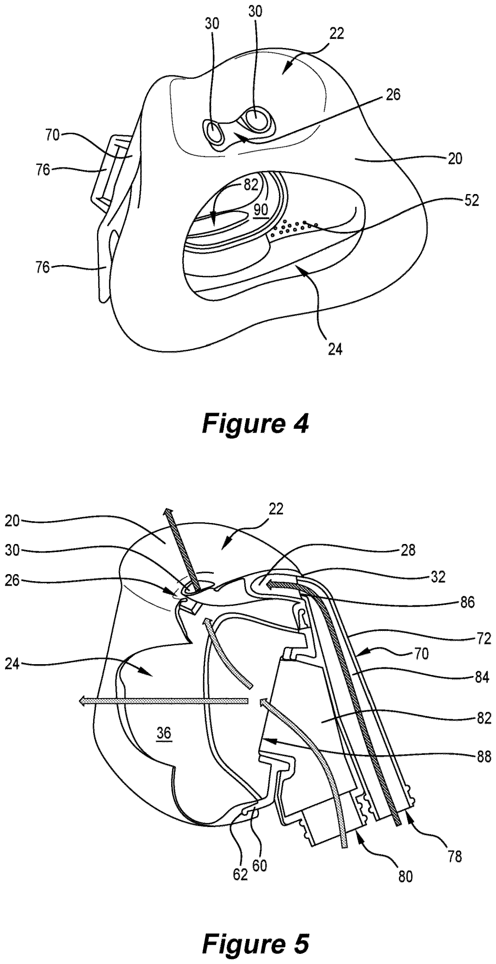

The housing 50 includes a series of tab members 60 which project outwardly around its perimeter, as seen in the cross-section view in Figure 5. The outer ends off the tab members 60 are linked to a bead 62 which runs continuously across all of the tab members 60, thereby forming a series of discrete windows just inside the perimeter of the housing 50. The seal member 12 is integrally formed with the housing by over-moulding a resilient material onto the housing 50 to fill the series of windows. Therefore, the tab members 60 and the bead 62 become embedded in the resilient material and are mechanically interlocked with the seal member 12. The seal member 12 and the housing 50, therefore, form a unitary cushion module 20 structure.

Seal member 12 is formed of soft, resilient material and includes an oral aperture 24 that, when fitted to a patient, circumscribes the patient's mouth and includes a nasal aperture 26 that is located in the valley of a nasal cradle 22 which is arranged to contact the underside of the patient's nose. The nasal aperture 24 is specifically located to align with the nares of the patient when the mask 10 is fitted to the patient to enable pressure therapy to be delivered via the nares. The seal member 12 further includes the flushing flow cavity 28. The flushing flow cavity 28 is integrally formed with the seal member 12. More specifically, the flushing flow cavity 28 is formed partly in the region of nasal cradle 22. In this particular embodiment, the flushing flow cavity is formed partly by a cavity wall 34 and partly by a wall of the seal member 12 in the region of the nasal cradle 22.

In this embodiment, the cavity wall 34 is shaped to provide a bifurcated flushing flow cavity 28 having a single inlet 32 which communicates with the flushing flow channel outlet 86 of the mask frames 70 and having two flushing flow outlets 30 (Figure 4). The flushing flow outlets 30 are flush with the nasal aperture 26. Furthermore, the flushing flow cavity 28 in a region immediately upstream of the flushing flow outlets 30, is turned upwardly to project respiratory gas upwardly into the nares of patient (Figure 5). Additionally, the flushing flow cavity 28 is tapered, such that the cross sectional area reduces along its length, to further accelerate the flushing flow of respiratory gas from an inlet and to the outlet end of the flushing flow cavity 28.

The seal member 12 and the housing 50 collectively define an interior volume which comprises first and second cavities, otherwise referred to herein as a primary flow cavity 36 and the flushing flow cavity 28 respectively. Respiratory gas delivered via the primary flow channel 82 flows into the primary flow cavity 36 where it is inhaled by the patient via the oral aperture 24 and/or via the nasal aperture 26. At the same time, respiratory gas is delivered via the flushing flow channel 84 to the flushing flow cavity 28 and is then delivered to the nares of the patient via the flushing flow outlets 30.

In operation, the patient is provided with the volume of respiratory gas through the primary flow cavity 36 that is sufficient to meet their tidal flow requirement during the inhalation phase. At the same time, a stream of flushing flow respiratory gas is provided via the flushing flow cavity 28 and may contribute to the tidal volume of respiratory gas required by the patient. The tidal flow is provided while maintaining a pressure above atmospheric pressure in the interface and lungs of the patient. Nevertheless, respiratory gas provided via the primary flow path generally is inhaled through the mouth via the oral aperture 24 or through the nares via the nasal aperture 26 or sometimes through both. During exhalation, while the gas pressure of the exhaled respiratory gas through the nares may exceed the gas pressure of the flushing flow, the exhaled respiratory gas enters the primary flow cavity 36 via either of the oral aperture 24 or the nasal aperture 26 and is vented externally of the mask via the vent apertures 52. Without wishing to be bound by this theory, the applicant believes that, as the gas pressure of the exhaled respiratory gas through the nares decreases towards the end of the exhalation phase, the gas pressure of the flushing flow will exceed the gas pressure of the exhaled respiratory gas through the nares at a point in the breathing cycle and, at that point, the flushing flow of fresh respiratory gas begins flowing through the nares. It is believed that the same applies when the patient is breathing through their mouth and nose. However, when the patient breathes only through their mouth, the flushing occurs throughout the whole exhalation cycle.

Without wishing to be bound by this theory, the applicant believes that the higher velocity flushing flow of respiratory gas into the nares flushes exhaled respiratory gas that remains in the nasal cavity, throat and mouth of the patient at the end of the breathing cycle when the air pressure from exhalation tapers off. The nasal cavity, throat and mouth can be collectively referred to as anatomical dead space of the patient. The flushing flow forces the exhaled respiratory gas into the primary flow cavity 636 whereon it is vented externally of the mask 10 via the vent apertures 52. The applicant believes that such flushing increases the overall oxygen intake in the next inhalation phase of the breathing cycle due to the reduction in rebreathing of exhaled respiratory gasses. The increase in oxygen intake as a result of anatomical dead space flushing is believed to improve the patient's respiration. In other words, the treatment described above is believed to make breathing easier and more effective for patients that suffer from obstructive respiratory diseases.

The applicant also believes that the dual-cavity patient interface, which accelerates one stream of respiratory gas into the nares to cause anatomical dead space flushing, enables the treatment therapy pressure applied through the patient interface 10 to be lower than the gas pressure applied through a typical patient interface during typical NIV treatment for the equivalent oxygen exchange due to the improved effectiveness of respiration occurring from an decrease of rebreathing of exhaled air. This effectively means the same gas exchange can be experienced by a patient at a relatively lower therapy pressure. Operating at lower gas pressures than the gas pressures used in existing NIV treatment is believed to potentially provide a considerable improvement in the effectiveness of treating obstructive respiration diseases because the lower gas pressures will reduce compliance problems and will also reduce the incidence of pressure sores. Alternatively, the patient interface according to embodiments disclosed herein can be used to enable higher oxygen exchange at the same NIV treatment pressure to thereby enable better treatment of the patient.

In a variation of this embodiment, the flushing flow path inlet 78 and the primary flow path inlet 80 may be combined into a single inlet in the mask frame 70. A partition located downstream of the single inlet designates a point at which the mask frame 70 separates into the separate flushing flow channel 84 and the primary flow channel 82. An advantage of this variation is that the patient interface 10 can be coupled to a single source of respiratory gas, such as a ventilator, flow generator, wall source of pressurised air or CPAP device that provides a single stream of respiratory gas. A further advantage is that a single conduit need only be connected to the patient interface which may reduce the perceived bulk of the therapy system.

Throughout the specification and claims, the term "flow generator" will be taken to include a flow generator, a ventilator, a CPAP device, a bi-PAP device, a VPAP device and a wall source of respiratory gas.

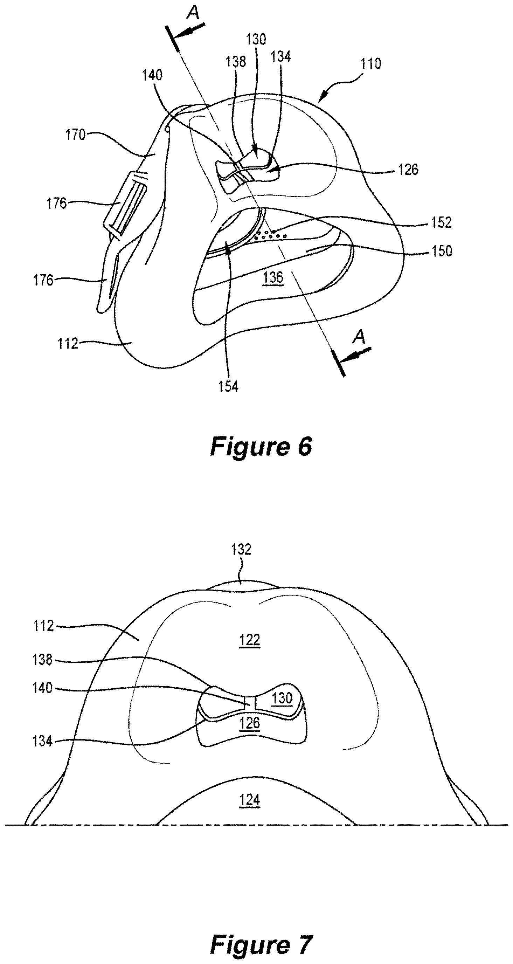

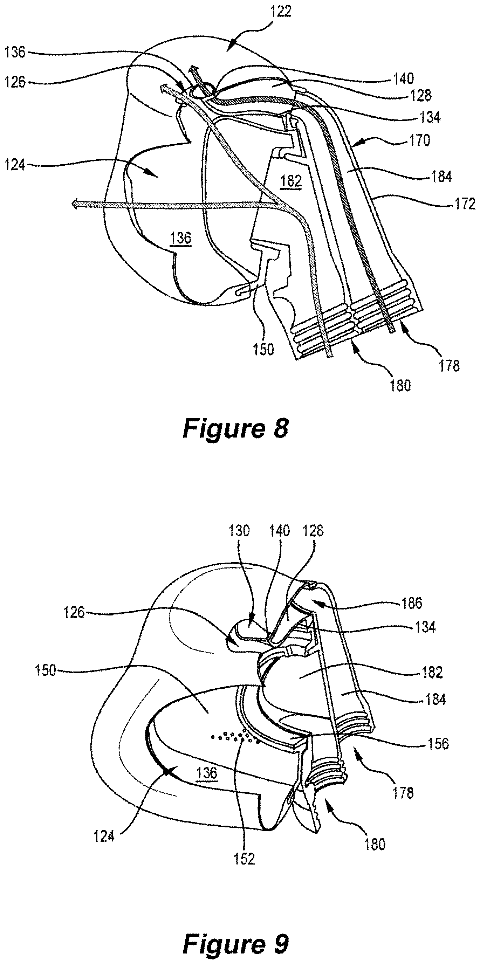

An embodiment of a mask 110 according to the second aspect is shown in Figures 6 to 9. In those figures, features that are the same as or similar to corresponding features in the first embodiment described above are denoted by like reference numerals preceded by the numeral "1".

The mask 110 forms first and second cavities in the form of a primary flow cavity 136 and a flushing flow cavity 128, respectively. The mask 110 differs from the mask 10 in that, instead of a bifurcated flushing flow cavity, the flushing flow cavity 128 is formed as a single passage from the flushing flow cavity inlet 132 to a single flushing flow cavity outlet 130. The outlet 130, as shown in figures 6 and 7, is defined partly by a distal portion of a rim 138 and partly by the cavity wall 134 terminating flush with the nasal aperture 126. The perimeter of the nasal aperture 126 is formed partly by the cavity wall 134 and partly by a proximal portion of the rim 138. Together, the outlet 130 and the nasal aperture 126 forms a combined respiratory gas aperture that is located in the seal member 112 to align with the nares when the mask 10 is fitted to a patient.

As with the mask 10, the cavity wall 134 of the mask 110 is shaped toward the outlet 30 such that respiratory gas is directed upwardly into the nares of a patient. This is provided by the cavity wall 134 being inclined upwardly toward the outlet 130 in a portion of the cavity wall 134 that is immediately upstream of the outlet 130.

The cavity wall 134 and the distal portion of the rim 138 together provide the outlet 130 with a narrow-waisted shape, such as a lemniscate, hippopede, figure eight or hourglass shape. This shape is specifically provided by a tether 140 which extends between opposite sides a lateral mid-point of the outlet 130. The tether 140 is flush with the nasal aperture 126. Additionally, its location means that it will generally align with the nasal septum when the mask 110 is fitted to a patient.

The tether 140 is integrally formed with the seal member 112 and, therefore, comprises the same resilient material. It will be appreciated that the tether 140 reduces the extent to which the outlet 130 is occluded when the mask 110 deforms to fit the contours of a patient's face. In other words, the tether 140 will reduce the extent to which the cavity wall 134 collapses toward the distal portion of the rim 138 to reduce the area of the outlet 130. It will also reduce the extent to which the cavity wall 134 can collapse toward the proximal portion of the rim 138 to reduce the area of the nasal aperture 126. If either were to occur, the effectiveness of the mask 110 to flush anatomical dead space and to permit inhalation through the nares would be reduced.

The tether 140 also counteracts a ballooning effect on the outlet 130 caused by the elevated pressure of the respiratory gas. The elevated gas pressure forces the cavity wall away from the rim 138 at the outlet 130, so without the tether 140, the outlet shape would not be retained. This means that the acceleration effect on the respiratory gas generated by the shape of the outlet 130 and the shape of the flushing flow cavity leading to the outlet 130 would be diminished.

In an alternative embodiment, the tether 140 may be recessed into the flushing flow cavity 128 to avoid contact with the patient when the mask 110 is fitted to a patient.

It will be appreciated that the flushing flow cavity 128 may be partitioned toward its outlet end so as to form adjacent outlets 130 which operate in effectively the same manner as the single outlet described above. In this variation, the partition or partitions may comprise the tether 140.

As with the mask 10, the flushing flow path inlet 178 and the primary flow path inlet may be connected to separate sources of respiratory gas. However, in a variation of this mask 110, both maybe connected to a single source of respiratory gas by either a bifurcated connection or any suitable form of path-splitting connection that allows one conduit to diverge into dual conduits.

In a further alternative variation, the flushing flow cavity inlet 132 may be located in the housing 150 so that both the primary flow channel 182 and the flushing flow channel 184 communicate respiratory gas via the housing 152 to the respective primary flow cavity 136 and the flushing flow cavity 128. In each case, the channels 182 and 184 connect with the housing 150 and/or seal member 120 in a sealed manner to enable pressurised respiratory gas to flow through the channels 182 and 184 and into the primary flow cavity 136 and the flushing flow cavity 128.

In the embodiments of the first and second aspects as described above, one benefit that results from integrating the flushing flow conduit and flushing flow outlet with the seal member is that, when the seal member deforms to fit the patient's facial features, such as when it is initially fitted or when it is adjusted, the flushing flow cavity and, therefore, the flushing flow outlet, generally follows the deformation of the seal member. This means that be patient's experience of the comfortable cushion module is maintained without interfering with the anatomical dead space flushing effect that it provides. As described above, another benefit is that the flushing flow of respiratory gas is believed to increase nasal dead space flushing which increases the efficiency of the treatment for patient suffering obstructive respiratory diseases.

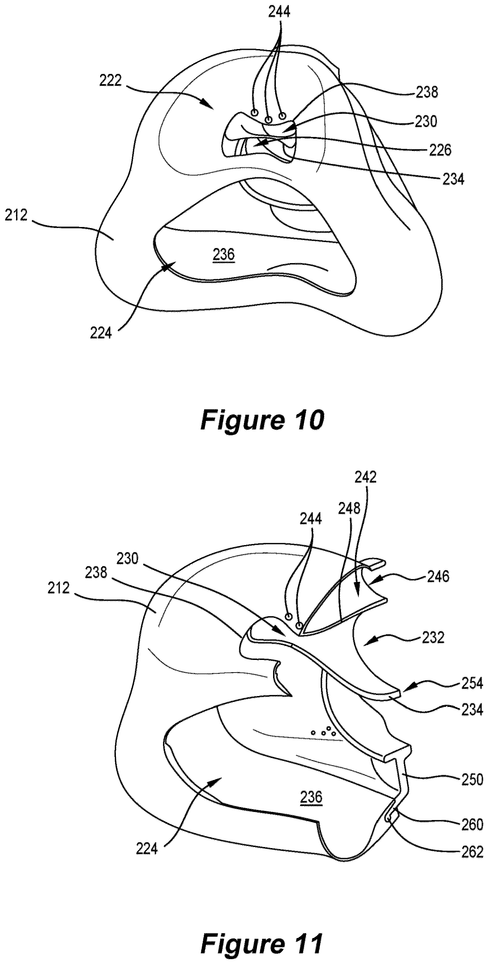

An embodiment of a mask 210 according to the third aspect is shown in figures 10 to 13. In those figures, features that are the same as or similar to corresponding features in the first embodiment described above are denoted with like reference numerals preceded by the numeral "2".

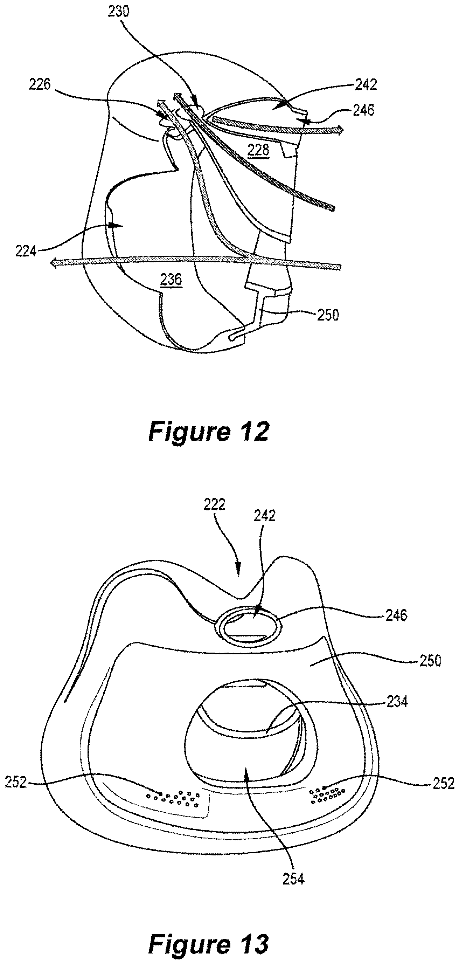

In the mask 210, the housing 250 is the same as the housing 150 in the embodiment of the mask 110 according to the second aspect described above. It includes first and second cavities in the form of a primary flow cavity 236 and a flushing flow cavity 228 respectively. Although not shown in the drawings, the mask 210 includes a mask frame that has a primary flow channel and a flushing flow channel, both deliver respiratory gas to an inlet 254 in the housing 250. Specifically, the primary flow channel delivers respiratory gas to a lower portion of the inlet 254 such that the respiratory gas flows into the primary flow cavity 236. The flushing flow channel delivers respiratory gas to an upper portion of the inlet 254 such that the respiratory gas flows into the flushing flow cavity 228 (see Figure 12).

Delivery of the respiratory gas via the inlet 254 is enabled by the cavity wall 234 extending across the inlet 254 of the housing (Figures 12 and 13). Accordingly, the flushing flow cavity inlet 232 is defined in part by the upper portion of the inlet flange 256 and in part by a distal end of the cavity wall 234. It can be seen in Figure 13 that the cavity wall 234 is inclined upwardly toward the nasal cradle 22 and terminates flush with the rim 238 of the seal member 212 to form the flushing flow outlet 230. As with the mask 110, the cavity wall 234 is shaped to form a volume that tapers inwardly toward the flushing flow outlet 234, thereby accelerating respiratory gas from the inlet 232 to the outlet 230.

The flushing flow outlet 230 has a lemniscate, figure eight or hippopede shape and is located immediately adjacent to an outlet to the nares from the primary flow cavity 236. Together, the nasal outlet 226 from the primary flow cavity 236 and the flushing flow outlet form a combined nasal aperture for delivering a primary flow of respiratory gas and a flushing flow of respiratory gas to the nares (Figures 10 and 12).

The mask 210 also differs in that it includes an exhaust flow cavity 242 that is formed in part by an exhaust cavity wall 248 (that separates the exhaust flow cavity 242 from the flushing flow cavity 228) and in part by the outer wall of the seal member 212. The exhaust flow cavity 242 has inlets 244 adjacent to the flushing flow outlet 230 so that the nares overlap the inlets 244, the flushing flow outlet 230 and the nasal outlet 226 when the mask 210 is fitted to a patient. The exhaust flow cavity 242 also has an outlet 246 in the seal member 212 and which outlet 246 is distal to the patient. The inlets 244 receive exhaled respiratory gas from the nares and vent it externally of the mask by allowing the exhaled respiratory gas to travel through the exhaust flow cavity 242 and travel out of the mask 210 via the outlet 246. In a further aspect outlet 246 may be in the form of a plurality of vent apertures. The proximity of the exhaust outlet 246 to the patient's nares creates a path of low resistance for exhaled air to be vented from the patient's nares to atmosphere thereby potentially increasing the efficiency of the dead space flushing.

In one variation of this embodiment, the inlets 244 may form part of the flushing flow path, for example the inlets 244 may be incorporated into the rim 238 or may be formed in the exhaust cavity wall 248. While Figure 10 shows the mask 210 having three inlets 244, it will be appreciated that in other variations of this embodiment, the mask 210 may have more or fewer inlets provided that they enable exhaled respiratory gas to enter the exhaust flow cavity 242. For example, one inlet 244 or two inlets 244 may be provided.

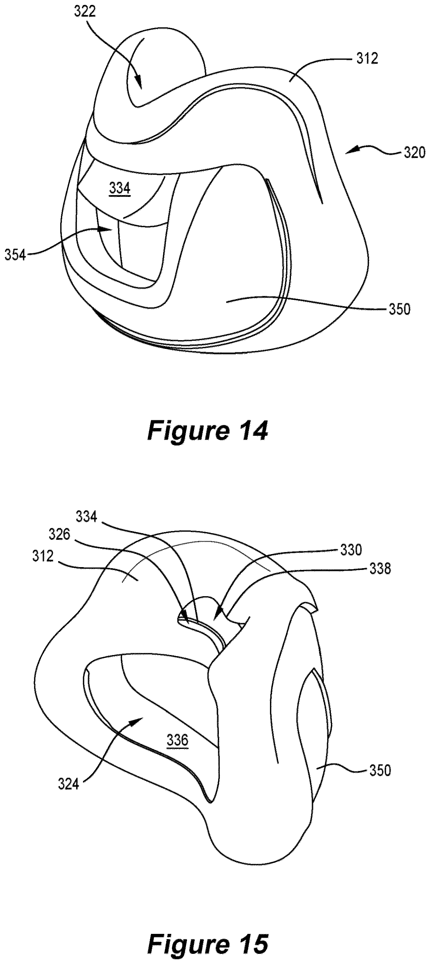

An embodiment of a mask 310 according to the fourth aspect is shown in Figures 14 to 18. In those figures, features that are the same as or similar to corresponding features in the first embodiment described above are denoted with like reference numerals preceded by the numeral "3".

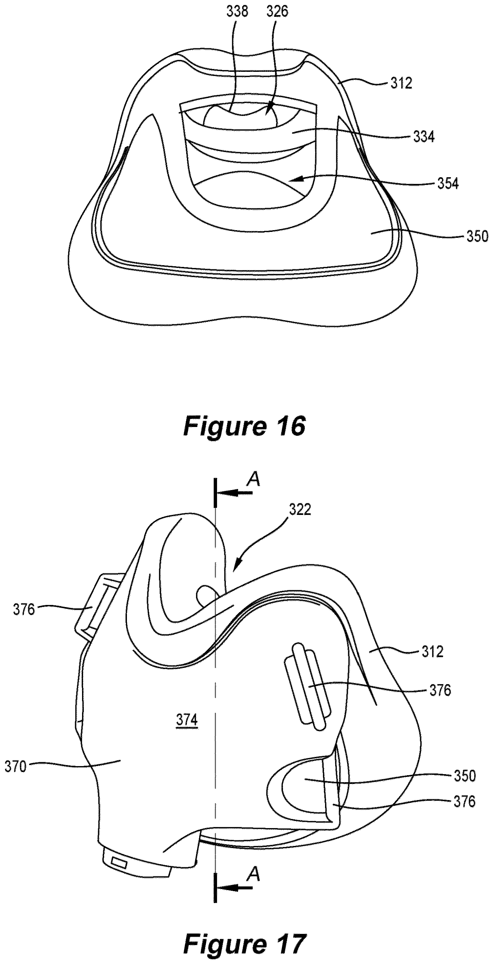

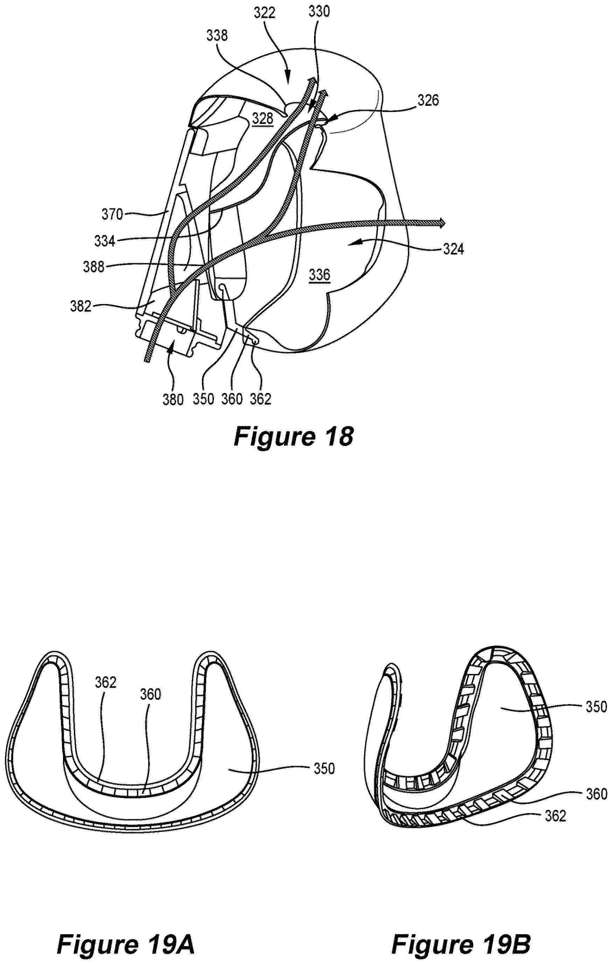

As with the mask 210, the mask 310 has a leading end of the cavity wall 334 which partitions the opening 354 so as to divide an incoming flow of respiratory gas between a first cavity and a second cavity, otherwise referred to herein as a primary flow cavity 336 and flushing flow cavity 328, respectively (Figures 14, 16 and 18). However, it is important to appreciate that the opening 354 is formed in the seal member 312, unlike in previous embodiment where the openings 54, 154, 254 are formed in the housing. The cavity wall 334 is inclined upwardly from the opening 354 (Figures 14 and 16) and terminates at a location short of a combined nasal aperture formed by the flushing flow outlet 330 and the nasal outlet 326 from the primary flow cavity 336 (Figures 15 and 18). This means that the flushing flow outlet 330 is formed partly by an upper rim of the cavity wall 334 and partly by the rim 338. However, the cavity wall 334 is connected to the wall of the cushion member at the rim 338 (Figure 15). Linking the cavity wall 334 at the rim 338 ensures that the cavity wall 334 moves with the rim 338 as the seal member 312 is adjusted and, therefore, the size of the nasal outlet 326 and the flushing flow outlet 330 is substantially maintained despite adjustments in the mask 310 which result in the contours of the seal member 312 changing to fit a patient. The linking also has the benefit of improving the structural stability of the seal member 312, thereby reducing the risk of occluding the nasal outlet 326 and the flushing flow outlet 330. Despite the similarities to the cavity wall 210 of the previous embodiment, the upper rim of the cavity wall 334 is recessed from and, therefore, is not flush with, the combined nasal aperture. However, as in previous embodiments, the cavity wall 334, in combination with the outer wall of the seal member 312, forms a tapered volume (see Figure 18) which will accelerate respiratory gas as it flows from the opening 354 to the flushing flow outlet 330.

Recessing the upper rim of the cavity wall 334 from the combined nasal aperture avoids contact with the septum of the patient and, therefore avoids irritation and improves patient comfort. Furthermore, the spacing between the upper rim and the nares forms a plenum chamber that enables smoother gas flow because there is a space for the gas to flow into as it exits the nares. By way of contrast, if the rim 338 of the combined nasal aperture contacted the nares, exhaled respiratory gas from the nares would be split by the rim 338 between the flushing flow cavity 328 and the primary flow cavity 336. With nasal exhalation, for example, the addition of the plenum chamber formed by the recessed upper rim of the cavity wall 334 means the exhaled respiratory gas would enter the plenum chamber and then flow into the primary flow cavity 336 without some respiratory gas being split-off and being sent into the flushing flow cavity 328.

As with other masks described above, the mask 310 has a primary flow cavity 336 which operates to deliver respiratory gas to the patient via an oral aperture 324 and a nasal outlet 326 (as shown in Figure 18 by the arrows indicating the flow of respiratory gas to the oral aperture 324 and the nasal outlet 326). The nasal outlet 326 is formed between the rim 338 and the cavity wall 334 on a proximal side of the seal member 312.

In contrast to the masks described above, the mask frame 370, while having the same cheek-side wings 374 and connector bars 376, includes only a primary flow channel 382 for communicating respiratory gas from a gas source to the opening 354. In view of the single flow channel in the mask frames 370, there is no flushing flow channel incorporated into the mask frame 370 because the flow of respiratory gas that passes into the flushing flow cavity 328 is delivered by the primary flow channel 382. The single flow channel simplifies the mask 310 and the connection between the mask frame 370 and the cushion module 320 and, therefore, is beneficial for example by reducing any risks associated with setting up the mask 310 correctly.

It will be appreciated that, with a single flow channel delivering respiratory gas into the primary flow channel 382 and the flushing flow channel 384, the resistance to flow in each of the flushing flow 328 and the primary flow cavity 336 is crucial to ensuring the appropriate delivery of respiratory gas at the pressure and velocity for achieving the desired treatment. In other words, the ratio of the resistance to flow through the flushing flow cavity 328 and the primary flow cavity 336 determines the split of respiratory gas flow between the two cavities. A corollary of this is that the flow can be set by designing the cavities with the required relative resistances to flow in each of the cavities. For the flushing flow outlet 330 and the nasal outlet 326, the flow resistance may be adjusted by changing the cross-sectional area through the flushing flow outlet 330 and the nasal outlet 326. The low flow resistance is also enabled by the low-angle flow direction changes (typically in the range of 0 to 20°) of the respiratory gas through the cushion module 320.

A significant difference between the mask 310 and the masks of previous embodiments is that the housing 350 which, as shown in figure is 19A and 19B, comprises a transverse member with lateral sections which are spaced apart and which define a spacing that opens outwardly on at least one side. As shown in Figures 19A and 19B, the housing 350 has a generally U-shaped form, but may alternatively have an inverted U-shaped, V-shaped or H-shaped form. As with previous embodiments, the periphery of the housing 350 includes a perimeter formation that permits fixing of a resilient seal member to form a cushion module that incorporates the housing. The perimeter formation comprises a series of holes which are dimensioned to permit fixing of the resilient seal member 312 by over-moulding. In this embodiment, the perimeter formation comprises a series of outwardly extending tab members 360 which, at their outer ends, support a continuous bead 362 which forms the perimeter of the housing 350. The tabs 360 and bead 362 form a series of holes, in the form of windows, over and through which seal member 312 is over-moulded to form a permanent connection, or interlock between the housing 350 and the seal member 312.

The U-shape of the housing 350 enables the opening 354 to be formed much larger than other embodiments (Figures 14 and 16). This means that there is a smoother transition of respiratory gas from the mask frame 370 into the flushing flow cavity 328 and the primary flow cavity 336 and, therefore, means that there is lower resistance to flow through both while also potentially reducing undesirable amounts of turbulence. The lower flow resistance is important for achieving the correct ratio of respiratory gas flow between the flushing flow cavity 328 and the primary flow cavity 336. The larger opening 354 also allows larger mould tool cores to be removed through the opening 354 when the seal member 312 is over moulded onto the housing 350 which is important when designing complex multi cavity silicone components for example. This enables considerably more flexibility with the shape of the cavities that can be formed within the cushion module to control the flow of respiratory gas.

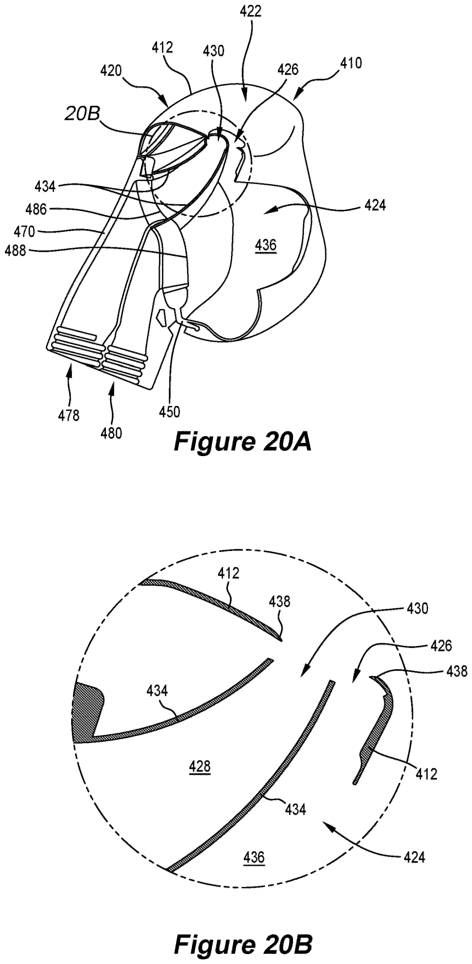

An embodiment of a mask 410 according to a variation of the first aspect is shown in Figures 20 to 24. In those figures, features that are the same as or similar to corresponding features in the first embodiment described above are denoted with like reference numerals preceded by the numeral "4".

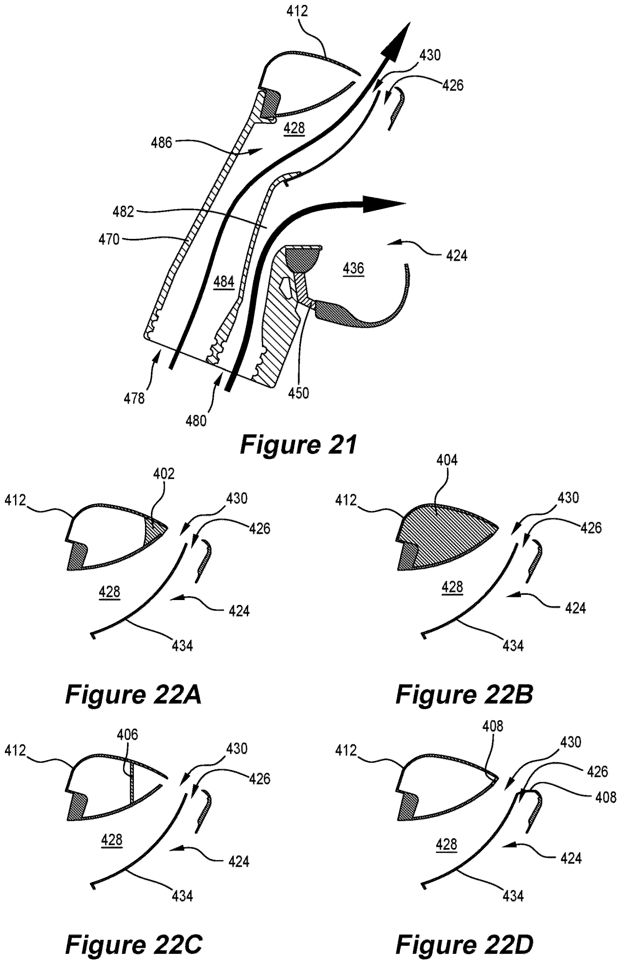

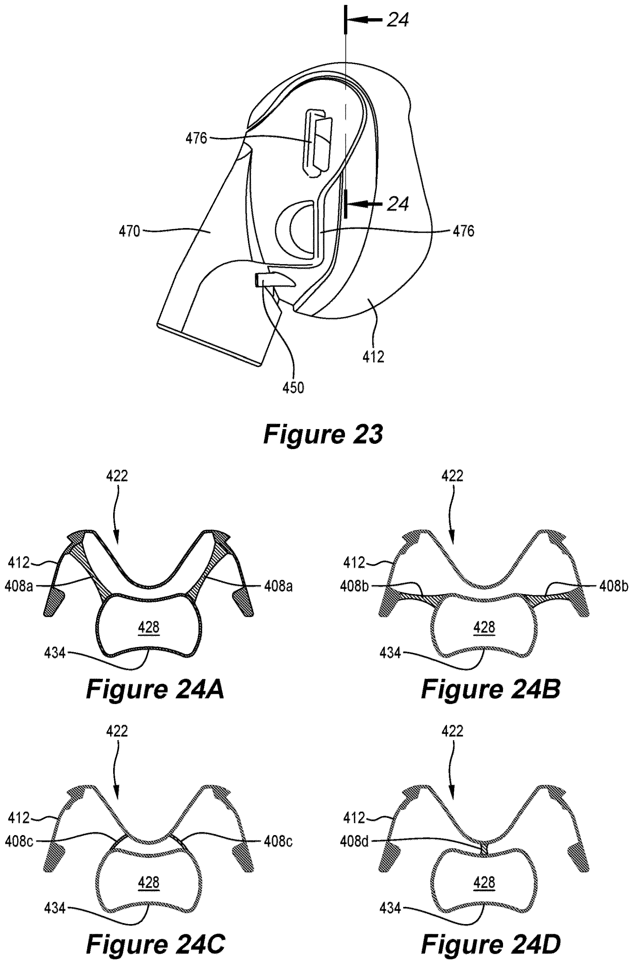

A cross-section of the mask 410 is shown in Figure 20A and front and side plan views of the mask 410 are shown in Figures 1 and 23 respectively. The housing 450 of the mask 410 is the same as the housing 350 of the mask 310, however, the seal member 412 differs in that the cavity wall 434 is formed as a conduit, in the form of a tube in this embodiment, which defines a flushing flow cavity 428 and which is surrounded by the primary flow cavity 436. The primary flow cavity 536 and the flushing flow cavity 428 respectively define first and second cavities. The flushing flow outlet 430 at the proximal end of the tube is not connected to the external wall of the cushion module 420 in the nasal cradle 422. In other words, the flushing flow outlet 430 is independently movable relative to seal member 412, and relative to the nasal outlet 426. This may provide a more comfortable mask or a mask which can cater to a wider range of facial geometries due to decoupling the movement between the nasal aperture 426 and the flushing flow outlet 430. The decoupling is shown more clearly in Figures 20B and 21 where the flushing flow outlet 430 is shown as recessed from and unconnected to the rim 438 of the seal member 412.

The flushing flow cavity 428 is integrally formed with the seal member 412 and is connected at its distal end to the seal member 412 where seal member 412 is over moulded onto the housing 50 (Figures 20A and 21).

The mask frame 470 includes two primary flow channels 482 (one associated with each inlet 480 when viewing Figure 21 in conjunction with Figure 1 which is a front plan view of the mask 410 shown in Figure 21) and a single flushing flow channel 484. Both primary flow channels 482 deliver respiratory gas into the primary flow cavity 436 and the single flushing flow channel 484 delivers respiratory gas into the flushing flow cavity 428. Of course, it is to be appreciated that the mask frame may have only a single primary flow channel. Figure 21 shows the flow of respiratory gas through the mask frame 470 and the cushion module 420, however, the figure is schematic and does not show one of the two primary flow channels 482. Alternatively, one of the primary flow channels 482 may be used to sample mask pressure while the other is used to deliver pressurised gas. Furthermore, in an additional embodiment one of the primary flow channels 482 may be used to deliver supplemental oxygen or a different gas mixture than that delivered through the remaining primary flow channel 482. Although Figure 21 shows a single arrow indicating respiratory gas flows through the primary flow channel 482 when into the primary flow cavity 36, it will be appreciated that the respiratory gas is subsequently delivered to a patient through the oral aperture 424, the nasal outlet 426 or both.

It will further be appreciated that the mask 410 operates in the same manner as the mask 10 to treat obstructive respiratory diseases by flushing anatomical dead space and by applying air pressure that is elevated above ambient air pressure to the patient's respiratory system.

Decoupling the flushing flow outlet 430 from the nasal cradle 422 avoids forming connections between the two areas that have a greater thickness of material than other areas. The areas of greater thickness are less flexible and, therefore, are less adaptable to facial geometries. This means that these stiffer regions can cause pressure sores or patient soreness when the mask 410 is worn for long periods of time. While decoupling the flushing flow outlet 430 from the nasal cradle 422 may improve patient comfort, the flushing flow outlet 430 will not track movement of the nasal outlet 426 so well. The variations shown in Figures 22 and 24 provide options that enable tracking (for the purposes of providing an effective treatment) and enable comfort that reduces the likelihood of patient soreness and pressure sores.

Figure 22 shows four options for support links between the flushing flow cavity 428 and the cushion module 420:
- Figure 22A shows a web 402 on an upper side of the flushing flow cavity 428 and extending in the distal direction from the rim 438 and the flushing flow outlet 430;
- Figure 22B shows a partition wall 404 on an upper side of the flushing flow cavity 428 and extending in the distal direction from the rim 438 and the flushing flow outlet 30 and extending the entire length of the flushing flow cavity 428;
- Figure 22C shows a single rib or tie 406 that connects the flushing flow cavity 428 to the nasal cradle 422 and which rib or tie 406 is located on an upper side of the flushing flow cavity 428 and is spaced in a distal direction from the rim 438 and from the flushing flow outlet 430; and
- Figure 22D shows two separate tethers 408 extending from the flushing flow outlet 430 in opposite directions towards and connecting with the rim 438.

Figure 24 shows examples of four different tether 408 configurations which may be used instead of or in combination with the support links shown in Figures 22A to 22D. The tethers 408 provide lateral, vertical or both lateral and vertical support for the flushing flow cavity 428. In particular:
- Figure 24A shows tethers 408a extending from reinforced shoulder portions 96 of the cushion module 420 to top corners of the flushing flow cavity 428. The tethers 408a taper outward from their midpoint to their ends.
- Figure 24B shows tethers 408b extending from side rib portions 98 of the cushion module 420 to top corners of the flushing flow cavity 428. The tethers 408b taper outward from their midpoint to their ends.
- Figure 24C shows tethers 408c extending from locations adjacent to the valley floor of the nasal cradle 422 to the top corners of the flushing flow cavity 428. The tethers 408c have a constant cross-section throughout their length.
- Figure 24D shows a short tether 408d which extends from a top mid-point of the flushing flow cavity 428 to the valley floor of the nasal cavity 422. The tether 408d tapers outwardly toward its ends.

It will be understood from the above options for support links and tether 408 configurations that the mask 410 may include one or more web members (such as the support links, the tethers or both) that link the flushing flow cavity 428 to the seal member 412 such that the flushing flow outlet 430 tracks the nasal outlet 426 when the cushion module is deformed, for example by fitting the mask 410 to a patient. In other words, the support links and tethers allow the flushing flow cavity 428 to track the nasal outlet 426 for different facial geometries. However, it is important to appreciate that the location and shape of the web members (such as the support links, the tethers or both) is selected to substantially retain the flexibility of the cushion module without the web members and avoid any areas of undesirably increased thicknesses which could lead to patient discomfort.

The tracking is achieved by the one or more web members (such as the support links, the tethers or both) linking to the cushion module 420 at locations that cause the one or more web members to impart a force on the flushing flow cavity 428 when the cushion module is deformed by the patient's nose during fitting or adjustment.

Figures 24A and 24B show one option for enabling tracking without increasing stiffness of regions of the seal member 412 that come into contact with the patient. Those figures show tethers 408a and 408b connecting with the reinforced shoulder 496 and side rib 498 portions of the cushion module. The selection of these tether points arises from the cushion module 420 including flexible regions which adapt to the shape of a patient's face and relatively inflexible structural regions that support that the flexible regions. The structural regions, namely the shoulder 496 and the side rib 498, in these variations comprise a portion of the cushion module 420 that is attached to the housing 450 via over-moulding. That is, the portion of the cushion module 420 in which the tab members 460 and bead 462 are embedded. It will be appreciated, however, that other structural regions may be used as anchor points for tethers and support links.

In variations of this embodiment, the one or more web members (such as the support links, the tethers or both) may be linked to the flexible regions of the cushion module 420 which adapt to the shape of the patient's face.

An embodiment of a mask 510 according to a further variation of the first aspect is shown in Figures 24 to 29.

Seal member 512 is formed of soft, resilient material and includes an oral aperture 524 that, when fitted to a patient, circumscribes the patient's mouth and includes a nasal aperture 526 that is located in the valley of a nasal cradle 522. The nasal aperture 526 is specifically located to align with the nares of the patient when the mask 510 is fitted to the patient. An opening 554 is formed in the cushion module 520 opposite the oral aperture 524 for communicating respiratory gas into or through the cushion module 520. The seal member 512 is permanently fixed to a housing 550 which is in the same form as the housing 450 described above and shown in Figures 19A and 19B. The cushion module 520 defines a primary flow cavity 536 (i.e. a first cavity) through which respiratory gas is communicated to the oral aperture 524 and the nasal aperture 526.

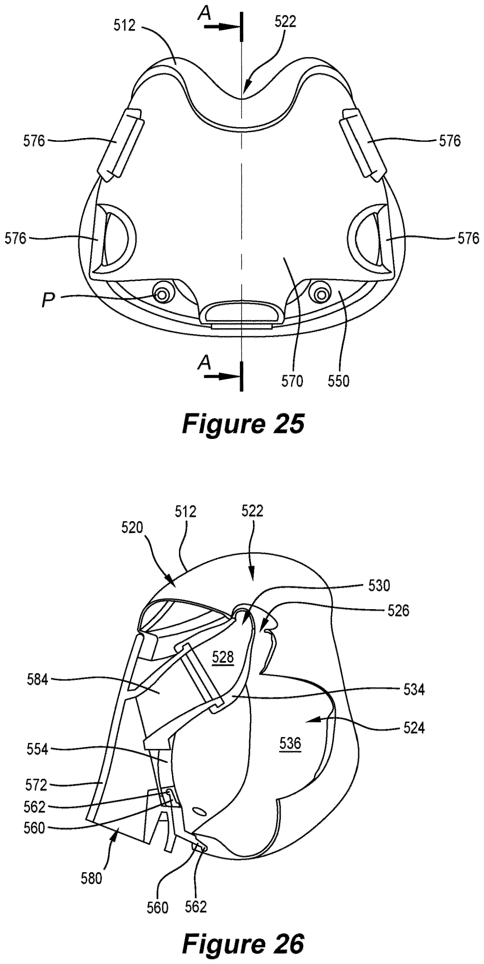

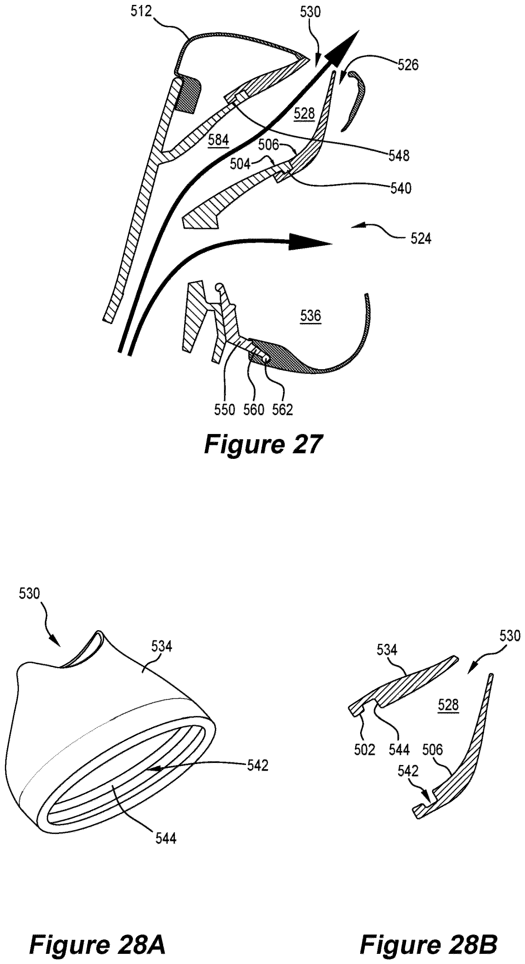

The mask 510 further includes a mask frame 570 which has side wings 574 and connector bars 576 and has a single primary flow path inlet 580 for communicating respiratory gas to a downstream primary flow channel outlet 588 which delivers respiratory gas via the opening 554 into the primary flow cavity 536. The mask 510 also has a flushing flow channel 584 downstream of the primary flow path inlet 580 for communicating respiratory gas via a flushing flow path inlet 578 to a flushing flow channel 584 and a subsequently into a flushing flow cavity 528 (i.e. a second cavity) as shown in Figures 26 and 27. The flushing flow channel 584 is formed as a tube which extends in a proximal direction away from a body 572 of the mask frame 570. When the mask frame 570 is fitted to the cushion module 520, the flushing flow channel 584 projects inside the cushion module 520 in a direction toward the nasal aperture 526.

The flushing flow cavity 528 (see Figures 26 and 27) is formed separately of the mask frame 570 and cushion module 520 and is connected to the mask frame 570 at the flushing flow channel outlet 586. The flushing flow cavity 528 is formed of resilient material that is soft and pliable so that the relatively thin-walled outlet 530 can deform readily to adapt to different facial geometries. By contrast, the inlet 532 has a thicker wall and, therefore, is less flexible and forms a firm connection with the mask frame 570 so that deformation of the outlet 530 will not cause the flushing flow cavity 528 to disconnect from the flushing flow channel 584. While flushing flow channel 584 has a generally uniform cross-section throughout, the flushing flow cavity 528 defines a passage which tapers inwardly from its inlet 532 to the outlet 530 to accelerate respiratory gas and to direct the respiratory gas into the nares of a patient.

The flushing flow cavity 528 and the flushing flow channel 584 have co-operable formations Figures 27 and 28), such as snap-fit formations, that enable the flushing flow cavity 528 to be fitted to the flushing flow channel 584 such that the outlet 530 is flush with or slightly recessed from the nasal aperture 526 (Figure 27). According to this embodiment, the co-operable formation on the flushing flow channel 584 comprises a flange 540 that projects radially outwardly from and at least partly about the outlet 586 of the flushing flow channel 584. An inner wall of the flushing flow cavity 28 has a flange-receiving groove 542 adjacent the inlet 532. The groove 542 has a profile that complements the profile of the flange 540 so that they fit together to securely couple the flushing flow cavity 530 to the flushing flow channel 584.

In one variation, the co-operable formations may comprise a barbed fitting of the flushing flow channel 584 and an inlet end to the flushing flow cavity 528 that is closely dimensioned to the barbed fitting such that the inlet end must be resiliently deformed to receive the barbed fitting. In another variation, the inlet end of the flushing flow cavity 528 may be over-moulded about a rigid connection member, such as a ring, which snap-fits with a co-operable formation on the flushing flow channel 584. A range of alternative co-operable formations may be used provided that they result in a substantially gas-tight connection, at elevated gas pressure, between the flushing flow cavity 528 and the flushing flow channel 584.

While the shape of the groove 542 and the flange 540 may take any suitable form that provides a secure connection, in this embodiment the groove 542 is shaped to limit the extent to which the flushing flow cavity 528 can be fitted onto the flushing flow channel 584 by limit wall 544 which abuts an end wall 546 of the flushing flow channel 584. The limit wall 544 thereby ensures proper alignment of the flushing flow cavity 528 to direct respiratory gas into nares of a patient when the interface is fitted to a patient.

The co-operable formations further include a recess 548 which is adjacent and distal to the end wall 546. The flange-receiving groove 542 defines a radially inwardly directed lip 502 which latches into the recess 548 when the flange 540 is seated in the groove 542. This arrangement ensures proper alignment of the flushing flow cavity 528 so that it directs respiratory gas into nares of a patient when the mask 510 is fitted to a patient.

To assist with reducing resistance to flow of respiratory gas an inner wall of the flushing flow channel 584 is flush with an inner wall of the flushing flow cavity 528 at the point where the flushing flow cavity 528 connects to the flushing flow channel 584.

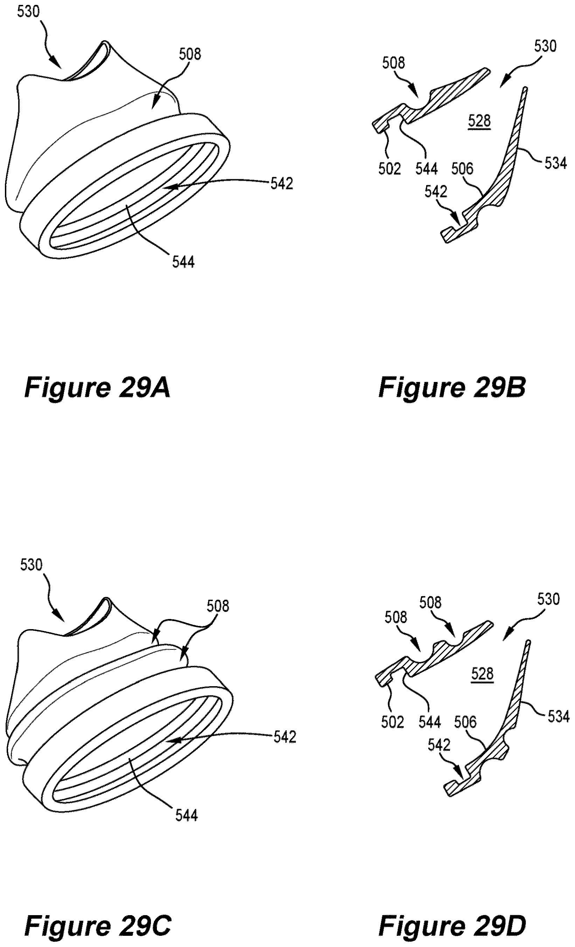

In a variation of this embodiment, the profile of the flushing flow cavity 528 includes one or more preferential deformation zones, in the form of bands 508 of reduced wall thickness (as shown in Figures 29A to 29D) or in the form of areas of reduced thickness in alternative embodiments, remote from the outlet 530 to enable the flushing flow cavity 528 to track movement of the nasal aperture 526 whilst substantially maintaining the shape of the flushing flow outlet 530. Locating the bands 508 close to the inlet end of the flushing flow cavity 528 means that deformation occurs in the region where the cross-sectional area of the flushing flow cavity 528 is large compared to downstream regions where the taper of the flushing flow cavity 528 means that they have a smaller cross-sectional area. The bands 508, therefore, reduce the likelihood that the flushing flow cavity 528 will be occluded when it deforms to track the nasal aperture 526.

The variation shown in Figures 29A and 29B shows a flushing flow cavity 528 with a single band 508. The band 508 comprises a region of reduced thickness of the cavity wall 534, compared to the wall 534 thickness of adjacent areas. Another variation shown in Figures 29C and 29D includes two bands 508. While the bands 508 have a curved profile, it will be appreciated that the bands 508 may have other profiles that permit preferential deformation at the location of the band. For example, an alternative profile is a square profile. The location of the bands 508 on the exterior of the flushing flow cavity 528 ensures that the inner wall 506 remains smooth and, therefore, provides a low flow-resistance flow path through the flushing flow cavity.

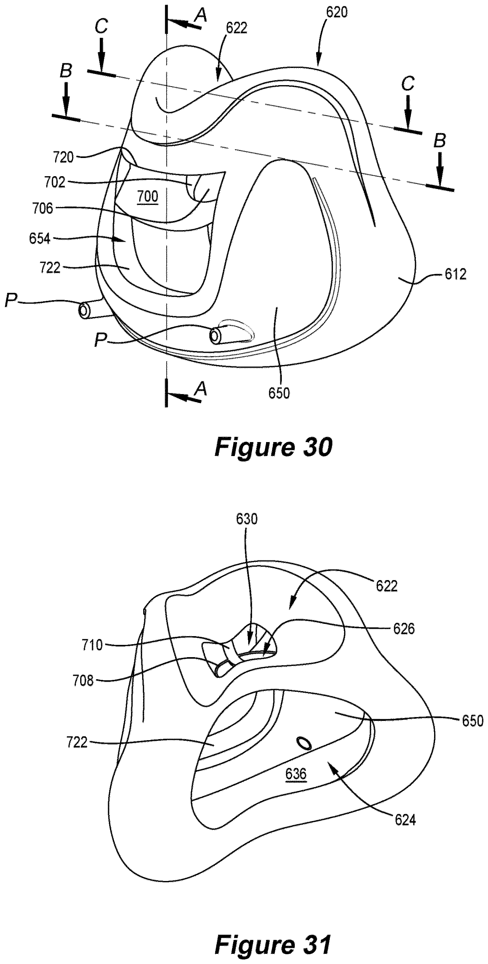

An embodiment of a mask 610 according to the seventh and eighth aspects is shown in Figures 30 to 42.

The mask 610 includes a seal member 612 that is permanently fixed to a housing 650 (in the same form as the housing shown in Figures 19A and 19B) by over-moulding to form a cushion module 620. The seal member 612 is formed of soft, resilient material, such as silicone, and includes an oral aperture 624 that, when fitted to a patient, circumscribes the patient's mouth and includes a combined nasal aperture 626, 630 that is located in the valley of a nasal cradle 622. The combined nasal aperture comprises the adjacently located primary flow outlet 626 and flushing flow outlet 630. A cavity wall 634 is formed inside the seal member 620 to define first and second cavities, i.e. a primary flow cavity 636 and a flushing flow cavity 628 respectively. The flushing flow cavity 628 is formed in an upper portion of the seal member 620 and the primary flow cavity 636 is formed within the remainder of the seal member 620 in combination with the housing 650. The nasal outlet 626 is specifically located to align with the nares of the patient when the mask 610 is fitted to the patient. Furthermore, the cavity wall 634 is located to enable respiratory gas from the flushing flow cavity 628 to enter the nares and to enable exhaled gas to exit the nares into the primary flow cavity 636. Furthermore, the cavity wall 634 is located to enable excess respiratory gas from the flushing flow cavity 628 to pass between the cavity wall and patients face to enter the primary flow cavity 636 and be vented to atmosphere through exhaust vents in the primary flow cavity 636.

An opening 654 is formed in the seal member 612 opposite the oral aperture 624 for communicating respiratory gas into or through the cushion module 620. Together, the seal member 612 and the housing 650 define the primary flow cavity 636 through which respiratory gas is communicated to the oral aperture 624 and the nasal outlet 626.

The housing 650 includes two pressure ports (P) that enable gas pressure measurement within the mask 610 when it is fitted to a patient. The housing further includes bias vents 652 for transmitting exhaled respiratory gas from within the mask 610 to outside of the mask 610. The bias vents 652 are in the same form as the bias vents 52 disclosed above.

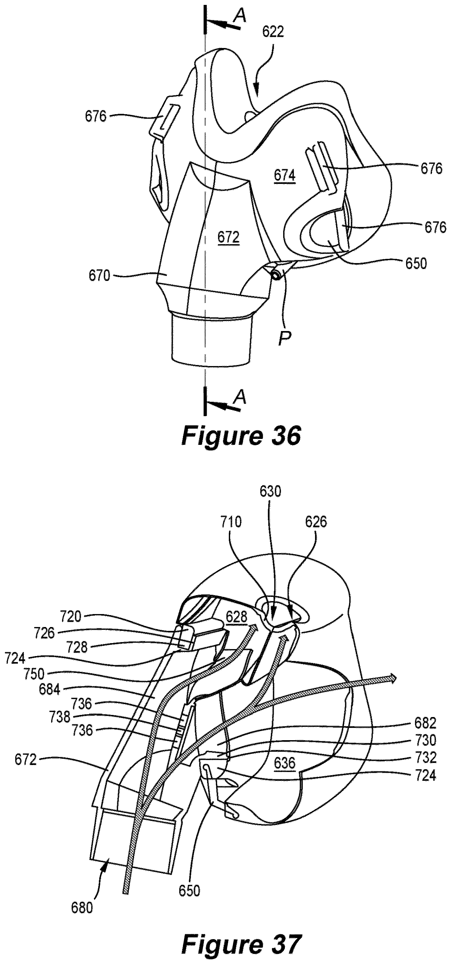

The mask 610 further includes a mask frame 670 (see Figures 36 to 41) which has side wings 674 and connector bars 676 and has a single primary flow path inlet 680 for communicating respiratory gas to a downstream primary flow channel outlet 688 which delivers the respiratory gas via the opening 654 into the primary flow cavity 636. The mask 610 also has a flushing flow channel 684 downstream of the primary flow path inlet 680 for communicating respiratory gas via a flushing flow path inlet 678 to a flushing flow channel 684 and a subsequently into the flushing flow cavity 628 (as shown in Figure 37).

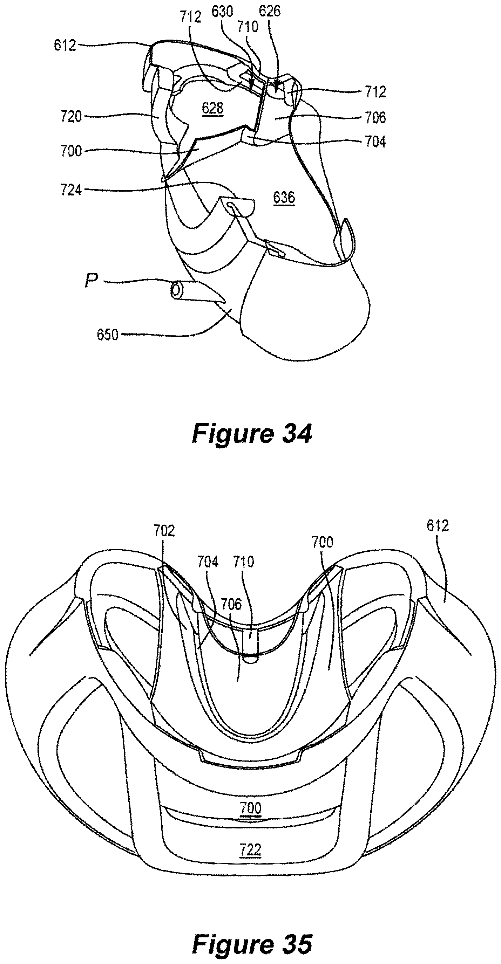

As shown in Figures 30 to 35, the cavity wall 634 includes one or more preferential deformation regions which accommodate deformation of the cavity wall 634 without occluding the flushing flow cavity 628. Specifically, the cavity wall 634 comprises a series of panels that enables preferential deformation of the cavity wall 634 in a way that reduces the likelihood of the flushing flow cavity 628 being occluded. Specifically, the cavity wall 634 comprises a main panel 700, a deflector panel 706, a transition panel 702 and a shear panel 704 (see Figures 35A to 35C). The main panel 700 extends to the opening 654 and partitions it to form the flushing flow cavity inlet 632 and an inlet to the primary flow cavity 636. The main panel 700 is configured to interact with the mask frame 670 to substantially isolate the primary flow cavity 636 from the flushing flow cavity 684 at the location where the mask frame and cushion module are attached. The cavity wall 634 is curved upwardly away from inlet 632 toward the sides of the seal member 620 (as shown in Figures 30 and 32 to 34).

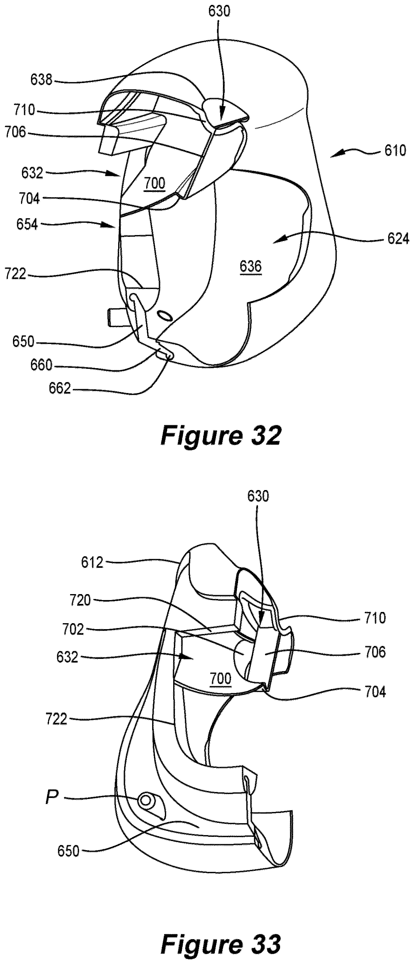

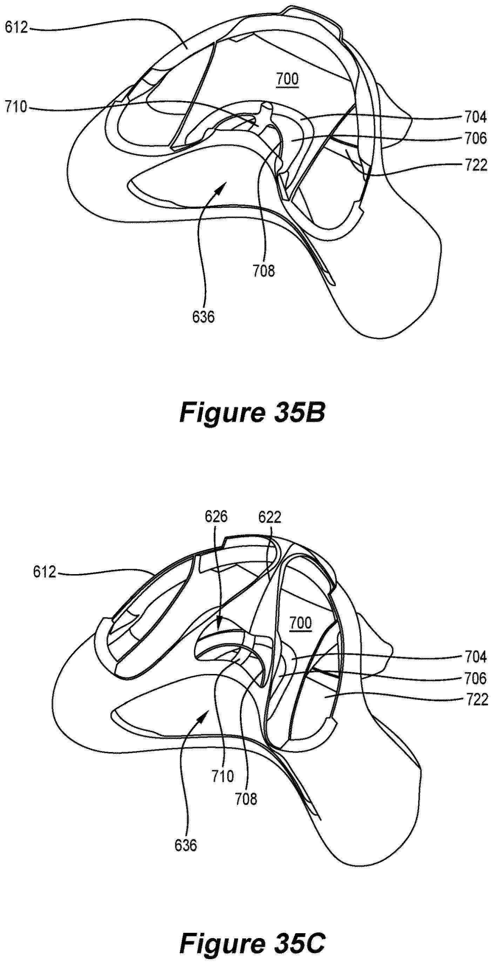

The cavity wall 634 is recessed from the combined nasal aperture 626, 630 and is linked to a rim 638 of the nasal aperture 626, 630 and is linked to the seal member 612 adjacent to the rim 638 by a tether 710 which is recessed from the rim 638 of the nasal aperture 626, 630 to avoid contact with the patient. The deflector panel 706 of the cavity wall 634 is curved proximally away from the opening 654 (as shown in Figures 35A, 35B and 35C) and terminates at a top edge in a rim 708 which is recessed from the rim 638 of the nasal aperture 626, 630 (Figures 32 and 37) to form a plenum chamber below the nares when the mask 610 is fitted to a patient, as described above with reference to the mask 410. The deflector panel 706 is connected directly to the perimeter of the nasal aperture 626, 630 at lateral sides and is connected to proximal and distal points of the perimeter of nasal aperture 626, 630 by the tether 710. In another aspect the deflector panel 706 may be connected at lateral sides to the seal member 612 at locations spaced away from the nasal aperture 626, 630. The tether 710 is curved downwardly from the perimeter of the nasal aperture 626, 630 to join the rim 708 of the deflector panel 706. Accordingly, the tether 710 will not come into contact with a patient when the mask 610 is fitted.

The shear panel 704 of the cavity wall 634 provides at least one of the preferential deformation regions. The shear panel 704 follows the perimeter of the deflector panel 706 and, therefore, has a generally U-shaped form (Figure 35) on account of extending about the deflector panel 706 from one side of the nasal cradle 622 to the other side. The shear panel has a curved profile (Figures 32, 34 and 37) from an edge of the deflector panel 706 to a proximal edge of the transition panel 702. The distal edge of the transition panel 702 joins the main panel 700.

It will be appreciated that the deformation region decouples one portion of the cavity wall from another portion of the cavity wall such that a force applied to one portion is not transferred to the other portion. Furthermore, the two portions of the cavity wall (i.e. the upstream main panel 700 and the downstream deflector panel 706) are each shaped to resist deformation. The decoupling can be seen in Figures 32 to 35 and 37 where the deflector panel 706 is inclined relative to the main panel and, therefore, reciprocal movement of the deflector panel 706 would be resisted by the main panel 700 if they were connected directly to each other. The shear panel 704, however, forms a flexible connection between the deflector panel 706 and the main panel 700 so that deflections of the deflector panel 706 are, up to a limit, absorbed by the shear panel 704 (i.e. not transmitted to the main panel 700). The shear panel 704 has a reduced wall thickness compared to the main panel 700 and the deflector panel 706. This means that the shear panel 704 is more flexible than the main panel 700 and the deflector panel 706 and, therefore, the shear panel 704 preferentially deforms before the deflector panel 706 or the main panel 704 are deformed. The shape of the shear panel 704 is selected to allow the shear panel 704 to roll over either the main panel 700, the deflector panel 706 or both when they translate with respect to each other. The resulting effect is that, when the seal member 612 is subject to deformation forces the shape of the flushing flow cavity 628 is substantially maintained due to the main panel 700 and the deflector panel 706 transferring the deformation forces into the deformation regions (e.g. the shear panel 704) such that deformation of the cavity wall 634 is substantially confined to the deformation regions.

With deformation focussed in the shear panel 704, the deflector panel 706 and the main panel 700 remain generally in their original shape and/or positions relative to each other and therefore retain the flushing flow cavity 628 open for the free flow of respiratory gas. Occlusion of the flushing flow cavity 628, either partially or fully, therefore is reduced because the likelihood of the deflector panel 706 and the main panel 700 buckling or folding so as to obstruct the flushing flow cavity 628 is reduced. Furthermore, with preferential deformation focussed into the shear panel 704, the flow resistance through the flushing flow cavity is unlikely to increase significantly. This means that the required flow ratio of respiratory gas through the primary flow cavity 636 and the flushing flow cavity 628 will generally maintained, thereby providing the patient with an effective treatment. The deflections that will generally be accommodated by preferential deformation of the shear panel 704 and by slight deformation of the deflector panel 706 are deformations associated with different facial geometries and with adjustment of the mask 610 on the face of a patient.

As mentioned previously, the ratio of the cross-sectional areas of the flushing flow outlet 630 and the nasal outlet 626 provides control of the flow of respiratory gas to deliver an effective treatment, including anatomical dead-space flushing. In this embodiment, the cavity wall 634 is connected to a rim 638 of the nasal aperture 626, 630 to define cross-sectional areas of the flushing flow outlet 630 and nasal outlet 626 and to resist changing the cross-sectional areas of the flushing flow outlet 630 and the nasal outlet 626 when the seal member 620 is deformed. Maintaining the ratio depends on maintaining the deflection panel 706 in a recessed position relative to the nasal aperture 626, 630. This is facilitated in this embodiment by a reinforcing bead 712 (Figure 34) that extends around the rim 638 of the nasal aperture 626, 630. The bead 712 has a wall thickness that is greater than the wall thickness of the surrounding seal member 620 such that the bead 712 is less flexible (i.e. more deformation resistant) than the surrounding seal member and, therefore, resists occlusion or deformation of the nasal aperture. The same applies to retain the shape of the rim 638 and the spacing of the deflector panel 706 from the nasal aperture 626, 630 when the seal member 612 is subject to elevated gas pressure during treatment. Accordingly, deflection of the nasal cradle 622 will be focussed by the bead 712 and by the tether 710 through to the deflector panel 706. However, the curved shape of the deflector panel 706 makes it relatively rigid compared to the shear panel 704 so that deflection of the deflector panel 706 is transmitted to the shear panel 704 which deflects. This means that the deflector panel 706 remains at its location relative to the nares such that a plenum chamber formed between the end of the deflector panel 706 and the nares remains even when the seal member 620 is deformed, up to an extent. Accordingly, preferential deformation of the shear panel 704, up to an extent, avoids buckling or deformation of the deflector panel 706 and the main panel 700. Beyond the point at which deformation of the deflector panel 706 and the main panel 700 occurs, it will be appreciated that their configuration means that they deform in a way that limits the extent to which the flushing flow cavity 728 is occluded. Accordingly, the mask 610 accommodates a greater range of facial geometries and, therefore, reduces the likelihood of the flushing flow cavity 628 and the nasal aperture 626, 630 being occluded.

During use, when the mask 610 is fitted, force will be applied to the face-contacting surface of the seal member 612 due to differing facial geometries, headgear preferences and pressure settings. These forces and the locations they are applied will differ. The configuration described above, however, focusses the forces and deflection into a preferential deformation region, (i.e. the shear panel 704 in this embodiment) to provide a predictable collapse and rebound movement. The predictable buckling pattern achieved via the preferable deformable region allows the mask 610 to be designed in such a way that, when forces are applied to the seal, the resulting deformation and compression that occurs in the elastomeric material that forms the seal member 612 happens in such a way that the apertures and cavities remain unobstructed. Without the preferential deformation region, collapse of the deflector wall 706 would be unpredictable, potentially leading to inconsistent flow through the apertures and cavities of the mask 610. This would cause inconsistencies in the therapy achieved, comfort, fitting procedure and in overall performance both between uses for the same patient and between different patients.

In a variation of this embodiment, the seal member 612 may have more than one preferential deformation region. For example, the additional preferential deformation regions may be incorporated into the cavity wall 634 or may be incorporated into the seal member 612 at other locations that enable the flushing flow cavity 628 and/or the nasal aperture 626, 630 to substantially retain its shape, enable the ratio of cross-sectional areas of the flushing flow outlet 630 and nasal outlet 630 to be substantially maintained or enable both.

As shown in Figures 36 to 41, the mask 610 includes a mask frame 670 in the same general form as described in the embodiments described above. Specifically, the mask frame 670 includes a body 672 having side wings at 674, each of which includes upper and lower connector bars 676 for fastening the mask frame 670 to head gear which holds the mask 610 on a patient's face.

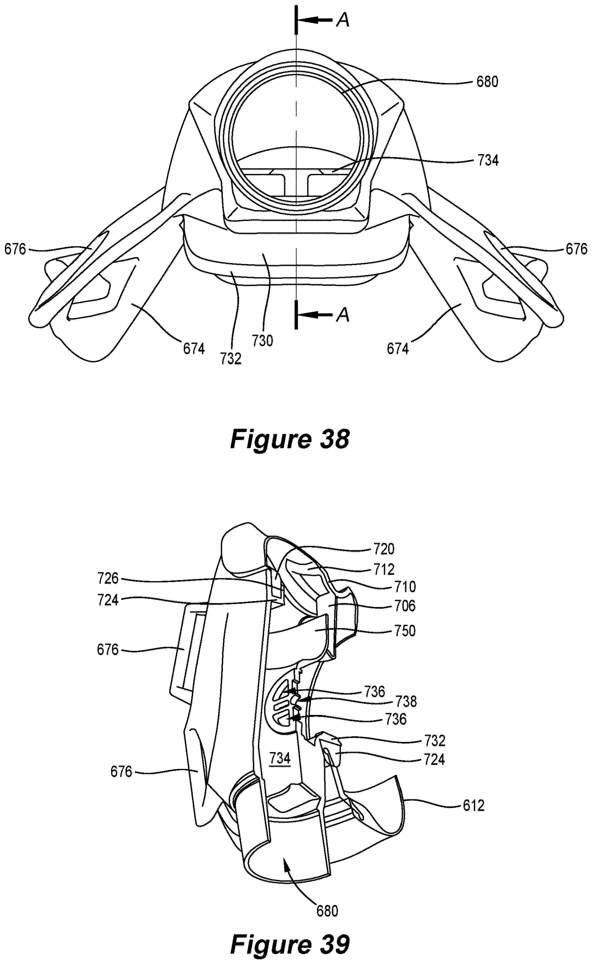

Figures 38 to 40 show the structure and flow paths of the mask frame 670. In particular, the mask frame 670 has a single primary flow path inlet 680 which leads to a primary flow channel 682. Downstream of the primary flow path inlet 680 is a partition 734 which splits the flow of respiratory gas between the primary flow channel 682 and a flushing flow channel 684. The respective cross-sectional areas of the primary flow channel 682 and the flushing flow channel 684 are selected to provide the required volumetric flow rates of respiratory gas for providing a flushing flow to flush anatomical dead space and for providing respiratory gas for respiration. Figures 37 and 38 show that the area of the entrance to the flushing flow channel 684 is greater than the combined areas of the entrances to the primary flow channel 682 and, because of this, flow through the flushing flow channel 684 has a lower resistance to flow (at least at the point of entry) than the resistance to flow through the primary flow channel 682. However, this may be reversed or adjusted in other embodiments to modify the bias of flow through the mask frame 670 depending on the treatment.

An upper portion of the flushing flow channel 684 comprises a dividing wall 750 which leads to an outlet 752. When the mask frame 670 is fitted to the cushion module 620 (comprising the seal member 612 and the housing 650) as shown in Figure 39, the dividing wall 750 is pressed firmly into a top side of the main panel 700 to form a generally air-tight seal. Accordingly, respiratory gas flowing through the flushing flow channel 684 goes past the dividing wall 750 and into the flushing flow cavity 628 as shown in Figure 37. The primary flow channel 682 opens at an outlet 754 from between an underside of the dividing wall 750 and a U-shaped lip 730 that extends proximally from the mask frame 670. Respiratory gas exiting the outlet 754 passes into the primary flow cavity 636 from which the respiratory gas is delivered to the patient via the oral aperture 624, the nasal outlet 626 or via both.

For connecting the cushion module 620 to the mask frame 670, the mask frame 670 includes a seat 728 formed as a groove by a proximally extending ledge 724 and a retaining wall 726 which extends generally perpendicularly to the ledge 724 and generally parallel to the body 672. Furthermore, the mask frame 670 includes a bead 732 on an outermost edge of the lip 730 (see Figure 38). As shown in Figure 37, and upper rim 720 of the opening 654 is seated in the seat 728 and a lower rim 724 of the opening 654 is passed over the bead 732 and is seated snuggly on the outer side of the lip 730 to form a generally air-tight seal between the opening 654 and the mask frame 670. Additionally, the mask frame 770 is co-operable with the cavity wall 634 to separate the primary flow cavity 636 from the flushing flow cavity 628.

Differing facial geometries and headgear conditions may lead to at least part of one of the flow paths to either the oral aperture 624, the nasal outlet 626 or the flushing flow outlet 630 being occluded, in use. If this occurs, the necessary flow rate needed to achieve the required pressure delivered to the patient may not be able to be delivered through the restrictive point of the unobstructed flow path (usually the point where the flow is split).

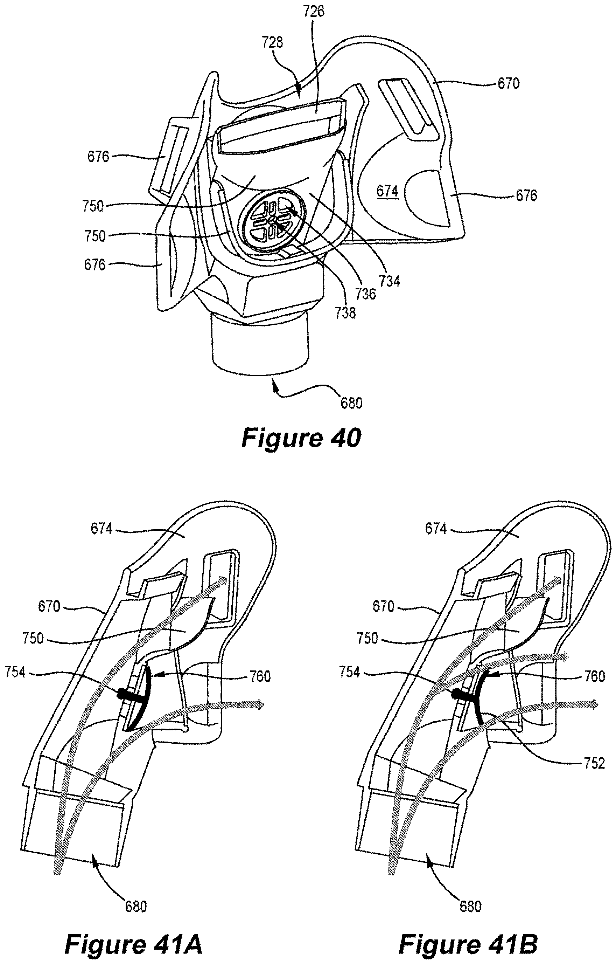

To address this, the partition 734 includes a pressure relief valve, in the form of a flap valve, mushroom valve or a flexible poppet valve 760, that enables respiratory gas from the flushing flow channel 684 to pass into the primary flow channel 682 via valve apertures 736 (Figures 39 and 40). A poppet valve 760 comprises a stem 754 which is seated in the valve seat 738 and comprises a cap 752 from a central point of which the stem 754 projects. The cap 752 has a generally domed shape with an outer rim which, when the poppet valve 760 is seated in the valve seat 738, extends over the valve apertures 736 and contacts and the partition 734 to form a seal that separates respiratory gas in the flushing flow cavity 684 from the respiratory gas in the primary flow channel 682 (as shown in Figure 41A).

At least the cap 752 is formed of a resilient, elastomeric material and the elasticity of the material is selected to enable respiratory gas in the flushing flow channel 684 to flow into the primary flow channel 682 when the respiratory gas in the flushing flow channel 684 exceeds a threshold gas pressure. When the threshold pressure is exceeded, the gas pressure causes the cap 752 to flex away from the partition (as shown in Figure 41B), thereby breaking the seal and allowing respiratory gas to flow from the flushing flow channel 684 to the primary flow channel 682. When the gas pressure in the flushing flow channel 684 reduces to the threshold pressure or below, the poppet valve 760 closes. In the event that the flow path through the primary flow cavity is occluded, the respiratory gas would continue to flow through the flushing flow channel 684, thereby delivering respiratory gas to the patient despite the obstructed flow path. This combination of flow allows for adequate therapy pressure to be delivered through only one of the flow path apertures (i.e. one of the oral aperture 624, the nasal outlet 626 or the flushing flow outlet 630). In some aspects, the threshold gas pressure may be defined by a pressure differential between the flushing flow channel 684 and the primary flow channel 682.

With the sizes of the flushing flow channel 684 and the primary flow channel 682 being different in alternative embodiments to provide alternative therapies, it will be appreciated that the valve 740 may be configured to allow respiratory gas flow from the primary flow channel 682 to the flushing flow channel 684 or may be configured to be bidirectional to allow gas to flow in both directions between the primary flow channel 682 to the flushing flow channel 684.

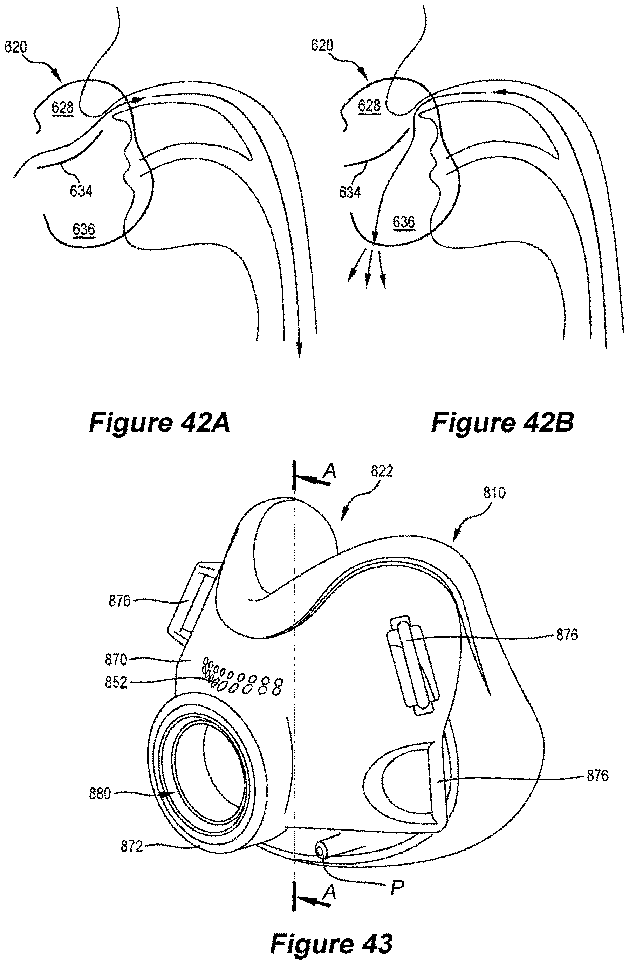

Without wishing to be held to any particular theory, the applicant believes that the interface 610 operates during the inhalation and exhalation phases of the breathing cycle in the manner shown in figures 42A and 42B. Specifically, during the inhalation phase (Figure 42A), respiratory gas is substantially provided by a flushing flow supplied via the flushing flow cavity 628, but some flow may be entrained from the primary flow cavity 636 via the nasal outlet 626 if the peak inspiratory demand exceeds the flow rate available via the flushing flow cavity 628. During the exhalation phase (Figure 42B), it is anticipated that any flow exiting the nasal cavity will enter the primary flow cavity 636 of the cushion module 620 (due to the relatively lower gas pressure in the primary flow cavity 636 compared to the gas pressure in the flushing flow cavity 628) so that it can be vented to the atmosphere through bias flow vents 652. For this to occur, there needs to be a gap formed between the nares and the cavity wall 634 which separates the primary flow cavity 636 and the flushing flow cavity. The recessed location of the rim 708 relative to the nasal outlet 626, together with the deformation region formed in the cavity wall 634, is believed to enable this flow arrangement to occur.

The problem of occluded or restricted gas flow through the seal member to the nares or to the oral aperture can occur in all of the embodiments described. It follows that the pressure relief valve can be adopted in any of the patient interfaces described above.

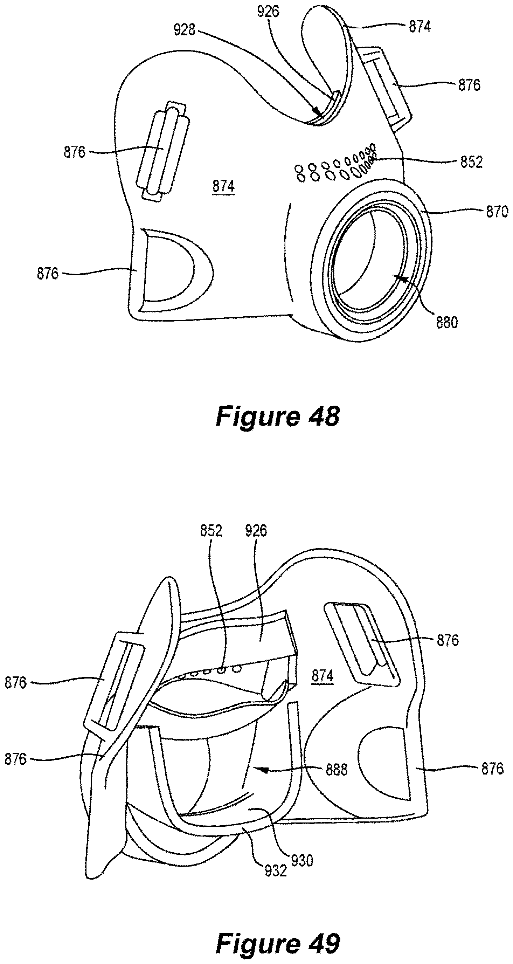

An embodiment of a mask 810 according to the ninth aspect disclosed above is shown in Figures 43 to 49.

The mask 810 includes a seal member 612 and a housing 650 in the same form as the cushion module 620 described above in respect of the mask 610, except that in this embodiment the housing 650 does not include bias vent holes for venting exhaled respiratory gas to outside of the mask 810. The same references numerals used in Figures 30 to 42 to describe the mask 610 are used in Figures 43 to 49 to denote the same features in the mask 810. It follows that the mask 810 includes, as shown in the figures, the same preferentially deformable cavity wall 634 that is disclosed with respect to the mask 610. Therefore, the following description should be read on the basis that the same cavity wall 634 is present in the mask 810.

The mask 810 further includes a mask frame 870 which differs from the mask frame 670 of the mask 610. Specifically, while the mask frame 870 includes a body 872 having side wings 874 and connector bars 876 for attaching the mask frame 870 to head gear, the mask frame 870 has a single primary flow path inlet 880 that delivers respiratory gas via the opening 654 into the primary flow cavity 636 and has a single outlet 888. The respiratory gas may then be inhaled by a patient via the oral aperture 624, via the nasal outlet 626 or via both. The mask frame 870 includes a primary flow channel 882 with an inlet 880 that is arranged relative to its outlet 888 such that the respiratory gas undergoes small (0 to 5°) changes in direction along the length of the channel 882. In this embodiment, the inlet 880 is opposite the outlet 888. This arrangement provides the primary flow channel 882 with a low resistance to gas flow. It is believed that, with a single inlet 880 and a single outlet 888, the flow of respiratory gases through the mask 810 will be less restricted because it avoids multiple streams of gases travelling in opposite directions which can impede each other.

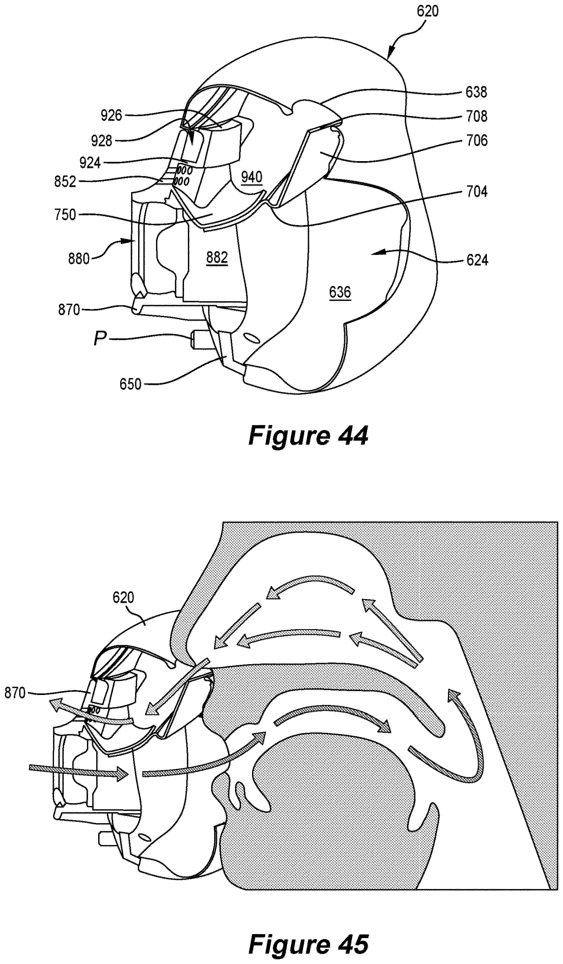

For connecting the cushion module 620 to the mask frame 870, the mask frame 870 includes a seat 928 formed as a groove by a proximally extending ledge 924 and a retaining wall 926 which extends generally perpendicularly to the ledge 924 and generally parallel to the body 672. Furthermore, the mask frame 870 includes a bead 932 on an outermost edge of the U-shaped lip 930. As shown in Figure 44, an upper rim 720 of the opening 654 is seated in the seat 928 and a lower rim 724 of the opening 654 is passed over the bead 932 and is seated snuggly on the outer side of the lip 930 to form a generally air-tight seal between the opening 654 and the mask frame 870.

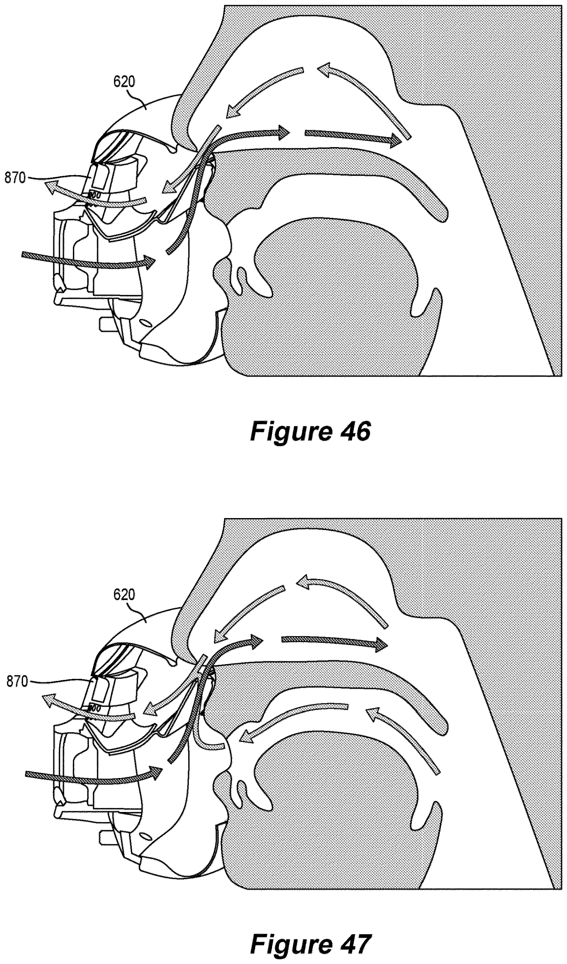

The mask 870 further differs from the mask frame 670 in that there is no inlet for fresh respiratory gas from a gas source into the flushing flow cavity. Instead, the mask frame 870 includes bias vent holes 652 in the body 672 below the ledge 924. The cavity, therefore, is an exhaust cavity 940 as shown in Figures 45 to 47 where exhaled respiratory gas (either from the mouth or from the nares or from both) flows through the exhaust cavity 940 and where exhaled respiratory gas flushed from dead space in the patient's nasal cavity flows through the exhaust cavity 940 (Figure 46) and out through the bias vent holes 652. The mask frame 870 is co-operable with the cavity wall 634 to separate the primary flow cavity 636 ( i.e. the first cavity) from the exhaust cavity 628 (i.e. the second cavity). More specifically, the dividing wall 950 is pressed firmly into a top side of the main panel 700 of the cavity wall 634 to form a generally air-tight seal so the exhaled respiratory gas from the nares does not flow into the primary flow cavity 636 and, instead, exits the mask 810 via the bias vent holes 652. Additionally, exhaled gas from the mouth is carried with respiratory gas into the exhaust cavity 940, as shown in Figure 47, and from which the exhaled gas exists the mask 810.

While the seal member 612 and the housing 650 are the same as in the previous embodiment, their effect with the mask frame 870 is different. Specifically, the dividing wall 950, the location of the vent holes in the exhaust cavity 940 with a single flow path entering the primary flow cavity 636 cavity which results in more efficient venting and the inducement of a flushing flow either within the user's anatomical dead space (if a mouth breather) or into the nares (if a nose breather) without actually having a dedicated flushing flow channel. For respiratory gas and exhaled gas to leave the mask 810 it either has to:
a) flow through the mouth and out through the nose thereby flushing expired gas out of the user's oro-nasal cavities during exhalation, or
b) flow past the dividing wall, which due to the dividing wall's proximity to the patient will create a flushing flow into the nares due to constriction of the flow path in that region of the cushion module 620.

Thus, in either of the above situations, the anatomical dead space of the patient will be at least partially flushed.

The arrangement described above for connecting the cushion module 620 to the mask frame 870 is one example of a connection. Other arrangements may be adopted provided the connection which can withstand the elevated gas pressure. For example, the connection described above allows the cushion module 620 to be separated from the mask frame 870 for cleaning. However, the connection may be a permanent connection. In a further alternative, the mask frame 870 and the housing 650 may be formed integrally so that the cushion module can be subsequently over-moulded to form a unitary patient interface.

In previous embodiments, anatomical dead space flushing is provided by accelerating respiratory gas through a flushing flow cavity to enter of the nares of the patient. With the mask 810, the anatomical dead space flushing is thought to occur in different ways as shown in the scenarios depicted in Figures 45 to 47. The first scenario shown in Figure 45 represents when a patient exhales through their nose with their mouth open. According to this scenario, at the end of exhalation, fresh pressurised respiratory gas enters the patients mouth via the oral aperture 624 from the primary flow cavity 636. It then flows into the throat and up through the nasal cavity, before exiting the nares into the exhaust cavity 940 and exiting to the atmosphere through the bias vent holes 852. The flow of fresh respiratory gas in through the mouth and out through the nares has the effect of flushing the anatomical dead space by removing the carbon dioxide-rich exhaled respiratory gas that remains in the throat and nasal passages at the end of the exhalation phase.

Figure 46 shows another scenario where anatomical dead space flushing is thought to occur when the patient's mouth is closed. (Figure 46 is a schematic cross-sectional view which, although showing the patient's mouth as being open, should be read as the patient's mouth being closed.) During exhalation through the nose and with the mouth closed, fresh pressurised respiratory gas enters the primary flow cavity 636 and travels between the cavity wall 634 and the portion of the seal member 622 which contacts the patient's upper lip. In the absence of such a portion, the patient's upper lip forms a side of flow path such that during exhalation through the nose and with the mouth closed, fresh pressurised respiratory gas enters the primary flow cavity 636 and travels between the cavity wall 634 and the patient's upper lip. This flow path has a small cross-sectional area which increases the velocity of the respiratory gas, thereby creating a jet of respiratory gas which enters the patient's nasal cavity mixing with the exhaled respiratory gas and flushing the carbon dioxide-rich anatomical dead space due to a combination of increased velocity and turbulence of the gas entering the patients nares. The exhaled respiratory gas enters the exhaust cavity 940 and exits to the atmosphere through the bias vent holes 652.

Figure 47 shows a third scenario where anatomical dead space flushing is thought to occur when the patient exhales through their mouth. In this scenario, it is expected that carbon dioxide-rich exhaled respiratory gas fills the nasal cavity. However, with the mask 810, during exhalation through the mouth fresh pressurised respiratory gas enters from the primary flow cavity 636 and travels between the cavity wall 634 and the patient's upper lip. As mentioned above, this flow path has a small cross-sectional area which increases the velocity of the respiratory gas, thereby creating a jet of respiratory gas. One portion of the jet enters the patient's nasal cavity, mixing with the exhaled respiratory gas and flushes the carbon dioxide-rich anatomical dead space. However, it is believed that the other portion of the jet flows across the rim 708 of the cavity wall 634 and into the exhaust cavity 940, thereby creating a venturi effect which draws gas from the nasal cavity, including at least some of the exhaled respiratory gas. The exhaled respiratory gas from the nasal cavity and from the primary flow cavity enters the exhaust cavity 940 and exits to the atmosphere through the bias vent holes 652.

In each of these three scenarios, the respiratory gas is supplied through a single inlet to the primary flow cavity 636. The single inlet, i.e. the primary flow path inlet 680, is formed as a large circular opening to reduce flow resistance so that the pressure therapy that the patient receives is not compromised. The constant availability of fresh, pressurised respiratory gas supplied to the current embodiment is thought to cause anatomical dead space flushing regardless of whether the mouth is open or closed and regardless of whether the patient is exhaling through their mouth, nose or both.

As with other embodiments described above, the mask frame 870 may be permanently connected to the cushion module 620. This may be achieved by suitable connections, such as snap-fit formations or welding, that are known to a person skilled in patient interfaces. Alternatively, the mask frame 870 may be releasably connected to the cushion module 620 to enable separation and cleaning of the mask frame 870 and the cushion module 620 for cleaning and for replacement parts. This also may be achieved by suitable connections that are known to a person skilled in patient interfaces such as a snap-fit, push fit or an interference fit.

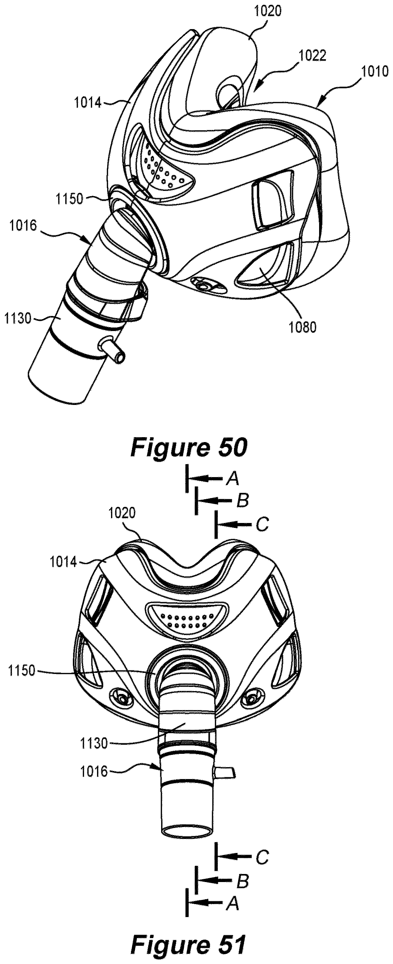

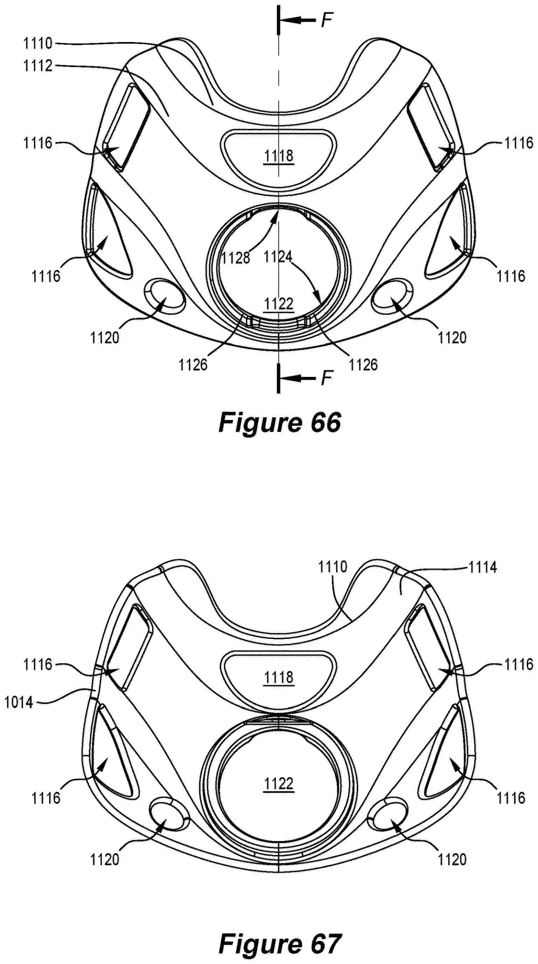

A further embodiment of a patient interface 1010 is shown in Figures 50 to 70. It is a variation of the mask 810 shown in Figure 43 to 49, although the respiratory gas flow and dead-space flushing functionality remains the same as described above with reference to Figures 45 to 47. The patient interface 1010 includes cushion module 1012, a frame 1014 and a conduit connector 1016 comprising an elbow 1130 and a socket insert 1150.

The cushion module 1012 (Figures 50 to 52, 54, 61 and 62) is a variation of the cushion module 620 shown in Figures 30 to 35C. The cushion module 1012 includes a seal member 1020 which is fixed to a housing 1080 to define an interior volume. The interior volume is divided into first and second cavities by a cavity wall 1050. The first cavity is a primary flow cavity 1032 and the second cavity is an exhaust flow cavity 1030 (also referred to herein as an exhaust cavity). The housing 1080 and the seal member 1020 are fixed by over-moulding. The seal member 1020 is formed of soft, resilient material, such as silicone. It includes an oral aperture 1028 that, when fitted to a patient, circumscribes the patient's mouth and includes a nasal aperture 1024. The seal member 1020 is formed with a nasal cradle 1022 in the valley of which is located the nasal aperture 1024.

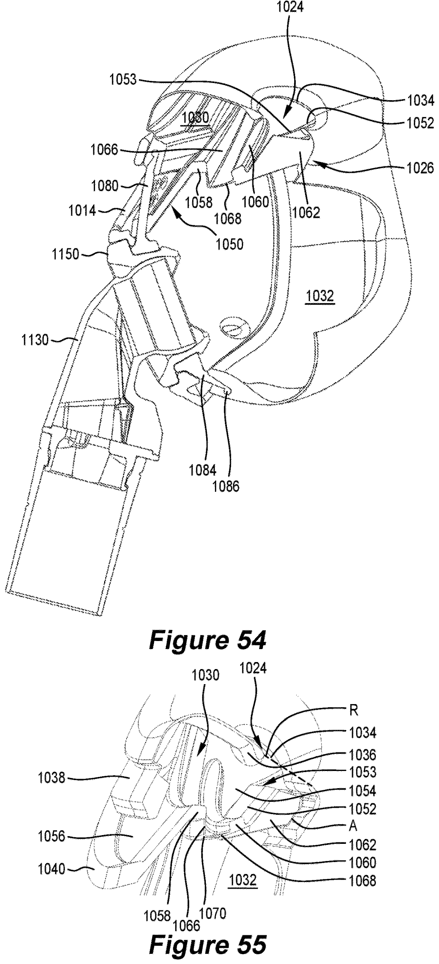

As shown in Figures 54 to 60, the oral aperture 1028 communicates with the primary flow cavity 1032 to enable transfer of respiratory gas between a primary flow cavity 1032 and the patient's mouth for breathing during the respiratory cycle and for dead-space flushing.

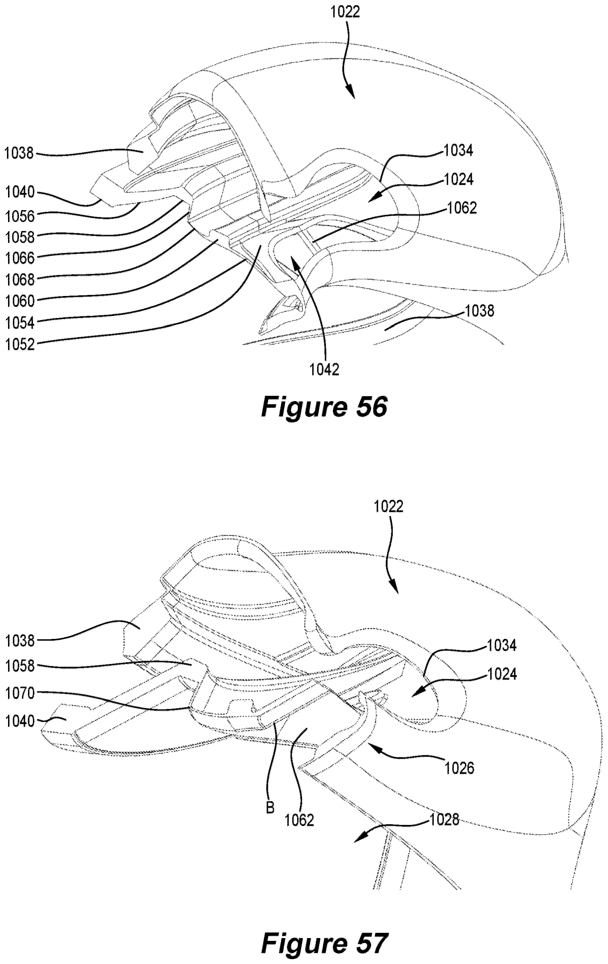

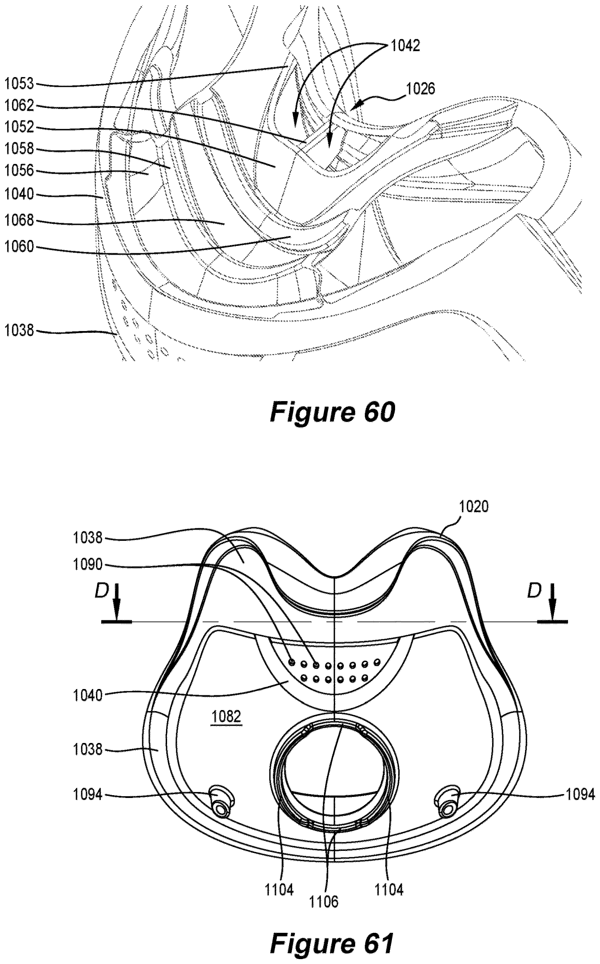

The nasal aperture 1024 is positioned in the seal member 1020 to align with the nares of the patient when the patient interface 1010 is fitted to the patient. This enables transfer of respiratory gas from the primary flow cavity 1032 via the nasal outlet 1042 (see Figures 56 and 60) and through the nasal aperture 1024 to the nares for breathing during the respiratory cycle and for anatomical dead-space flushing. The nasal outlet 1042 is recessed from the nasal aperture 1024. The positioning of the nasal outlet 1042 relative to the nasal aperture 1024 enables excess respiratory gas from the primary flow cavity 1032 to pass into the exhaust flow cavity 1030 and be vented to ambient through vent apertures 1090 in the housing 1080. The nasal aperture 1024 is defined, at the outer surface of the seal member 1020, by a rim 1034. However, given that the nasal outlet 1042 and an opening into the exhaust flow cavity 1030 are recessed from the rim 1034, the nasal aperture 1024 comprises a volume between the level of the rim 1034 and the combined opening formed by the nasal outlet 1042 and the opening to the exhaust flow cavity 1030. It is through this volume, i.e. the nasal aperture 1024, that respiratory gas can flow from the primary flow cavity 1032 to the exhaust flow cavity 1030. In other words, flow between the primary flow and exhaust cavities occurs past an edge of the cavity wall. This edge is located distal from the gas inlet opening and proximal to the nasal aperture. The deformation region is configured to maintain a spaced relationship between this edge of cavity wall and the nasal aperture.

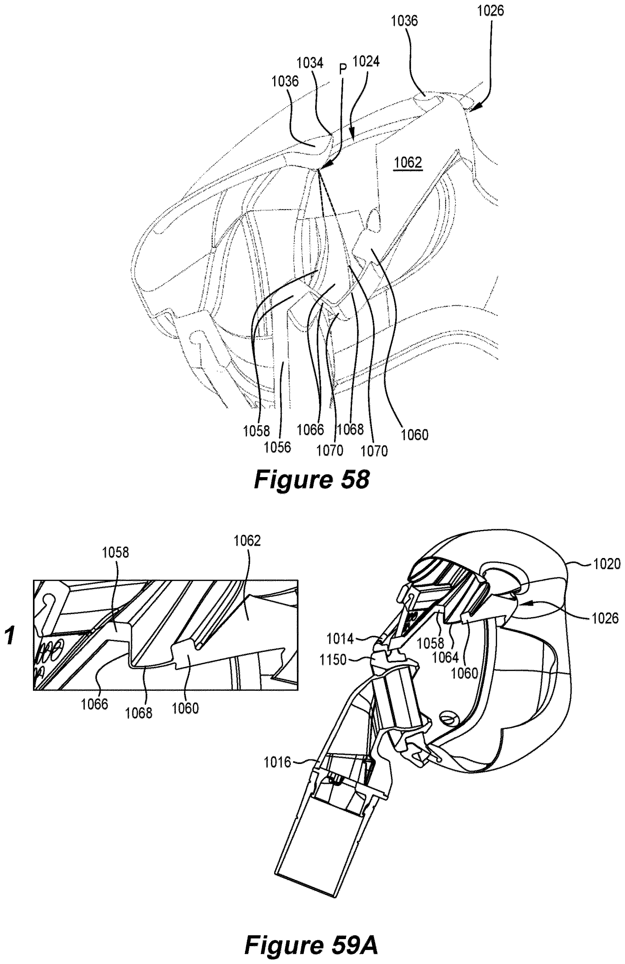

A bead 1036 circumscribes the rim 1034 on an inner surface of the seal member 1020. The bead 1036 comprises, in this embodiment, a region of increased wall thickness as shown in Figure 58. The increased thickness of the rim 1034 increases the resistance of the rim 1034 to a blow-out when the patient interface 1010 receives pressurised respiratory gas from a flow generator. The increased thickness of the rim 1034 also increases the resistance of the rim 1034 to undesired deformation which may occur when the patient interface 1010 is fitted to the patient.

The seal member 1020 also includes the cavity wall 1050 (as shown in Figures 54 to 58 and 60) which partitions the cushion module 1012 internally to define the primary flow cavity 1032 and the exhaust flow cavity 1030. However, the cavity wall 1050 is arranged to permit respiratory gas to flow from the primary flow cavity 1032 to the exhaust flow cavity 1030. The exhaust flow cavity 1030 is located in an upper portion of the internal volume of the seal member 1020 (see Figure 54). The primary flow cavity 1032 comprises the lower portion of the internal volume of the cushion module 1012.

The cavity wall 1050 is configured to enable preferential deformation of the cavity wall 1050 in a way that reduces the likelihood of the exhaust flow cavity 1030 and the nasal aperture 1024 being occluded. In this embodiment, the cavity wall 1050 is linked to a wall portion 1026 of the seal member 1020 which is between the nasal aperture 1024 and the oral aperture 1028 by a linking member 1062 (see Figures 54 to 58). The linking member 1062 directs forces applied to the wall portion 1026 into the cavity wall 1050 where the deflection force is absorbed. In doing so, the linking member 1062 substantially retains the position of the cavity wall 1050 relative to the nasal aperture 1024 and the wall portion 1026. Another way of understanding the effect of the linking member 1062 is to understand that it braces the cavity wall in position relative to the wall portion 1026. This enables a patient treatment to continue with little interference to the flow of respiratory gas (a) through the nasal outlet 1042, (b) through the nasal aperture 1024 and (c) through the exhaust flow cavity 1030.

The linking member 1062 functions similarly to the tether 710 described above and shown in Figure 37. That is, the linking member 1062 braces the cavity wall 1050 so that the spacing of the wall portion 1026 from the cavity wall will be substantially maintained when a deformation force is applied to the wall portion 1026. This will reduce the likelihood of the nasal outlet 1042 being occluded. This substantially fixed spacing also works in the other direction, in that ballooning or blow-out of the wall portion 1026 away from the cavity wall 1050 is inhibited by the connection of the wall portion 1026 to the cavity wall 1050. Additionally, the linking member 1062 will focus a deformation force into the cavity wall 1050 which is designed to preferentially deform at a position away from the nasal aperture 1024 so that the deformation is unlikely to occlude the nasal aperture 1024.

The deformation absorption effect is shown in Figures 59A to 59C which also show that the cavity wall 1050 comprises a deflector panel 1052, a lateral panel 1054, a main panel 1056 and a deformation region 1074 linking the lateral panel 1054 and the main panel 1056. The deformation region 1074 comprises first and second resilient regions 1058, 1060 and first and second walls 1066, 1068. The deformation panel 1064 comprises the first wall 1066 which projects from the first resilient region 1058 (see Figure 58), the second wall 1068 which extends from the second resilient region 1060 and a connecting portion 1070 which connects the first wall 1066 to the second wall 1068. The connecting portion 1070 has a bend profile which, at rest, aligns with the first direction of the first wall 1066 and aligns with an end of the second wall 1068 remote from the second resilient region 1060.

The deformation region 1074 structurally decouples the deflector panel 1052 from the main panel 1056. The decoupling occurs because the deformation panel 1064 accommodates a reduction in distance between the first and second resilient regions 1058, 1060.

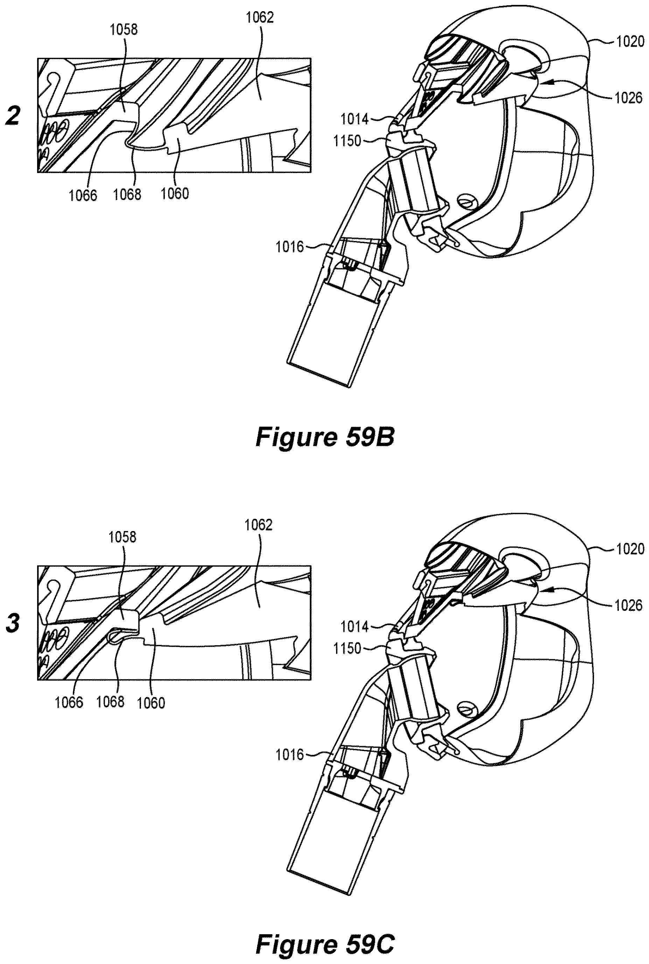

Deformation of the deformation panel 1064 occurs in two stages. The first stage, as the second resilient region is initially displaced toward the first resilient region causes the first wall to fold about its line of connection with the first resilient region until it contacts, or is located adjacent, an underside of the first resilient region. At this point, there is still a gap between the first and second resilient regions 1058, 1060 because the length of the second wall 1068 is longer than the length of the first wall 1066. In the second stage of deformation, as the second resilient region 1060 approaches the first resilient region 1058, the second wall 1068 buckles and translates over the first wall 1066 until the second resilient region 1060 contacts, or is located adjacent, the first resilient region 1058 (as shown in Figure 59C). This buckling and translating motion may be referred to as "rolling". The bucking occurs on account of the curvature of the second wall 1068 and the greater length of the second wall 1068 compared to the first wall 1066. In particular, as shown in Figure 59B, an inflection point which is furthest from the second resilient region is the connecting portion 1070. However, as the first wall 1066 reaches its limit of accommodating the displacement of the second resilient region 1060, the position of the connecting portion 1070 will become fixed so that further advancement of the second resilient region 1060 toward the first resilient region 1058 will cause the second wall 1068 to buckle by adopting an increased curvature. The increased curvature results in the second wall 1068 adopting a U-shape with an inflection point that shifts from the connecting portion 1070 to a position along the second wall 1070 away from the connecting portion 1070. When the second resilient region 1060 contacts the first resilient region 1058, the inflection point has shifted along the second wall 1068 such that a portion of the second wall 1068 is beyond the inflection point. In other words, as the second wall 1068 buckles and translates over the first wall 1066, the length of the second wall 1068 beyond the inflection point increases and the length of the second wall 1068 behind the inflection point decreases.

Depending on the geometry and wall thickness of the first and second walls 1066, 1068, the buckling and translation of the second wall 1068 may occur before the connecting portion 1070 becomes fixed. However, the action of the inflection point shifting (and, therefore, the length of the second wall 1068 beyond and behind the inflection point changing) remains the same.

Having regard to Figures 55 and 56, the deflector panel 1052 is a curved panel with an end rim 1053 recessed from the level of the rim 1034 of the nasal aperture 1024 (denoted by the dashed line R in Figure 55). The recessed position of the deflector panel 1052 ensures that the deflector panel 1052 does not touch the patient's septum (which may cause discomfort) and provides a plenum chamber between the end of the deflector panel 1052 and the rim of the nasal aperture for respiratory gas to flow through from the primary flow cavity 1032 to the nares or the exhaust flow cavity 1030. The preferential deformation of the deformation region 1074 ensures that the plenum chamber remains even when the seal member 1020 is deformed. The deflector panel 1052 is joined at its lower end to the lateral panel 1054 and is inclined upwardly therefrom to the end rim 1053. Disposed adjacently to the deflector panel 1052 is the wall portion 1026 of the seal member 1020 which is between the nasal aperture 1024 and the oral aperture 1028. The deflector panel 1052 is connected to the inner wall of the seal member 1020 either side of the nasal aperture 1024 beyond the bead 1036. This arrangement avoids further stiffening of the bead 1036 and the rim 1034 which might impact on patient comfort.

The seal member 1020 is configured to accelerate respiratory gas through the primary flow cavity 1032 and to direct the accelerated respiratory gas toward the nasal aperture 1024. In particular, the deflector panel 1052 and the wall portion 1026 define a channel leading toward the nasal aperture 1024. The channel terminates at the nasal outlet 1042, i.e. recessed from the nasal aperture 1024. The channel provides a taper in the primary flow cavity 1032 formed between the deflector panel 1052 and the wall portion 1026. In other words, the cross sectional area formed between the deflector panel 1052 and wall portion 1026 in the primary flow cavity 1032 reduces leading toward the nasal aperture 1024. The tapering causes the flow of respiratory gas through the channel to accelerate. Depending on the point in the breathing cycle, the accelerated respiratory gas enters the nares to provide anatomical dead space flushing.

As with other embodiments, an accelerated stream of respiratory gas is delivered to a patient to provide anatomical dead space flushing. The method with this embodiment involves delivering respiratory gas at an elevated pressure to the primary flow cavity 1032 of the cushion module 1012. The primary flow cavity 1032 defines a first cavity. As explained above, the primary flow cavity 1032 supplies the respiratory gas to the mouth and nares of a patient. This embodiment differs in that the flow of respiratory gas to the nares is accelerated through a portion of the primary flow cavity, i.e. the portion between the deflector panel 1052 and the wall portion 1026 in the primary flow cavity 1032 which reduces in cross-section area toward the nasal aperture 1024. The accelerated respiratory gas can then be delivered to the nares of a patient. The accelerated flow of respiratory gas occurs at the same time that respiratory gas is available for delivery to the mouth from the primary flow cavity 1032.

In this embodiment, the method further involves exhausting respiratory gas from the exhaust flow cavity 1030 in the cushion module 1012. The exhaust flow cavity 1030 defines a second cavity. The exhaust flow cavity 1030 is in fluid communication with the primary flow cavity 1032. In practical terms, the exhaled respiratory gas from the mouth and excess respiratory gas in the primary flow cavity flows into the exhaust flow cavity 1030 owing to them being in fluid communication. Additionally, exhaled respiratory gas from the nares flows into the exhaust flow cavity 1030. In this embodiment, the fluid communication between the primary flow cavity 1032 and the exhaust flow cavity 1030 is enabled by the recessed position of the cavity wall 1050 relative to the rim 1034 of the nasal aperture 1024 and a plenum chamber formed when the cushion module 1012 is fitted so the patient's nares are positioned over the nasal aperture 1024.

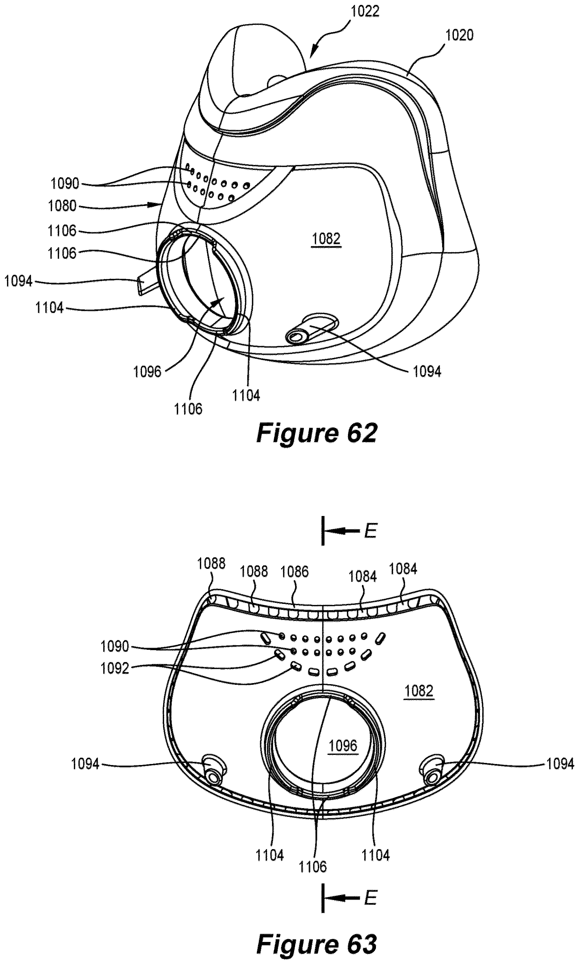

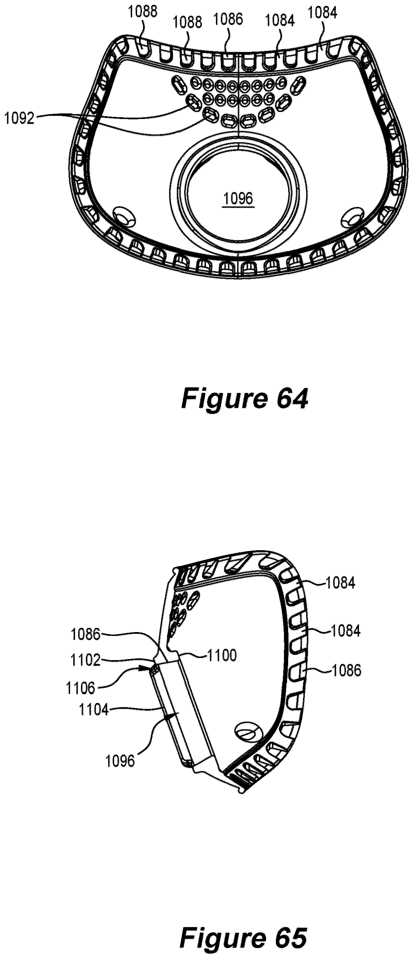

The lateral panel 1054 extends about the lower end of the deflector panel 1052 and extends laterally outwardly to join with the inner side walls of the seal member 1020. The lateral sides of the lateral panel 1054 also extend upwardly in a concave shape from the deflector panel 1052. The main panel 1056 extends upwardly from a line of connection with the housing 1080. In this embodiment, the housing 1080 includes a series of inner over-mould windows 1092 (see Figures 63 and 64) through which the main panel 1056 is over-moulded with the housing 1080 at the time of over-moulding the seal member 1020 with the housing 1080 to form the cushion module 1012. The material used to form the seal member 1020 flows through the over-mould windows 1092 during moulding so that the material takes on the shape of the housing 1080 and the windows 1092 prior to solidifying or curing. Having the material extend through the windows 1092 results in a mechanical connection with the housing. The windows 1092 may take the form of apertures which extend completely through the housing 1080.

The deformation region 1074 links the lateral panel 1054 to the main panel 1056. It comprises first and second resilient regions 1058, 1060 (Figure 60) and a deformation panel 1064 comprising the first and second walls 1066, 1068. The first resilient region 1058 connects the deformation region 1074 to the terminal wall. It has a generally polygonal profile and has a contour that is similar to the concave contour of the main wall. In this embodiment, the first resilient region 1058 smoothly merges with the main wall as it extends laterally toward the sides of the seal member 1020.

The second resilient region 1060 abuts the lower end of the deflection panel 1064 and is elongate. It is disposed generally parallel to the first resilient region 1058. The second resilient region 1060 is at least as wide as the nasal aperture 1024. It has this form to act as a load-spreader for forces that are transmitted through the linking member 1062. It may, however, be wider than the nasal aperture 1024 in other embodiments. While the second resilient region 1060, in this embodiment, is formed as a rib and has discrete ends, in other embodiments it may taper smoothly into the lateral panel 1054. The linking member 1062 extends from a line of connection (A in Figure 55) at the wall portion 1026 to a line of connection (B in Figure 57) and underneath the second resilient region 1060. The linking member 1062 increases the resilience (i.e. stiffens) of this region of the cavity wall 1050. It follows that forces applied to the wall portion 1026 in the direction of the housing 1080 are transmitted through to the second resilient region 1060 which is urged toward the first resilient region 1058. However, the first and second resilient regions 1058, 1060 are configured to encourage deformation of the deformation panel 1064 instead of deformation in the main panel 1056, the lateral panel 1054 or deflector panel 1052.

Figures 59A to 59C show the sequence of initial and subsequent deformation of the seal member 1020 when a deformation force is applied through the wall portion 1026. The patient interface 1010 is shown at rest in Figure 59A with the first and second resilient regions 1058, 1060 spaced apart. When a force is applied through the linking member 1062, the relatively thin wall thickness of the first and second walls 1066, 1068 compared to the relatively thick wall thickness of the first and second resilient regions 1058, 1060 causes the deformation to be absorbed by deformation of the first and second walls 1066, 1068. This initially occurs by folding of the first wall 1066 underneath the first resilient region 1058 as the spacing between the first and second resilient regions 1058, 1060 reduces (as shown in Figure 59B). As that spacing reduces further with additional deformation applied through the wall portion 1026 and the linking member 1062, the first wall 1066 folds flat against the underside of the first resilient region 1058 and the second wall 1068 buckles and rolls over the first wall 1066 (as shown in Figure 59C). This rolling action continues until the first resilient region 1058 abuts the second resilient region 1060.

The first wall 1066 extends below the level of the first resilient region 1058. In profile, the angle between an underside of the first resilient region 1058 and the first wall 1066 is in the range of 5° to 135° at rest. However, in the embodiment shown in Figures 50 to 70, the angle at rest is 85°. It has a contour that follows the contour of the first resilient region 1058 and it tapers inwardly towards its ends. As a result of the taper, a notional line following the intersection of the first resilient region 1058 with the first wall intersects a notional line following the line of the connecting portion 1070 at a pivot point (P in Figure 58). The first wall 1066 has a length in the rage of 1 mm to 10 mm and optionally in the range of 2 mm to 5 mm. The first wall 1066 has a thickness that is in the range of 0.15 mm to 1 mm. The thickness is constant along the width and length of the first wall 1066. To ensure that the first wall 1066 deforms in preference to the first resilient region 1058, the first resilient region 1058 has a wall thickness that is at least three times the wall thickness of the first wall 1066.

The connecting portion 1070 is disposed at the end of the first wall 1066 away from the first resilient region 1058 and the second wall 1068 extends from the second resilient region 1060 to meet the connecting portion 1070. In the current embodiment both the connecting portion 1070 and the second wall 1068 follow the concave contour of the first wall 1066. The second wall 1068 extends from the second resilient region 1060 initially in a direction inclined downwardly from a plane intersecting the second resilient region 1060 and the curved corner 1070. However, the second wall 1068 curves upwardly away from the second resilient region to meet the connecting portion 1070. Additionally, the second wall 1068 is tapered in thickness which increases from the connecting portion1070 to the second resilient region 1060. Both the curve and the taper in the second wall 1068 cause the initial deformation of the deformation panel 1064 to be accommodated by the first wall 1066 and cause further deformation to be accommodated by the second wall 1068 buckling and rolling over the first wall 1066.

The second wall 1068 has a length in the range of 2mm to 15 mm. Optionally, the length is in the range of 2 mm to 10mm. In the embodiment shown in Figures 50 to 70, the second wall 1068 has a length of 5 mm. The length may be different depending on the travel required to absorb deformation of the seal member 1020. The thickness of the second wall 1068 may vary in thickness from 0.15 mm where it joins the curved corner 1070 to 2 mm where it joins the second resilient region 1060. In the embodiment shown, the thickness ranges from 0.27 mm to 0.4 mm.

The geometries of the first and second walls 1066, 1068 and their thicknesses are selected so that the cushion module 1012 can accommodate a wide range of facial geometries and deformation forces associated with application and use of the patient interface. However, it is possible for different cushion modules to be produced to fit specific ranges of facial geometries which fall toward the ends of the facial geometry spectrum.

While the first and second resilient regions 1058, 1060 are formed with greater wall thicknesses to provide greater resilience compared to the deformation panel 1064, this is done so that a single material can be used to form the seal member 1020. However, it will be appreciated that the first and second resilient regions 1058, 1060 may be stiffened by alternative means provided that the deformation panel 1064 preferentially deforms when a force is applied to the seal member 1020. For example, the first and second resilient regions 1058, 1060 may be formed of more resilient materials (such as a different grade of silicone or a plastic material) or may have a different structure.

The housing 1080 (shown in more detail in Figures 61 to 65) has a body 1082 with front and rear surfaces 1112, 1114. The rear surface forms part of the primary flow cavity 1032 and the exhaust flow cavity 1030. The periphery of the body 1082 includes outwardly projecting tab members 1084 and a bead 1036 disposed on the end of the tab members 1084 to define a series of outer over-mould windows or apertures 1088. The seal member 1020 is over-moulded with the windows to form a permanent mechanical connection and seal between the housing 1080 and the seal member 1020.

As explained above, inner over-mould windows 1092 (Figures 63 and 64) are formed in the housing 1080 extending in a U-shape across the body 1082. The inner over-mould windows 1092 extend from and back to the outer over-mould windows 1088 across the top of the body 1082. To enable respiratory gas to be vented to ambient from the exhaust flow cavity 1030, a group of vent apertures 1090 is located within the region bound by the inner over-mould windows 1092.

Pressure ports 1094 are located at lower lateral sides of the body 1082 and a respiratory gas inlet opening 1096 is located in a lower central position in the body 1082. The inlet opening 1096 is adapted to connect the frame 1014 and the conduit connector 1016 with the cushion module 1012. In particular, the inlet opening 1096 is defined by a sleeve 1098 which has an inner end wall 1100 and an outer end wall 1102. The outer end wall 1102 has a pair of laterally opposed arcuate flanges 1104 which are spaced apart to define upper and lower recesses 1106 between them.

The recesses 1106 act as keying formations because they assist to retain the frame 1014 and the socket insert in alignment with the cushion module 1012. Specifically, the conduit connector 1016 plugs-in to the inlet opening 1096 and traps the frame 1014 between the conduit connector 1016 and the housing 1080. In addition to controlling alignment, this arrangement allows the conduit connector 1016 and the frame 1014 to be released from and re-assembled with the cushion module 1012 whenever required.

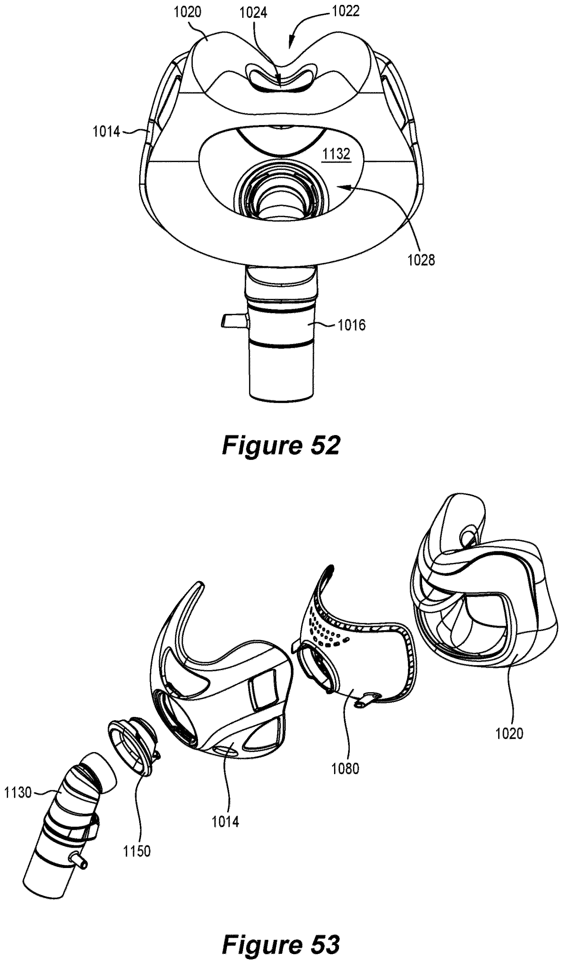

The conduit connector 1016 comprises an elbow 1130 (Figures 53 and 54) and a socket insert 1150 (Figures 53, 54, 69 and 70). One end of the elbow 1130 is a taper connection that is connectable with an inspiratory gas flow conduit from a flow generator or ventilator. The other end of the elbow 1130 has a channel 1140 that links the taper connection to a neck portion 1138 which, in turn, transitions into a ball element 1134. The outer surface of the ball element 1134 is shaped as a spherical segment.

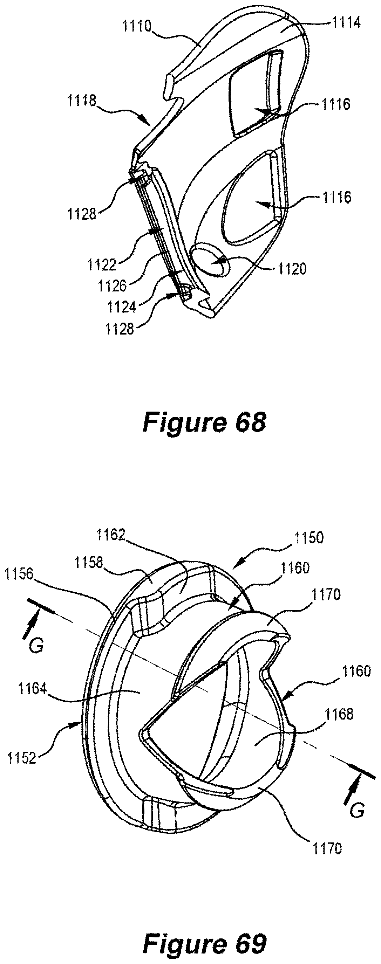

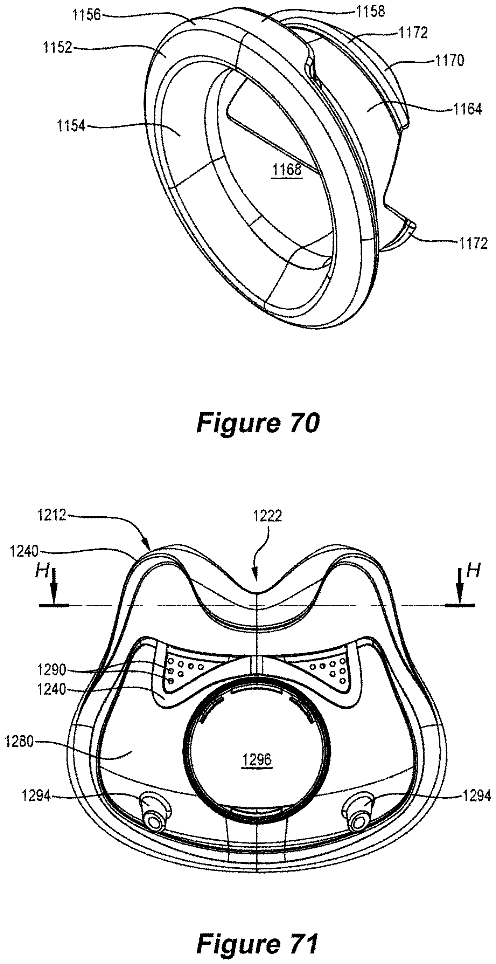

The socket insert 1150 has an annular flange 1152 with an inner wall 1154 that tapers inwardly. The inward taper co-operates with the neck portion 1138 and the ball element 1134 of the elbow 1130 to provide rotational freedom of movement vertically and laterally. It also has an outer wall 1156 that defines a first lip 1162 which includes formations 1158 which interact with the recesses 1106 of the housing 1080 to restrict rotational movement of the socket insert 1150 and the frame 1014 relative to the mask housing 1080. The socket insert 1150 further has a two fingers 1160 (Figures 69 and 70) that extend axially from the flange 1152. Each finger 1160 has an outer wall 1156 that has a shape which corresponds to the shape of the inside of the sleeve 1098 and an inside wall 1168 that is correspondingly shaped to the outer surface of the ball element 1134 so the fingers 1160 collectively define a socket for the ball element 1134. An arcuate flange portion 1170 is disposed on an end of each finger 1160 to define a radially projecting second lip 1172.

In the assembled patient interface 1010, the socket insert 1150 sandwiches the frame 1014 against the housing 1080 as part of the interference fit of the conduit connector 1016 with the housing 1080. This is enabled by the shape of the frame 1014, as shown in Figures 54 and 66 to 68. Specifically, the frame 1014 has a body 1110 which is roughly shaped to follow the contour of the cushion module 1012. The frame 1014 enables the patient interface 1010 to be connected to headgear which holds the patient interface 1010 in position during treatment. For this reason, the frame 1014 includes upper and lower headgear connection points 1116. Standard headgear connections can be used to connect the headgear. In this embodiment, the headgear connection points 1116 comprise upper and lower pairs of apertures on the lateral side of the frame 1014.

The frame 1014 includes a conduit opening 1122 through which the conduit connection 1016 passes for connection to the housing 1080. To facilitate this, the frame 1014 includes the conduit opening 1122 with a stepped profile (see Figure 68) which defines arcuate shoulders 1126 that are recessed from a front surface 1112 and are opposed to define upper and lower recesses 1128 between them. An innermost perimeter of the conduit opening 1122 is dimensioned to fit about the inlet opening 1096 of the housing 1080. When assembled with the cushion module 1012, the recesses 1128 are shaped to receive the formations 1158 of the socket insert 1150. This interaction between the recesses 1128 and the formations 1158 fixes the orientation of the frame 1014 relative to the cushion module 1012.

It will be appreciated, however, that the frame 1014 may be connected to the housing 1080 by any conventional means, such as gluing or welding. The frame 1014 also includes a bias vent opening. In the patient interface, the bias vent opening 1118 aligns with the vent apertures 1090 of the housing 1080 to permit exhausted respiratory gas from the exhaust flow cavity 1030 to vent to ambient without interference from the frame 1014.

While this embodiment includes the frame 1014, headgear connection points may be integrated with or connected to the housing 1080 in alternative embodiments. If so, the frame 1014 is not necessary and could be omitted from such embodiments.

As shown in Figure 54, the conduit connector 1016 and the frame 1014 are assembled with the housing 1080 by fitting the fingers 1160 of the socket insert 1150 through the sleeve 1098 such that the second lip abuts the inner end wall 1100. This involves positioning the frame 1014 between the socket insert 1150 and the housing 1080 and aligning the formations of the socket insert 1150 with the recesses of the frame 1014 and the housing 1080. The socket insert 1150 is then pressed and inserted into the inlet opening 1096 until the second lip abuts the inner end wall 1100. The elbow 1130 is connected to the cushion module 1012 by inserting the ball element 1134 into the socket insert 1150, as shown in Figure 54.

In an alternative embodiment, a cushion module 1212 (Figure 71) is provided in a similar form to the cushion module 1012, but is adapted to receive a larger conduit connector 1016.

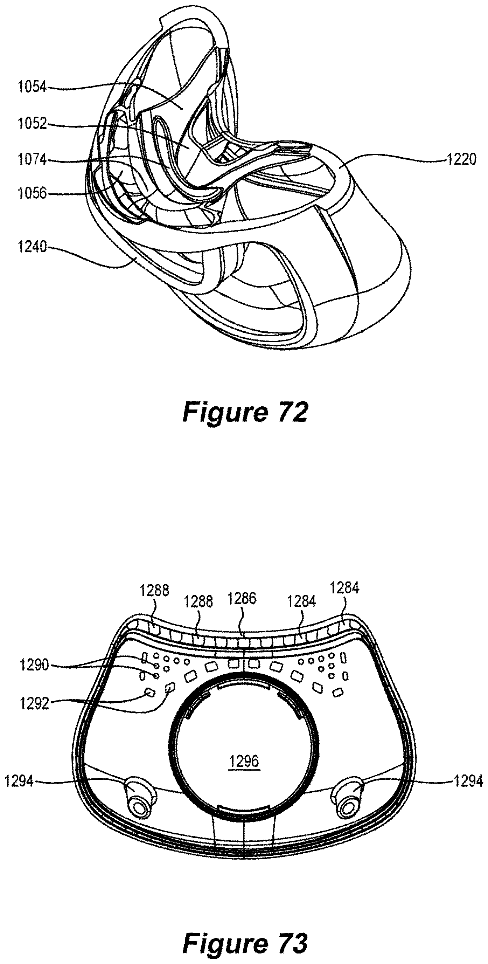

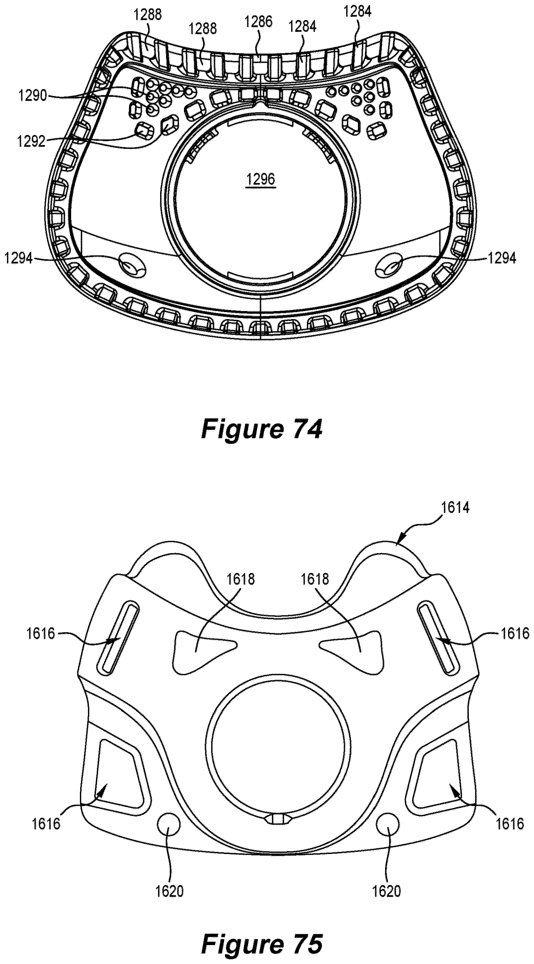

As with the cushion module 1012, the cushion module 1212 comprises a housing 1280 and a seal member 1220. The housing 1280 includes tab members 1284, a bead 1286, outer over-mould windows 1288 and pressure ports 1294 that are the same as their counterparts in the cushion module 1012. The description of those features in respect of the cushion module 1012 applies equally to the corresponding features of the cushion module 1212 shown in Figures 73 and 74. However, in the housing 1280, an inlet opening 1296 is centrally located and extends up to a short distance from outer over-mould windows 1288. In contrast to the cushion module 1012, bias vent apertures 1290 are grouped on each lateral side of the inlet opening 1296. This is a consequence of the inlet opening 1296 extending closer to the outer over-mould windows 1288 across the top of the housing 1280. The inner over-mould windows 1292 form a boundary together with the outer over-mould windows 1288 about each grouping of bias vent apertures 1290. In this embodiment, inner over-mould windows 1292 are arranged in a W-shape. This shape results in a corresponding W-shape inner over-mould 1240 of the seal member 1220 as seen in Figure 71.

It will be appreciated, however, that the inner over-mould windows 1292 may alternatively be formed in two separate V-shape or U-shape arrangements that extend about each grouping of bias vent apertures 1290. Other embodiments may have different arrangements of bias vent apertures 1290 that result in different shapes being defined by the inner over-mould windows 1292.

The cushion module 1212 includes a cavity wall 1050 that includes a deflector panel 1052, lateral panel 1054 and deformation region 1074 that are the same as their counterparts in the cushion module 1012. The description of those features in respect of the cushion module 1012 apply equally to the cushion module 1212 and the corresponding features are shown in Figure 72. However, instead of having a main panel 1056 that contacts the housing 1280 along a U-shape line of contact which follows the inner over-mould windows 1092, a main panel 1056 of the cushion module 1212 traces a W-shaped line of contact with the housing 1280. As a result, the main panel 1056 extends downwardly from a first resilient region 1058 deeper at lateral areas of the terminal wall than in the centre of the main panel 1056.

In a further alternative embodiment, the main panel 1056 may be formed without over-moulding of the main panel 1056 to the housing 1280 so that the inner over-mould 1240 is omitted. In such embodiments, bias vent apertures 1290 may be formed in the seal member 1220 instead of in the housing 1280 so that the main panel 1056 joins with the seal member 1220. Alternatively, the main panel 1056 may abut or may interact with the housing 1280 to form a seal, for instance by gluing or welding.

To accommodate the configuration of the housing 1280, the frame 1014 is replaced by a reconfigured frame 1614 (Figure 75). The frame 1614 includes upper and lower headgear connection points 1616, pressure port openings 1620, conduit opening 1622 and recesses 1628 that are the same as their counterparts in the frame 1014. The description of those features in respect of the frame 1014 apply equally to the frame 1614 and the corresponding features are shown in Figure 75. However, the frame 1614 differs in that the bias vent openings 1618 are located on lateral sides of the conduit opening 1622 to align with the bias vent apertures 1290 of the housing 1280 when the frame 1614 is assembled with the cushion module 1212.

Alternative embodiments of the patient interface may be adapted to connect to a flow generator to deliver respiratory gas from a flow generator to a cushion module and to transfer respiratory gas from the cushion module to the flow generator. Patient interfaces that are configured in this manner are known as a dual limb patient interfaces. They capture the exhausted respiratory gas and direct it back to the flow generator, rather than venting the exhausted respiratory gas to ambient atmosphere.

Three different embodiments of dual limb patient interfaces are described below and are respectively based on the concepts of co-axial, divided inlet and separate inlet conduit arrangements. While these embodiment are described in respect of different cushion modules, it will appreciated that the conduit arrangement concepts can be adapted to work with other cushion modules disclosed in this specification or in respect of other available or known cushion modules.

In each of the following embodiments, bias flow is increased by incorporating a bias leak in the expiratory flow path. The bias leak may be adjustable. Additionally, the bias leak may be in the range of 5 to 15 L/m. This leak rate is expected to reduce interference with the operation of flow generators or ventilators operating in a dual limb setup.

The bias leak is believed to induce higher bias flow rate which, in turn, is expected to improve dead-space flushing while using the patient interfaces disclosed in this specification. It follows that the bias leak may make patient interfaces described in this specification suitable for use with flow generators or ventilators that provide insufficient gas flow volumes for anatomical dead-space flushing to occur in a dual limb setup. In other words, the patient interfaces described here may make some flow generators or ventilators useful for anatomical dead-space flushing treatment while operating in a dual limb setup.

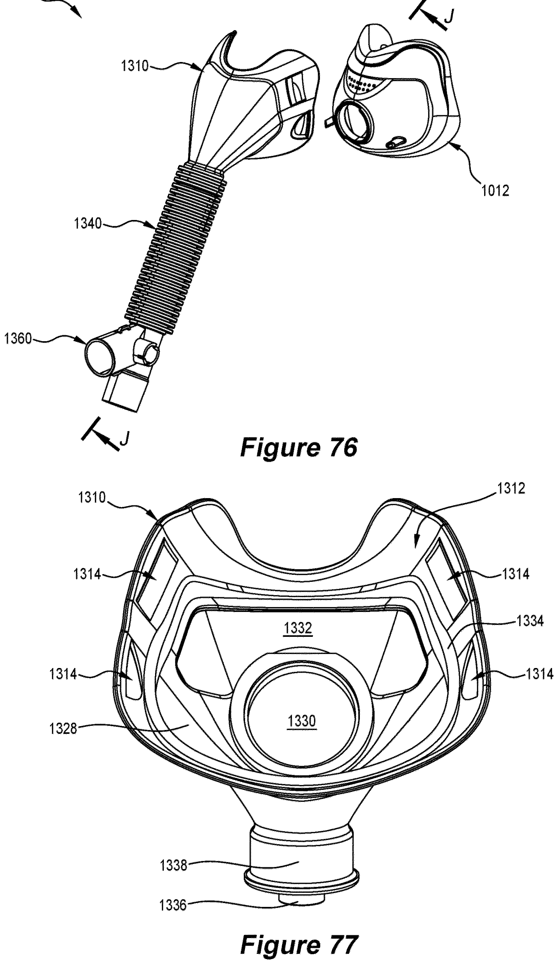

One embodiment of a co-axial patient interface 1300 is shown in Figures 76 to 81. The patient interface include the cushion module 1012 as described above and a frame 1310 The patient interface 1300 includes an inlet path 1324 that is configured to deliver respiratory gas to the inlet opening 1096 of the cushion module 1012 and an exhaust path 1326 that is configured to receive respiratory gas from the cushion module 1012 and where the inlet path 1324 and the exhaust path 1326 are co-axial.

In the embodiment shown in Figures 76 to 81, the inlet and exhaust paths 1324, 1326 are defined by a co-axial conduit 1340 having an inner conduit 1342 and an outer conduit that surrounds the inner conduit 1342. The inner conduit 1342 has an inner bore 1346 and an outer conduit co-axially surrounding inner conduit 1342 to define an annular outer bore 1348. In this embodiment, the inner conduit 1342 defines the inlet path 1324 and the outer conduit 1344 defines the exhaust path 1326. This may be switched in other embodiments so the inner conduit 1342 defines the exhaust path and the outer conduit 1344 defines the inlet path. The conduit may be any length. Optionally, it is a length that is sufficient to decouple forces between the cushion module 1012 and the inspiratory and expiratory limbs (conduits) of a flow generator or ventilator.

The co-axial conduit 1340 is connected to a frame 1310 which is adapted to extend the inlet path 1324 to the inlet opening 1096 of the cushion module 1012 and which is adapted to extend the exhaust path 1326 from the bias vent apertures 1090 of the cushion module 1012 to the outer conduit 1344. More specifically, the frame 1310 has an inner duct 1316 which defines an inner passage 1318 that connects to the inner conduit 1342 to extend the inlet path 1324 of the co-axial conduit 1340 to the inlet opening 1096 of the cushion module 1012. The connection is made via an inner conduit-connecting flange 1336. The inner duct 1316 interacts with the inlet opening 1096 to deliver respiratory gas from the inlet path 1324 to the primary flow cavity 1032 of the cushion module 1012. Similarly, the frame 1310 has an outer duct 1320 surrounding the inner duct 1316 to define an outer passage 1322 which connects the bias vent apertures 1090 to the outer conduit 1344. The connection is made via an outer conduit connecting flange 1338. In this arrangement, the exhaust path 1326 extends through the outer passage 1322 from the cushion module 1012 and through the outer bore 1348 of conduit 1342.

The outer duct 1320 terminates in a flange 1334 which is configured to seal with the cushion module 1012 an area that surrounds the vent apertures 1090. More specifically, the flange 1334 is shaped to seal against an outer over-mould 1038 of the cushion module 1012. In an alternative embodiment, the flange 1334 may be formed to seal with the inner over-mould 1040 and a portion of the outer over-mould 1038 to seal the outer passage 1322 with the cushion module 1012.

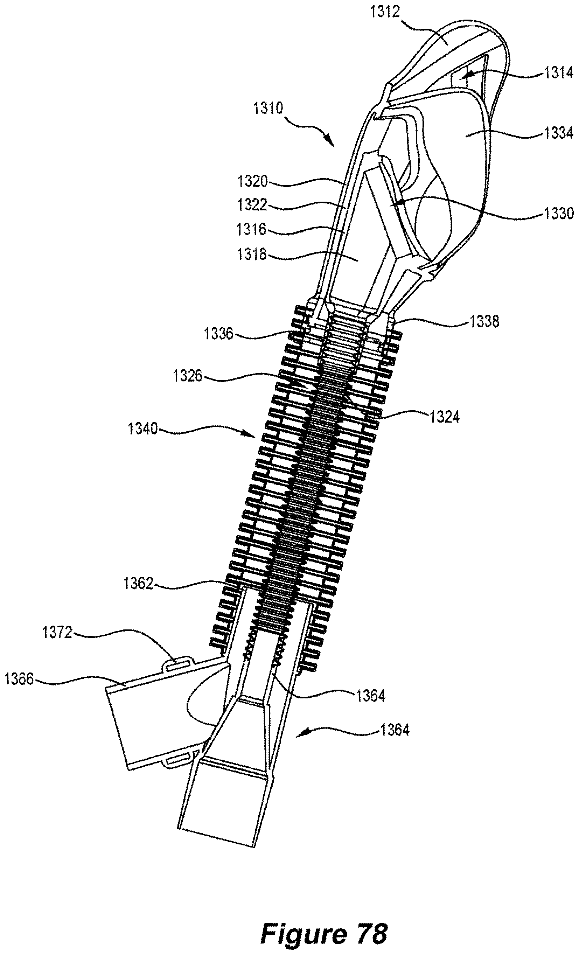

As shown in Figures 77 and 78, the frame 1310 includes a partition wall 1328 that includes an inner passage opening 1330 which is configured to connect the inner passage 1318 with the primary flow cavity 1032 of the cushion module 1012. The inner passage opening 1330 is integrated with the partition wall 1328 so that the link is made when the flange 1334 seals against the cushion module 1012. The link may be maintained by a connection 1380 to provide an interference fit between the frame 1310 and cushion module 1012. The connector 1380 may be in the form of the socket insert 1150 described above for the patient interface shown in Figures 50 to 70. However, the connector 1380 may be formed integrally with the partition wall 1328 or it may be formed separately in a form that enables connection of the frame to the cushion module 1012.

While any suitable connection may be used to connect the frame 1310 to the cushion module 1012 to enable respiratory gas flow between the cushion module 1012 and the frame 1310, one option includes an interference fit connector. For example, the connector may comprise a series of deformable fingers on the frame 1310 that deflect to pass through the inlet opening 1096 and that have a return lip which snap into contact with the inner end wall 1100. Alternatively, the inlet opening 1096 may be formed with formations that interlock with corresponding formations on the frame 1310. The formations on both may be designed for interference fit, twist-lock fit, press-fit, taper connection or any other suitable form of connection that fixes the frame 1310 to the cushion module 1012.

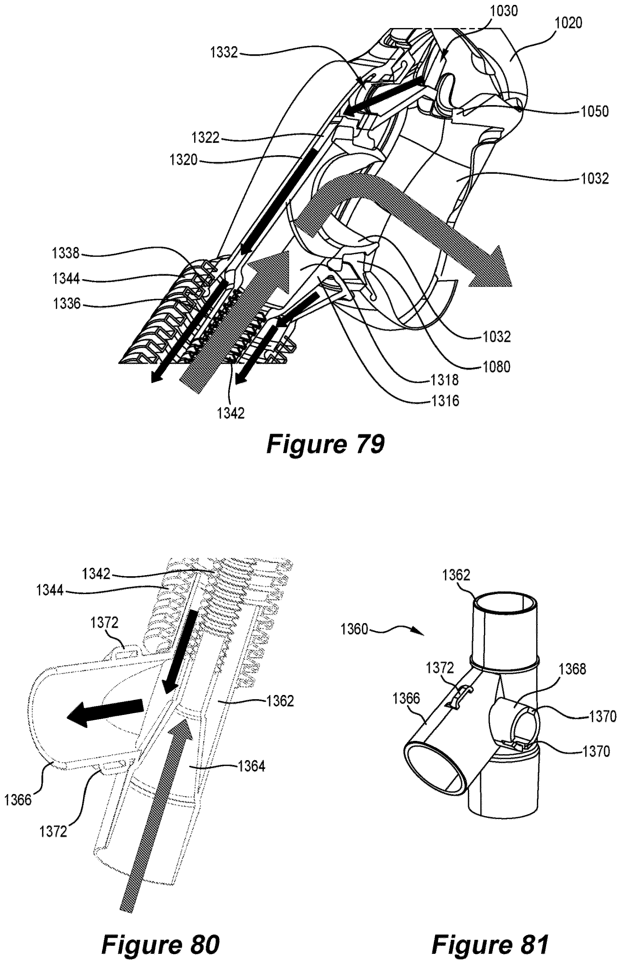

The partition wall 1328 also includes an exhaust path opening 1332 which opens into the outer passage 1322. The flow of respiratory gas into the cushion module 1012 through the inner passage opening 1330 and from the cushion module 1012 through the exhaust path opening 1332 is shown in Figure 79 by respective arrows.

At the other end of the co-axial conduit 1340 (remote from the frame), a co-axial conduit connector 1360, in the form of a splitter, connects separate, spaced inspiratory and expiratory limbs of a flow generator or ventilator to the co-axial conduit 1340. The connector is configured to link the inner conduit 1342 to the inspiratory limb and is configured to link the outer conduit 1344 to an expiratory limb. More specifically, the connector has an outer conduit connector 1362 and an inner conduit connector 1364 to form these links. The inner conduit connector 1364 is formed as a funnel that transitions respiratory gas flow from the inspiratory limb to the inner bore 1346 of the inner conduit 1342. Both the inner and outer conduit connectors 1362, 1364 have a standard profile size and shape for connecting with the inspiratory and expiratory limbs of a flow generator. In the embodiment shown in Figures 76, 78, 80 and 81, the inner conduit connector 1364 is contiguous with the co-axial conduit 1340. The outer conduit connector 1362, however, branches from a side of the co-axial conduit connector 1360. The angle of the outer conduit connector 1362 relative to the inner conduit connector 1364 results in an acute-angle change in direction for the exhaust path 1326.

The outer conduit connector 1362 includes integrated loops 1372 which may interact with the expiratory limb. The loops, as shown in Figures 80 and 81, are integrated with the exterior surface of the outer conduit connector 1362.

The co-axial conduit connector 1340 includes a bias flow vent 1368 which is configured to vent gas from the exhalation path to ambient atmosphere. The bias flow vent 1368 may be adjustable to vary the flow of respiratory gas to ambient atmosphere. For example, the flow may be adjusted to a flow in the range of 5 to 15 L/m. In this embodiment, the leak via the boas flow vent 1368 is around 10L/m. The bias flow vent 1368 may include a filter to mitigate infection risks associated with leaked respiratory gas.

Additionally, the bias flow vent 1368 may be configured to inhibit connection to another conduit. Such connection may inhibit flow of respiratory gas via the bias flow vent 1368 and, therefore, may reduce the dead-space flushing effect provided by the patient interface. To address this, the bias flow vent 1368 includes one or more formations that provide a visual indication that a removable conduit should not be connected with the bias flow vent 1368. The one or more formations inhibit a sealed connection with a removable conduit or prevent occlusion of the bias flow vent. The formations, in this embodiment comprise three recesses 1370 formed in the end rim of the bias flow vent 1368.

As an alternative or in addition to the recesses 1370, the bias flow vent 1368 may have a non-standard size or shape so that a removable conduit cannot be connected with the bias flow vent 1368.

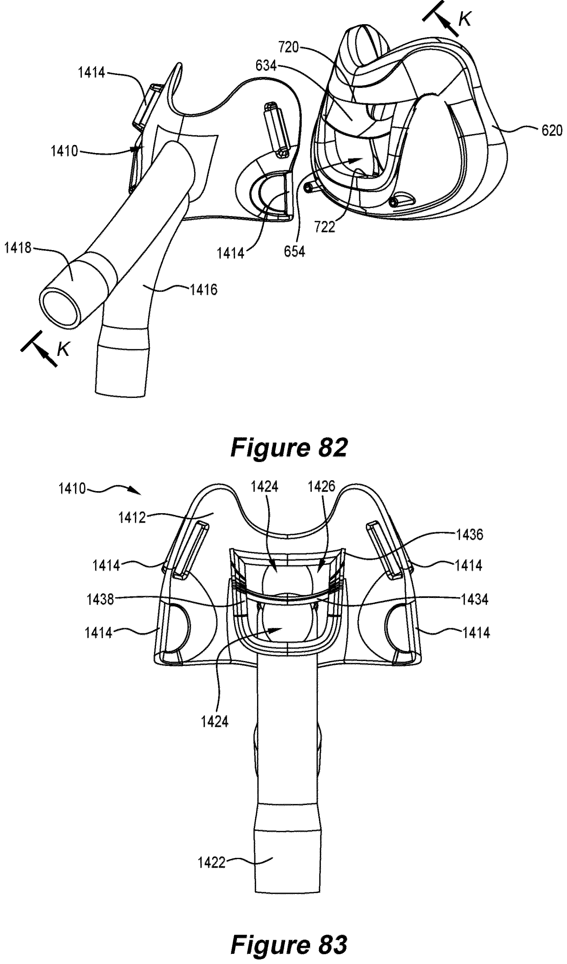

An alternative embodiment of a dual limb patient interface 1400 is shown in Figures 82 to 85 and includes the cushion module 620 as show in Figures 30 to 34. The description above relating to the cushion module 620 applies equally here to this embodiment.

The patient interface 1400 includes a frame 1410 which is adapted to connect with inspiratory and expiratory limbs of a flow generator. The frame 1410 comprises a body 1412 with upper and lower headgear connector points 1414. In this embodiment, the dual limb aspect is provided by the frame 1410 having an inspiratory conduit 1422 that is configured to deliver respiratory gas to the primary flow cavity 636 of the cushion module 620 and an expiratory conduit 1418 that is configured to receive respiratory gas from the cushion module 620. These conduits 1418, 1422 define respective inlet and outlet channels 1420, 1424 that are partly separate channels in a single conduit 1416 and partly separate channels in respective separate conduits 1418, 1422.

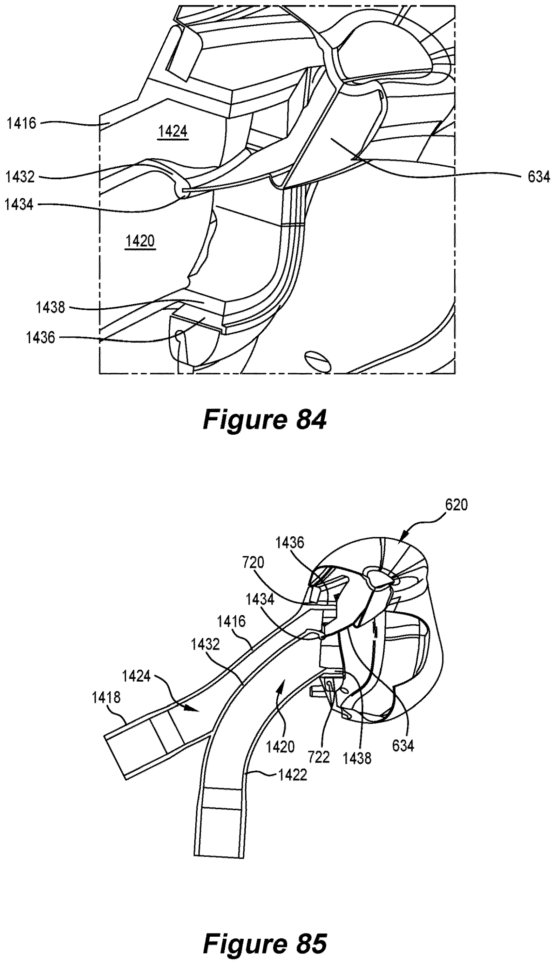

As shown in Figures 82 and 85, in the single conduit 1416, the channels 1420, 1424 are separated by a common dividing wall 1432. However, as the channels 1420, 1424 diverge as they extend away from a cushion-module end of the frame 1410 to become separate conduits, namely the inspiratory conduit 1422 and the expiratory conduit 1418. The inspiratory conduit 1422 associated with the inlet channel 1420 is connectable with the inspiratory limb of a flow generator or ventilator and the expiratory conduit 1418 associated with the outlet channel 1424 is connectable with the expiratory limb of a flow generator or ventilator. As with the co-axial conduit embodiment described above, each of the inspiratory and expiratory conduits 1418, 1422 has a length that is sufficient to decouple forces between the cushion module 620 and the inspiratory and expiratory limbs.

As shown in Figures 83 and 84, the inlet and outlet channels 1420, 1424 terminate in a combined opening 1426 at the cushion-module end of the frame 1410. The dividing wall 1432 terminates in a transom 1434 that extends across the combined opening 1426 of the single conduit 1416. Although the dividing wall 1432 is shown as extending across the single conduit 1416, in an alternative embodiment, the frame 1410 may include a transom 1434 extending across the combined opening 1426 so that the dividing wall 1432 meets the transom 1434 of the frame 1416 to separate the inlet channel 1420 from the outlet channel 1424. Either way, the dividing wall 1432 and the cavity wall 634 of the cushion module 620 separate the inlet channel 1420 from the outlet channel 1424. In one option, the dividing wall 1432 and the cavity wall 634 of the cushion module 620 interact with each to form a seal that separates the inlet channel 1420 from the outlet channel 1424. In this embodiment, as shown in Figures 84 and 85, the interaction involves an edge of the dividing wall 1432 being received in a groove along an edge of the transom 1434. However, it will be appreciated that alternative embodiments involve other forms of interaction which result in a seal between the dividing wall 1432 and the cavity wall 634 will also be suitable.

The combined opening 1426 is bound by a fitting member 1438 which is configured to couple the single conduit 1416 to a sleeve portion 1436 of the frame 1410. The sleeve portion 1436 is configured to connect the frame 1410 to the cushion module 620. The sleeve portion 1436 is adapted to co-operate with the upper and lower rims 720, 722 of the opening 654 in the cushion module 620 to couple the frame to the cushion module 620. The sleeve portion 1436 is configured for releasable coupling of the single conduit 1416 to the cushion module 620. Such coupling may be enabled by friction or interference fit formations that interact with the upper and lower rims 720, 722. The single conduit 1416 may be formed with friction or interference fit formations that enable releasable coupling with the sleeve 1436. Alternatively, the single conduit 1416 may be glued or welded to the sleeve 1436 or fixed by other means, such as a permanent coupling.

Although not shown in the drawings, the outlet channel 1420 includes a bias flow vent in the form described above in respect of the embodiment involving co-axial conduits.

An alternative dual limb arrangement for coupling one of the cushion modules disclosed in this specification to a dual limb flow generator or ventilator involves separate conduits that connect to the cushion module separately.

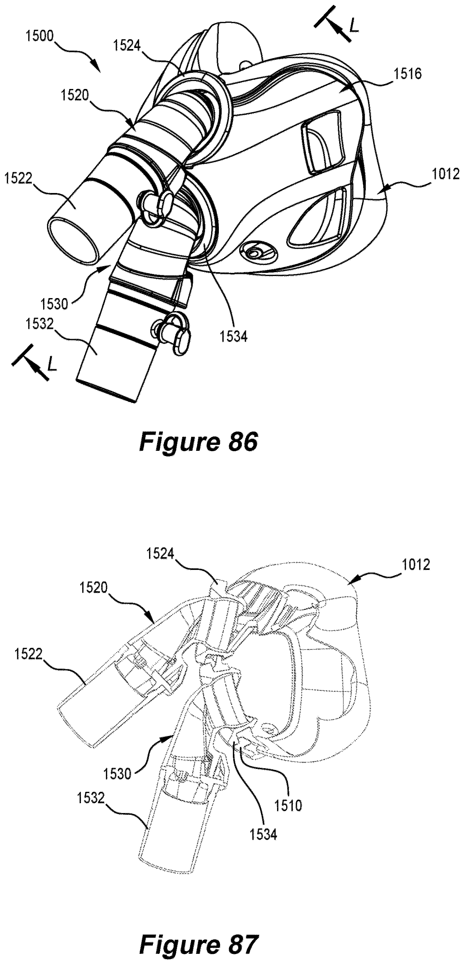

An embodiment of this is shown in Figures 86 and 87 in which a patient interface 1500 includes the cushion module 1012 shown in Figures 61 and 62 and a frame 1510 that incorporates two separate conduits 1520, 1530. More specifically, the frame 1510 includes a first conduit 1520 that is configured to deliver respiratory gas to the primary flow cavity 1032 of the cushion module 1012 and a second conduit 1530 that is configured to receive respiratory gas from the cushion module 1012 and the first and second conduits 1520, 1530 are spaced apart.

In this embodiment, the first conduit 1520 is configured to open into the primary flow cavity 1032 and the second conduit 1530 is configured to open into the exhaust flow cavity 1030. To accommodate this arrangement, the frame 1510 has separate respective openings through which pass the first and second conduits 1520, 1530.

In this embodiment, both conduits 1520, 1530 have the form of the conduit connector 1016, including the elbow 1130 and the socket insert 1150, as shown in Figures 50 to 54. The frame 1510 is adapted to accommodate this stacked arrangement of conduits 1520, 1530. However, in variations of this embodiment one or neither of the two conduits 1520, 1530 has the socket insert 1150 and the elbow 1130 which permit rotational movement of the conduits 1520, 1530 relative to the cushion module 1012. In other words, one or both of the conduits 1520, 1530 may be coupled to the cushion module 1012 in a fixed orientation and/or position.

As with the co-axial and divided inlet embodiments, the exhaust conduit 1530 includes a bias flow vent to vent respiratory gas to ambient.

Those skilled in the art of the present invention will appreciate that many variations or modifications may be made to the preferred embodiment without departing from the scope of the appended claims.

In the claims which follow, and in the preceding description, except where the context requires otherwise due to express language or necessary implication, the word "comprise" and variations such as "comprises" or "comprising" are used in an inclusive sense, i.e. to specify the presence of the stated features but not to preclude the presence or addition of further features in various embodiments of the apparatus as disclosed herein.

In the foregoing description of preferred embodiments, specific terminology has been resorted to for the sake of clarity. However, the invention is not intended to be limited to the specific terms so selected, and it is to be understood that each specific term includes all technical equivalents which operate in a similar manner to accomplish a similar technical purpose. Terms such as "front" and "rear", "inner" and "outer", "above", "below", "upper" and "lower", "up" and "down" and the like are used as words of convenience to provide reference points and are not to be construed as limiting terms. The terms "vertical" and "horizontal" when used in reference to the patient interface throughout the specification, including the claims, refer to orientations relative to the normal operating orientation.

The reference in this specification to any prior publication (or information derived from it), or to any matter which is known, is not, and should not be taken as, an acknowledgement or admission or any form of suggestion that prior publication (or information derived from it) or known matter forms part of the common general knowledge in the field of endeavour to which this specification relates.

Furthermore, invention has been described in connection with what are presently considered to be the most practical and preferred embodiments, it is to be understood that the invention is not to be limited to the disclosed embodiments, but on the contrary, is intended to cover various modifications included within the scope of the invention. Also, the various embodiments described above may be implemented in conjunction with other embodiments, for example, aspects of one embodiment may be combined with aspects of another embodiment to realize yet other embodiments. Further, each independent feature or component of any given assembly may constitute an additional embodiment.

## Claims

1. A non-invasive patient interface (1010) that is configured to deliver pressurized respiratory gas to the mouth and nares of a patient, the patient interface (1010) comprising a cushion module (1012) which comprises:
(a) a seal (1020) for sealing (1020) around the mouth and nares of the patient;
(b) a housing (1080) connected to the seal (1020);
(c) an interior volume defined by the seal (1020) and the housing (1080);
(d) the seal (1020) includes a cavity wall (1050) located within the interior volume so as to define first and second cavities (1032, 1030) within the interior volume of the cushion module; and
(e) a preferential deformation region (1074) that is part of the cavity wall (1050) and that includes a deformation panel (1064) and includes first and second resilient regions (1058, 1060) between which the deformation panel (1064) is disposed.

2. The patient interface (1010) as claimed in claim 1, wherein the deformation panel (1064) includes first and second walls (1066, 1068) and a connecting portion (1070) between the first and second walls (1066, 1068).

3. The patient interface (1010) as claimed in claim 2, wherein the first wall (1066) projects from the first resilient region (1058) in a first direction, the second wall (1068) projects from the second resilient region (1060) in a second direction different from the first direction.

4. The patient interface (1010) as claimed in claim 3, wherein deformation of the deformation region (1074) involves a reduction in the spacing between the first and second resilient regions (1058, 1060) and an associated deformation of the deformation panel (1064) to accommodate the reduction in spacing.

5. The patient interface (1010) as claimed in claim 3 or claim 4, wherein the second direction is inclined downwardly from a plane intersecting the second resilient region (1060) and the connecting portion (1070).

6. The patient interface (1010) as claimed in any one of claims 3 to 5, wherein the connecting portion (1070) has a bend profile which, at rest, aligns with the first direction of the first wall (1066) and aligns with an end of the second wall (1068) remote from the second resilient region (1060).

7. The patient interface (1010) as claimed in claim any one of claims 3 to 6, wherein the second wall (1068) has a curved profile from the connecting portion (1070) to the second resilient region (1060) and preferably the second wall (1068) increases in thickness from the connecting portion (1070) to the second resilient region (1060).

8. The patient interface (1010) as claimed in any one of claims 3 to 7, wherein the deformation panel (1064) has a wall thickness that is less than the wall thickness of the first and second resilient regions (1058, 1060) and preferably the first and second resilient regions (1058, 1060) have a wall thickness that is at least three times the wall thickness of the deformation panel (1064).

9. The patient interface (1010) as claimed in any one of claims 3 to 8, wherein the cavity wall (1050) further includes a main panel (1056) which connects the housing (1080) to the first resilient region (1058).

10. The patient interface (1010) as claimed in any one of claims 3 to 9, wherein the cavity wall (1050) further includes a deflector panel (1052) which is recessed from a rim (1034) of a nasal aperture (1024) and which forms a channel configured to direct respiratory gas from the first cavity (1032) to the nasal aperture (1024) and preferably the deflector panel (1052) abuts the second resilient region (1060).

11. The patient interface (1010) as claimed in claim 10, wherein the deformation region (1074) is arranged to structurally decouple the deflector panel (1052) from the main panel (1056).

12. The patient interface (1010) as claimed in any one of claims 1 to 9, wherein the cavity wall (1050) is positioned relative to a nasal aperture (1024) and an oral aperture (1028) to enable respiratory gas to flow from the first cavity (1032) to the nares through the nasal aperture (1024).

13. The patient interface (1010) as claimed in any one of claims 1 to 9, wherein the cavity wall (1050) is positioned relative to a nasal aperture (1024) and an oral aperture (1028) to enable exhaled respiratory gas from the mouth and nares to flow into the second cavity (1030).

14. The patient interface (1010) as claimed in any one of claims 1 to 13, wherein the cushion module (1012) further comprises an exhaust vent (1090) to communicate respiratory gas from within the cushion module (1012) to externally of the cushion module (1012), preferably the second cavity (1030) is an upper cavity disposed above the first cavity (1032) and is in communication with the exhaust vent (1090) and preferably the exhaust vent (1090) comprises one or more groups of apertures.

15. The patient interface (1010) as claimed in claim 14, wherein the housing (1080) includes the exhaust vent (1090) and the cavity wall connection with the housing (1080) at least partially surrounds the exhaust vent (1090) and preferably the exhaust vent is bound by the cavity wall connection with the housing (1080) and a connection between the perimeter of the seal (1020) and the perimeter of the housing (1080).

## Patentansprüche

1. Nicht-invasive Patientenschnittstelle (1010), die zur Abgabe von druckbeaufschlagtem Atemgas an den Mund und die Nasenlöcher eines Patienten ausgestaltet ist, wobei die Patientenschnittstelle (1010) ein Polstermodul (1012) umfasst, die Folgendes umfasst:
(a) eine Dichtung (1020) zum Abdichten (1020) um den Mund und die Nasenlöcher des Patienten,
(b) ein Gehäuse (1080), das mit der Dichtung (1020) verbunden ist,
(c) ein Innenvolumen, das von der Dichtung (1020) und dem Gehäuse (1080) definiert wird,
(d) wobei die Dichtung (1020) eine Hohlraumwand (1050) aufweist, die in dem Innenvolumen angeordnet ist, um einen ersten und einen zweiten Hohlraum (1032, 1030) in dem Innenvolumen des Polstermoduls zu definieren, und
(e) einen bevorzugten Verformungsbereich (1074), der Teil der Hohlraumwand (1050) ist und der eine Verformungsplatte (1064) aufweist sowie einen ersten und einen zweiten nachgiebigen Bereich (1058, 1060) aufweist, zwischen denen die Verformungsplatte (1064) angeordnet ist.

2. Patientenschnittstelle (1010) nach Anspruch 1, wobei die Verformungsplatte (1064) eine erste und eine zweite Wand (1066, 1068) sowie einen Verbindungsabschnitt (1070) zwischen der ersten und der zweiten Wand (1066, 1068) aufweist.

3. Patientenschnittstelle (1010) nach Anspruch 2, wobei die erste Wand (1066) von dem ersten nachgiebigen Bereich (1058) in einer ersten Richtung vorragt, und die zweite Wand (1068) von dem zweiten nachgiebigen Bereich (1060) in einer zweiten Richtung vorragt, die von der ersten Richtung verschieden ist.

4. Patientenschnittstelle (1010) nach Anspruch 3, wobei die Verformung des Verformungsbereichs (1074) eine Reduzierung des Abstands zwischen dem ersten und dem zweiten nachgiebigen Bereich (1058, 1060) und eine zugeordnete Verformung der Verformungsplatte (1064) beinhaltet, um die Reduzierung des Abstands aufzunehmen.

5. Patientenschnittstelle (1010) nach Anspruch 3 oder Anspruch 4, wobei die zweite Richtung von einer Ebene, die den zweiten nachgiebigen Bereich (1060) und den Verbindungsabschnitt (1070) schneidet, nach unten geneigt ist.

6. Patientenschnittstelle (1010) nach einem der Ansprüche 3 bis 5, wobei der Verbindungsabschnitt (1070) ein Biegungsprofil aufweist, das in Ruhe auf die erste Richtung der ersten Wand (1066) ausgerichtet ist und auf ein von dem zweiten nachgiebigen Bereich (1060) abgesetzten Ende der zweiten Wand (1068) ausgerichtet ist.

7. Patientenschnittstelle (1010) nach einem der Ansprüche 3 bis 6, wobei die zweite Wand (1068) ein gekrümmtes Profil von dem Verbindungsabschnitt (1070) zu dem zweiten nachgiebigen Bereich (1060) aufweist und vorzugsweise die Dicke der zweiten Wand (1068) von dem Verbindungsabschnitt (1070) zu dem zweiten nachgiebigen Bereich (1060) zunimmt.

8. Patientenschnittstelle (1010) nach einem der Ansprüche 3 bis 7, wobei die Verformungsplatte (1064) eine Wanddicke aufweist, die kleiner als die Wanddicke des ersten und des zweiten nachgiebigen Bereichs (1058, 1060) ist, und vorzugsweise der erste und der zweite nachgiebige Bereich (1058, 1060) eine Wanddicke aufweisen, die mindestens das Dreifache der Wanddicke der Verformungsplatte (1064) beträgt.

9. Patientenschnittstelle (1010) nach einem der Ansprüche 3 bis 8, wobei die Hohlraumwand (1050) ferner eine Hauptplatte (1056) aufweist, die das Gehäuse (1080) mit dem ersten nachgiebigen Bereich (1058) verbindet.

10. Patientenschnittstelle (1010) nach einem der Ansprüche 3 bis 9, wobei die Hohlraumwand (1050) ferner eine Ablenkplatte (1052) aufweist, die von einem Rand (1034) der Nasenöffnung (1024) zurückgesetzt ist und die einen Kanal bildet, der zum Leiten von Atemgas von dem ersten Hohlraum (1032) zu der Nasenöffnung (1024) ausgelegt ist, und vorzugsweise wobei die Ablenkplatte (1052) an dem zweiten nachgiebigen Bereich (1060) anliegt.

11. Patientenschnittstelle (1010) nach Anspruch 10, wobei der Verformungsbereich (1074) zur strukturellen Entkopplung der Ablenkplatte (1052) von der Hauptplatte (1056) angeordnet ist.

12. Patientenschnittstelle (1010) nach einem der Ansprüche 1 bis 9, wobei die Hohlraumwand (1050) bezüglich der Nasenöffnung (1024) und der Mundöffnung (1028) positioniert ist, um Strömung von Atemgas von dem ersten Hohlraum (1032) zu den Nasenlöchern durch die Nasenöffnung (1024) zu ermöglichen.

13. Patientenschnittstelle (1010) nach einem der Ansprüche 1 bis 9, wobei die Hohlraumwand (1050) bezüglich der Nasenöffnung (1024) und der Mundöffnung (1028) positioniert ist, um Strömung von ausgeatmetem Atemgas von dem Mund und den Nasenlöchern in den zweiten Hohlraum (1030) zu ermöglichen.

14. Patientenschnittstelle (1010) nach einem der Ansprüche 1 bis 13, wobei das Polstermodul (1012) ferner eine Entlüftungsöffnung (1090) zur Weiterleitung von Atemgas von innerhalb des Polstermoduls (1012) zu der Außenseite des Polstermoduls (1012) umfasst, vorzugsweise wobei der zweite Hohlraum (1030) ein oberer Hohlraum ist, der über dem ersten Hohlraum (1032) angeordnet ist und mit der Entlüftungsöffnung (1090) in Verbindung steht, und vorzugsweise wobei die Entlüftungsöffnung (1090) ein oder mehr Gruppen von Öffnungen umfasst.

15. Patientenschnittstelle (1010) nach Anspruch 14, wobei das Gehäuse (1080) die Entlüftungsöffnung (1090) aufweist und die Hohlraumwandverbindung mit dem Gehäuse (1080) die Entlüftungsöffnung (1090) mindestens teilweise umgibt und vorzugsweise die Entlüftungsöffnung von der Hohlraumwandverbindung mit dem Gehäuse (1080) und einer Verbindung zwischen dem Umfang der Dichtung (1020) und dem Umfang des Gehäuses (1080) begrenzt wird.

## Revendications

1. Interface patient non invasive (1010) qui est configurée pour délivrer un gaz respiratoire sous pression à la bouche et aux narines d'un patient, l'interface patient (1010) comprenant un module de coussin (1012) qui comprend :
(a) un joint (1020) pour le scellement (1020) autour de la bouche et des narines du patient ;
(b) un boîtier (1080) relié au joint (1020) ;
(c) un volume intérieur défini par le joint (1020) et le boîtier (1080) ;
(d) le joint (1020) comprenant une paroi de cavité (1050) située dans le volume intérieur de manière à définir des première et seconde cavités (1032, 1030) dans le volume intérieur du module de coussin ; et
(e) une région de déformation préférentielle (1074) qui fait partie de la paroi de cavité (1050) et qui comprend un panneau de déformation (1064) et comprend des première et seconde régions élastiques (1058, 1060) entre lesquelles le panneau de déformation (1064) est disposé.

2. Interface patient (1010) selon la revendication 1, le panneau de déformation (1064) comprenant des première et seconde parois (1066, 1068) et une partie de liaison (1070) entre les première et seconde parois (1066, 1068).

3. Interface patient (1010) selon la revendication 2, la première paroi (1066) faisant saillie à partir de la première région élastique (1058) dans une première direction, la seconde paroi (1068) faisant saillie à partir de la seconde région élastique (1060) dans une seconde direction différente de la première direction.

4. Interface patient (1010) selon la revendication 3, la déformation de la région de déformation (1074) impliquant une réduction de l'espace entre les première et seconde régions élastiques (1058, 1060) et une déformation associée du panneau de déformation (1064) pour tenir compte de la réduction de l'espace.

5. Interface patient (1010) selon la revendication 3 ou la revendication 4, la seconde direction étant inclinée vers le bas à partir d'un plan coupant la seconde région élastique (1060) et la partie de liaison (1070).

6. Interface patient (1010) selon l'une quelconque des revendications 3 à 5, la partie de liaison (1070) ayant un profil courbé qui, au repos, s'aligne sur la première direction de la première paroi (1066) et s'aligne sur une extrémité de la seconde paroi (1068) éloignée de la seconde région élastique (1060).

7. Interface patient (1010) selon l'une quelconque des revendications 3 à 6, la seconde paroi (1068) ayant un profil courbé de la partie de liaison (1070) à la seconde région élastique (1060) et, de préférence, la seconde paroi (1068) augmentant en épaisseur de la partie de liaison (1070) à la seconde région élastique (1060).

8. Interface patient (1010) selon l'une quelconque des revendications 3 à 7, le panneau de déformation (1064) ayant une épaisseur de paroi qui est inférieure à l'épaisseur de paroi des première et seconde régions élastiques (1058, 1060) et, de préférence, les première et seconde régions élastiques (1058, 1060) ayant une épaisseur de paroi qui est au moins trois fois l'épaisseur de paroi du panneau de déformation (1064).

9. Interface patient (1010) selon l'une quelconque des revendications 3 à 8, la paroi de cavité (1050) comprenant en outre un panneau principal (1056) qui relie le boîtier (1080) à la première région élastique (1058).

10. Interface patient (1010) selon l'une quelconque des revendications 3 à 9, la paroi de cavité (1050) comprenant en outre un panneau déflecteur (1052) qui est en retrait d'un bord (1034) d'une ouverture nasale (1024) et qui forme un canal configuré pour diriger le gaz respiratoire de la première cavité (1032) à l'ouverture nasale (1024) et, de préférence, le panneau déflecteur (1052) venant en butée contre la seconde région élastique (1060).

11. Interface patient (1010) selon la revendication 10, la région de déformation (1074) étant agencée pour découpler structurellement le panneau déflecteur (1052) du panneau principal (1056).

12. Interface patient (1010) selon l'une quelconque des revendications 1 à 9, la paroi de cavité (1050) étant positionnée par rapport à une ouverture nasale (1024) et une ouverture orale (1028) pour permettre au gaz respiratoire de s'écouler de la première cavité (1032) aux narines à travers l'ouverture nasale (1024).

13. Interface patient (1010) selon l'une quelconque des revendications 1 à 9, la paroi de cavité (1050) étant positionnée par rapport à une ouverture nasale (1024) et à une ouverture orale (1028) pour permettre au gaz respiratoire expiré provenant de la bouche et des narines de s'écouler dans la seconde cavité (1030).

14. Interface patient (1010) selon l'une quelconque des revendications 1 à 13, le module de coussin (1012) comprenant en outre un évent d'évacuation (1090) pour communiquer le gaz respiratoire de l'intérieur du module de coussin (1012) à l'extérieur du module de coussin (1012), de préférence la seconde cavité (1030) étant une cavité supérieure disposée au-dessus de la première cavité (1032) et étant en communication avec l'évent d'évacuation (1090) et de préférence l'évent d'évacuation (1090) comprenant un ou plusieurs groupes d'ouvertures.

15. Interface patient (1010) selon la revendication 14, le boîtier (1080) comprenant l'évent d'évacuation (1090) et la liaison de paroi de cavité avec le boîtier (1080) entourant au moins partiellement l'évent d'évacuation (1090) et, de préférence, l'évent d'évacuation étant délimité par la liaison de paroi de cavité avec le boîtier (1080) et une liaison entre le périmètre du joint (1020) et le périmètre du boîtier (1080).
